# EUROPEAN PATENT SPECIFICATION

(11) **EP 1 546 355 B1**
(45) Date of publication and mention of the grant of the patent: **21.11.2007**
(21) Application number: 03797505.9
(22) Date of filing: 19.09.2003
(51) Int. Cl.: C12P 19/34, C12Q 1/68, C12N 15/10

(54) **METHODS OF USE FOR THERMOSTABLE RNA LIGASES**
METHODE UND VERWENDUNG VON THERMOSTABILEN RNA-LIGASEN
PROCEDES D'UTILISATION DE LIGASES D'ARN THERMOSTABLES

(30) Priority: 20.09.2002 US 251667
(43) Date of publication of application: 29.06.2005
(73) Proprietor: Prokaria ehf., 112 Rejkjavik (IS)
(72) Inventor: AEVARSSON, Arnthor, IS-Hveragerdi 810 (IS); FRIDJONSSON, Olafur, H., IS-Reykjavik 108 (IS); BLONDAL, Thorarinn, IS-210 Gardabaer (IS); SKIRNISDOTTIR, Sigurlaug, IS-112 Reykjavik (IS); HJORLEIFSDOTTIR, Sigridur, IS-110 Reykjavik (IS); HREGGVIDSSON, Gudmundur, Oli, IS-101 Reykjavik (IS); KRISTJANSSON, Jakob, IS-210 Gardabaer (IS)
(74) Representative: Arnason Faktor
(86) International application number: PCT/IS2003/000029
(87) International publication number: WO 2004/027056

(56) References cited:
- WO-A-01/71027
- WO-A-01/79420
- WO-A-94/14972
- US-A- 4 661 450
- US-A1- 2001 031 466
- US-B1- 6 242 189
- DATABASE EMBL [Online] 14 December 2000 (2000-12-14) retrieved from EBI, accession no. AAB46749 XP002269375 -& WO 00 75335 A (DECODE GENETICS EHF) 14 December 2000 (2000-12-14)
- MARIE I. MOSEMAN MCCOY ET AL: "T4 Ribonucleic Acid Ligase Joins Single-Strand Oligo(deoxyribonucleotides)" BIOCHEMISTRY, vol. 19, 1980, pages 635-642, XP002269356
- DATABASE BIOSIS [Online] BIOSCIENCES INFORMATION SERVICE, PHILADELPHIA, PA, US; 1995 VRATSKIKH L V ET AL: "Solid-phase synthesis of oligoribonucleotides using T4 RNA ligase and T4 polynucleotide kinase" Database accession no. PREV199598401154 XP002269357 & BIOCHIMIE (PARIS), vol. 77, no. 4, 1995, pages 227-232, ISSN: 0300-9084

### SEQUENCE LISTING

<110> Aevarsson, Arnthor
   Blondal, Thorarinn
   Fridjonsson, Olafur H.
   Skirnisdottir, Sigurlaug
   Hjorleifsdottir, Sigridur
   Hreggvidsson, Gudmundur O.
   Kristjansson, Jakob K.
<120> METHOD OF USE FOR THERMOSTABLE RNA LIGASES
<130> 2739.2007-000
<140> 10/251,667
   <141> 2002-09-20
<160> 23
<170> FastSEQ for Windows Version 4.0
<210> 1
   <211> 1308
   <212> DNA
   <213> Rhodothermus marinus
<400> 1
   atgaacgtaa aatacccggt tgagtacctt atcgaacacc tgaactcttt tgagtctccg 60
   gaagtagccg tcgaatccct tcgcaaggag gggattatgt gcaaaaaccg gggtgatcta 120
   tacatgttca aatatcacct tggttgtaag tttgataaga tatatcacct tgcctgtcgc 180
   ggggcgattc tccgcaaaac ggatagtggt tggaaagttc tgtcttatcc ctttgacaaa 240
   tttttcaact ggggggaaga actccagccg gaaatcgtaa actattatca gacgcttcgt 300
   tacgcgtctc ccctgaatga aaagcgcaaa gccggtttca tgttcaaact tcccatgaaa 360
   ctggttgaaa agctggatgg tacttgtgtg gttttatatt atgatgaagg gtggaaaatt 420
   cacactcttg ggagtattga cgcaaatgga tccattgtca aaaacggaat ggttaccact 480
   catatggata aaacatatcg agaattgttc tgggaaacct ttgaaaagaa atatccgcct 540
   taccttctct atcatttgaa ctcctcatac tgttacatat ttgaaatggt tcatccggac 600
   gcgcgagtgg tggttcctta tgaggagcca aatatcattc tgatcggtgt gcgttcggtg 660
   gatccggaga agggatattt cgaggtgggt ccctccgaag aagccgtacg cattttcaac 720
   gaaagtggcg gaaaaataaa tcttaagcta ccggctgttc tgtctcaaga gcaaaactat 780
   actctttttc gtgccaatcg ccttcaggaa ctatttgagg aagttacacc gcttttcaaa 840
   agcctgagag acggttatga ggtggtatat gaaggatttg tagccgtaca ggaaattgcc 900
   ccgcgtgttt attaccgcac aaagatcaag cacccggtat atctggagct ccaccggatt 960
   aaaactacaa tcactcctga gaagctcgcc gatctttttc ttgaaaacaa acttgatgat 1020
   tttgtactta ccccggatga acaggaaacc gtgatgaaac tcaaagaaat ttataccgat 1080
   atgcgaaatc agcttgagtc atcttttgat acgatttata aagagatttc cgaacaggtt 1140
   tctccggaag aaaaccccgg agagtttcgc aaaaggttcg ctcttcgact tatggattat 1200
   catgataaaa gttggttttt tgcccgcctt gacggcgacg aagagaaaat gcaaaagtcg 1260
   gaaaagaagc ttctaacgga gagaattgaa aaggggttat ttaaataa              1308
<210> 2
   <211> 435
   <212> PRT
   <213> Rhodothermus marinus
<400> 2
   Met Asn Val Lys Tyr Pro Val Glu Tyr Leu Ile Glu His Leu Asn Ser
   1 5 10 15
   Phe Glu Ser Pro Glu Val Ala Val Glu Ser Leu Arg Lys Glu Gly Ile
   20 25 30
   Met Cys Lys Asn Arg Gly Asp Leu Tyr Met Phe Lys Tyr His Leu Gly
   35 40 45
   Cys Lys Phe Asp Lys Ile Tyr His Leu Ala Cys Arg Gly Ala Ile Leu
   50 55 60
   Arg Lys Thr Asp Ser Gly Trp Lys Val Leu Ser Tyr Pro Phe Asp Lys
   65 70 75 80
   Phe Phe Asn Trp Gly Glu Glu Leu Gln Pro Glu Ile Val Asn Tyr Tyr
   85 90 95
   Gln Thr Leu Arg Tyr Ala Ser Pro Leu Asn Glu Lys Arg Lys Ala Gly
   100 105 110
   Phe Met Phe Lys Leu Pro Met Lys Leu Val Glu Lys Leu Asp Gly Thr
   115 120 125
   Cys Val Val Leu Tyr Tyr Asp Glu Gly Trp Lys Ile His Thr Leu Gly
   130 135 140
   Ser Ile Asp Ala Asn Gly Ser Ile Val Lys Asn Gly Met Val Thr Thr
   145 150 155 160
   His Met Asp Lys Thr Tyr Arg Glu Leu Phe Trp Glu Thr Phe Glu Lys
   165 170 175
   Lys Tyr Pro Pro Tyr Leu Leu Tyr His Leu Asn Ser Ser Tyr Cys Tyr
   180 185 190
   Ile Phe Glu Met Val His Pro Asp Ala Arg Val Val Val Pro Tyr Glu
   195 200 205
   Glu Pro Asn Ile Ile Leu Ile Gly Val Arg Ser Val Asp Pro Glu Lys
   210 215 220
   Gly Tyr Phe Glu Val Gly Pro Ser Glu Glu Ala Val Arg Ile Phe Asn
   225 230 235 240
   Glu Ser Gly Gly Lys Ile Asn Leu Lys Leu Pro Ala Val Leu Ser Gln
   245 250 255
   Glu Gln Asn Tyr Thr Leu Phe Arg Ala Asn Arg Leu Gln Glu Leu Phe
   260 265 270
   Glu Glu Val Thr Pro Leu Phe Lys Ser Leu Arg Asp Gly Tyr Glu Val
   275 280 285
   Val Tyr Glu Gly Phe Val Ala Val Gln Glu Ile Ala Pro Arg Val Tyr
   290 295 300
   Tyr Arg Thr Lys Ile Lys His Pro Val Tyr Leu Glu Leu His Arg Ile
   305 310 315 320
   Lys Thr Thr Ile Thr Pro Glu Lys Leu Ala Asp Leu Phe Leu Glu Asn
   325 330 335
   Lys Leu Asp Asp Phe Val Leu Thr Pro Asp Glu Gln Glu Thr Val Met
   340 345 350
   Lys Leu Lys Glu Ile Tyr Thr Asp Met Arg Asn Gln Leu Glu Ser Ser
   355 360 365
   Phe Asp Thr Ile Tyr Lys Glu Ile Ser Glu Gln Val Ser Pro Glu Glu
   370 375 380
   Asn Pro Gly Glu Phe Arg Lys Arg Phe Ala Leu Arg Leu Met Asp Tyr
   385 390 395 400
   His Asp Lys Ser Trp Phe Phe Ala Arg Leu Asp Gly Asp Glu Glu Lys
   405 410 415
   Met Gln Lys Ser Glu Lys Lys Leu Leu Thr Glu Arg Ile Glu Lys Gly
   420 425 430
   Leu Phe Lys
   435
<210> 3
   <211> 1188
   <212> DNA
   <213> Thermus scotoductus
<400> 3
   atgagctctt tggccccgtg gaggactacc tcctggagcc cgcttgggag cccgcctagc 60
   ctcgaggacg ccctccgcct cgcccggacc accagggcct tcgcggtgcg gcgggacggg 120
   gaggggcggg ccctcgtgac ctacctctac ggcaccccgg agctcttcag cctcccgggg 180
   gcaagggagc tccggggcat cgtctaccgg gaggaggacg gcaccgtgct cagccgcccc 240
   ttccacaagt tcttcaactt cggggagccc ctggccccgg gggaggaggc cttcaaggcc 300
   ttccgggaca gcatggtccc cctcttcgtg gccgagaagg tggacggcta tctggcccag 360
   gcctacctgg acgggggcga gctccgcttc gcctcccggc actccctgaa cccgcccctg 420
   gtaggggcgc tcctgcgcaa ggccgtggac gaggaggcga tggctcgcct ggggaagctc 480
   ctcgccgccg aagggggaag gtggacggcg cttttggagg tggtggaccc cgaggccccg 540
   gtgatggtgc cctaccagga gcccggggtc tacctcctgg ccctgaggag catcggggag 600
   gggcactacc tcctccccgg ggtccacttc cccctgcccg aggcgctccg ctacgtgcgg 660
   tgggagccca ggatggattt tgacccccac cgcttccggg gggagatcag ggacctccag 720
   ggggtggagg ggtacgtggt cacggacggg gcagagttcg tcaagttcaa gacagggtgg 780
   gccttccgcc tcgcccgctt cctcatggac ccggaggggg tgttcctcga ggcctacgcc 840
   gaggaccgcc tagacgacct cgtgggcgcc ctcgcggggc gggaggacct cctgcgggcg 900
   gtggccaggg cccaggacta cctcgccggg ctctacgggg aagcggtggg cgcgggggac 960
   gccctgaggc ggatgggcct cccccggaag gaggcctggg cccgggtgca ggaggaggcc 1020
   gggcgctggg gcggcttcgc ccccgcctac gcccgggcgg ccatggcggc ctacgagggg 1080
   ggagaggcgc gcgaggcctt cctggtggag ctcaggaaga ggagcgcgcg gaaggccctc 1140
   gaggcgctcc acctcttccc ccgggtgggc ggggagctta gggggtga            1188
<210> 4
   <211> 395
   <212> PRT
   <213> Thermus scotoductus
<400> 4
   Met Ser Ser Leu Ala Pro Trp Arg Thr Thr Ser Trp Ser Pro Leu Gly
   1               5                   10                  15
   Ser Pro Pro Ser Leu Glu Asp Ala Leu Arg Leu Ala Arg Thr Thr Arg
               20                  25                  30
   Ala Phe Ala Val Arg Arg Asp Gly Glu Gly Arg Ala Leu Val Thr Tyr
           35                  40                  45
   Leu Tyr Gly Thr Pro Glu Leu Phe Ser Leu Pro Gly Ala Arg Glu Leu
       50                  55                  60
   Arg Gly Ile Val Tyr Arg Glu Glu Asp Gly Thr Val Leu Ser Arg Pro
   65                  70                  75                  80
   Phe His Lys Phe Phe Asn Phe Gly Glu Pro Leu Ala Pro Gly Glu Glu
                   85                  90                  95
   Ala Phe Lys Ala Phe Arg Asp Ser Met Val Pro Leu Phe Val Ala Glu
               100                 105                 110
   Lys Val Asp Gly Tyr Leu Ala Gln Ala Tyr Leu Asp Gly Gly Glu Leu
           115                 120                 125
   Arg Phe Ala Ser Arg His Ser Leu Asn Pro Pro Leu Val Gly Ala Leu
       130                 135                 140
   Leu Arg Lys Ala Val Asp Glu Glu Ala Met Ala Arg Leu Gly Lys Leu
   145                 150                 155                 160
   Leu Ala Ala Glu Gly Gly Arg Trp Thr Ala Leu Leu Glu Val Val Asp
                   165                 170                 175
   Pro Glu Ala Pro Val Met Val Pro Tyr Gln Glu Pro Gly Val Tyr Leu
               180                 185                 190
   Leu Ala Leu Arg Ser Ile Gly Glu Gly His Tyr Leu Leu Pro Gly Val
           195                 200                 205
   His Phe Pro Leu Pro Glu Ala Leu Arg Tyr Val Arg Trp Glu Pro Arg
       210                 215                 220
   Met Asp Phe Asp Pro His Arg Phe Arg Gly Glu Ile Arg Asp Leu Gln
   225                 230                 235                 240
   Gly Val Glu Gly Tyr Val Val Thr Asp Gly Ala Glu Phe Val Lys Phe
                   245                 250                 255
   Lys Thr Gly Trp Ala Phe Arg Leu Ala Arg Phe Leu Met Asp Pro Glu
               260                 265                 270
   Gly Val Phe Leu Glu Ala Tyr Ala Glu Asp Arg Leu Asp Asp Leu Val
           275                 280                 285
   Gly Ala Leu Ala Gly Arg Glu Asp Leu Leu Arg Ala Val Ala Arg Ala
       290                 295                 300
   Gln Asp Tyr Leu Ala Gly Leu Tyr Gly Glu Ala Val Gly Ala Gly Asp
   305                 310                 315                 320
   Ala Leu Arg Arg Met Gly Leu Pro Arg Lys Glu Ala Trp Ala Arg Val
                   325                 330                 335
   Gln Glu Glu Ala Gly Arg Trp Gly Gly Phe Ala Pro Ala Tyr Ala Arg
               340                 345                 350
   Ala Ala Met Ala Ala Tyr Glu Gly Gly Glu Ala Arg Glu Ala Phe Leu
           355                 360                 365
   Val Glu Leu Arg Lys Arg Ser Ala Arg Lys Ala Leu Glu Ala Leu His
       370                 375                 380
   Leu Phe Pro Arg Val Gly Gly Glu Leu Arg Gly
   385                 390                 395
<210> 5
   <211> 17
   <212> DNA
   <213> Artificial Sequence
<220>
   <223> Synthesized primer
<400> 5
   agcgtttttt tcgctaa 17
<210> 6
   <211> 93
   <212> DNA
   <213> Artificial Sequence
<220>
   <223> Synthesized primer
<400> 6
   cggcgaattc tttatgggtc cggaaaccct gtgcggtgct gaactggttg atgctctgca 60
   attcgtttgc ggtgatcgtg gtttctactt caa                               93
<210> 7
   <211> 40
   <212> DNA
   <213> Artificial Sequence
<220>
   <223> Synthesized primer
<221> misc_feature
   <222> 35
   <223> n = A,T,C or G
<221> misc_feature
   <222> 35
   <223> n = A,T,C or G
<400> 7
   gatatttaat atgtttagct gcatcaattc kraanccrtc 40
<210> 8
   <211> 23
   <212> DNA
   <213> Artificial Sequence
<220>
   <223> Synthesized primer
<400> 8
   aagggtgcca acctcttcaa ggg 23
<210> 9
   <211> 20
   <212> DNA
   <213> Artificial Sequence
<220>
   <223> Synthesized primer
<400> 9
   cttgaagagg ttggcaccct 20
<210> 10
   <211> 61
   <212> DNA
   <213> Artificial Sequence
<220>
   <223> Synthesized primer
<400> 10
   caaaccgacc ggttacggtt cttcttctcg tcgtgctccg caaaccggta tcgttgatga 60
   a 61
<210> 11
   <211> 86
   <212> DNA
   <213> Artificial Sequence
<220>
   <223> Synthesized primer
<400> 11
   tgctgcttcc gttcttgcga tctgcgtcgt ctggaaatgt actgcgctcc gctgaaaccg 60
   gctaaatctg cttaaggatc ccggcg 86
<210> 12
   <211> 20
   <212> DNA
   <213> Artificial Sequence
<220>
   <223> Synthesized primer
<400> 12
   ccgggatcct taagcagatt 20
<210> 13
   <211> 21
   <212> DNA
   <213> Artificial Sequence
<220>
   <223> Synthesized primer
<400> 13
   tcatcaacga taccggtttg c 21
<210> 14
   <211> 26
   <212> DNA
   <213> Artificial Sequence
<220>
   <223> Synthesized primer
<400> 14
   ggcgaattct ttatgggtcc ggaaac 26
<210> 15
   <211> 21
   <212> DNA
   <213> Artificial Sequence
<220>
   <223> Synthesized primer
<400> 15
   accgaccggt tacggttctt c 21
<210> 16
   <211> 240
   <212> DNA
   <213> E. Coli
<400> 16
   cggcgaattc tttatgggtc cggaaaccct gtgcggtgct gaactggttg atgctctgca 60
   attcgtttgc ggtgatcgtg gtttctactt caacaaaccg accggttacg gttcttcttc 120
   tcgtcgtgct ccgcaaaccg gtatcgttga tgaatgctgc ttccgttctt gcgatctgcg 180
   tcgtctggaa atgtactgcg ctccgctgaa accggctaaa tctgcttaag gatcccggcg 240
<210> 17
   <211> 44
   <212> DNA
   <213> Artificial Sequence
<220>
   <223> Synthesized primer
<400> 17
   cgacuggagc acgaggacac ugacauggac ugaaggagua gaaa 44
<210> 18
   <211> 23
   <212> DNA
   <213> Artificial Sequence
<220>
   <223> Synthesized primer
<400> 18
   cgactggagc acgaggacac tga 23
<210> 19
   <211> 26
   <212> DNA
   <213> Artificial Sequence
<220>
   <223> Synthesized primer
<400> 19
   ggacactgac atggactgaa ggagta 26
<210> 20
   <211> 22
   <212> DNA
   <213> Artificial Sequence
<220>
   <223> Synthesized primer
<400> 20
   gacctggccg tcaggcagct cg 22
<210> 21
   <211> 123
   <212> DNA
   <213> Artificial Sequence
<220>
   <223> Synthesized Primer
<400> 21
   atgggtccgg aaaccctgtg cggtgctgaa ctggttgatg ctctgcaatt cgtttgcggt 60
   gatcgtggtt tctacttcaa gttcttcttc tcgtcgtgct ccgcaaaccg gtatcgttga 120
   tga                                                               123
<210> 22
   <211> 132
   <212> DNA
   <213> Artificial Sequence
<220>
   <223> Synthesized Primer
<400> 22
   ccgaccggtt acggttcttc ttctcgtcgt gctccgcaaa ccggtatcgt tgatgaatgc 60
   tgcttccgtt cttgcgatct gcgtcgtctg gaaatgtact gcgctccgct gaaaccggct 120
   aaatctgctt aa                                                     132
<210> 23
   <211> 155
   <212> DNA
   <213> Artificial Sequence
<220>
   <223> Synthesized Primer
<400> 23
   atgggtccgg aaaccctgtg cggtgctgaa ctggttgatg ctctgcgatt cgtttgcggt 60
   gatcgtggtt tctacttcaa tgctgcttcc gttcttgcga tctgcgtcgt ctggaaatgt 120
   actgcgctcc gctgaaaccg gctaaatctg cttaa                            155

## Description

### Background of the invention

RNA ligase is abundant in T4-infected cells and has been purified in high yields. Bacteriophage T4 RNA ligase catalyzes the ATP-dependent ligation of a 5'- phosphoryl-terminated nucleic acid donor (*i.e.* RNA or DNA) to a 3'-hydroxyl-terminated nucleic acid acceptor. The reaction can be either intramolecular or intermolecular, *i.e.,* the enzyme catalyzes the formation of circular DNA/RNA, linear DNA/RNA dimers, and RNA-DNA or DNA-RNA block co-polymers. The use of a 5'-phosphate, 3'-hydroxyl terminated acceptor and a 5'-phosphate, 3'-phosphate terminated donor limits the reaction to a unique product. Thus, RNA ligase can be an important tool in the synthesis of DNA of defined sequence (McCoy and Gumport, Biochemistry 19:635-642 (1980), Sugion, A. et al., J. Biol. Chem. 252:1732-1738 (1977)).

The practical use of T4 RNA ligase has been demonstrated in many ways. Various ligation-anchored PCR amplification methods have been developed, where an anchor of defined sequence is directly ligated to single strand DNA (following primer extension, *e.g.* first strand cDNA). The PCR resultant product is amplified by using primers specific for both the DNA of interest and the anchor (Apte, A. N., and P. D. Siebert, BioTechniques. 15:890-893 (1993); Troutt, A. B., et al., Proc. Natl. Acad. Sci. USA. 89: 9823-9825 (1992); Zhang, X. H., and V. L. Chiang, Nucleic Acids Res. 24:990-991(1996)). Furthermore, T4 RNA ligase has been used in fluorescence-, isotope- or biotin- labelling of the 5'-end of single stranded DNA/RNA molecules (Kinoshita Y., et al., Nucleic Acid Res. 25: 3747-3748 (1997)), synthesis of circular hammer head ribozymes (Wang, L., and D. E. Ruffner. Nucleic Acids Res 26: 2502-2504 (1998)), synthesis of dinucleoside polyphosphates (Atencia, E. A., et al. Eur. J. Biochem. 261: 802-811 (1999)), and for the production of composite primers (Kaluz, S., et al., BioTechniques, 19: 182-186 (1995)).

The use of thermostable enzymes has revolutionized the field of recombinant DNA technology. Thermostable enzymes, foremost DNA polymerases used in amplification of DNA, are of great importance in the research industry today. In addition, thermophilic enzymes are also used in commercial settings (*e.g.*, proteases and lipases used in washing powder, hydrolidic enzymes used in bleaching). Identification of new thermophilic enzymes will facilitate continued DNA research as well as assist in improving commercial enzyme-based products (Wang,T, et al, J. Biol. Chem. 278: 29454-29462 (2003)).

RNA ligase activity was originally identified as activity induced through infection of *E. coli* by T-even bacteriophages (Silber, R., et al. Proc, Natl. Acad. USA, 69:3009-3013 (1972)). The RNA ligase from bacteriophage T4 is the product of gene 63 (Snopek, T.J., et al., Proc. Natl. Acad. Sci. USA, 74:3355-3359 (1977)) and is the best characterized RNA ligase of very few known homologous RNA ligases.

The properties of RNA ligase from bacteriophage T4 have been extensively studied including its ability to catalyze reactions with various substrates (for review see Gumport and Uhlenbeck, in "Gene Amplification and Analysis", Vol. II: Analysis of Nucleic Acid Structure by Enzymatic Methods, Chirikjian and Papas, eds. Elsevier North Holland, Inc. (1980)). In general, the T4 RNA ligase catalyzes the ATP-dependent formation of a phosphodiester bond between a 3'-hydroxyl nucleic acid acceptor and a 5'-phosphate nucleic acid donor. T4 RNA ligase can use single-stranded nucleic acids as substrates and does not require a complementary template strand to align donor phosphates with acceptor hydroxyls.

5'-phosphorylated oligonucleotides are appropriate donors for the ATP-dependent T4 RNA ligase reaction but the minimal donor is a nucleoside 3', 5'-biphosphate (pNp). Suitable minimal acceptor molecules for the T4 RNA ligase reaction are trinucleoside biphosphates.

T4 RNA ligase is adenylated in the presence of ATP thereby forming a covalent
bond between AMP and a lysyl residue. The adenyl group can then be transferred from the enzyme to the 5'-phosphate of a nucleic acid. T4 RNA ligase can accept ATP analogs and adenylate nucleic acid substrates with the nucleotide analog. Additionally, T4 RNA ligase is able to catalyze a class of reactions that do not require ATP. The enzyme is able to accept a wide variety of ADP derivatives as substrates and join the extra moiety of the ADP derivative to a nucleic acid acceptor with the elimination of AMP. Examples of ADP derivative of this type include ADP-riboflavin and ADP-hexylamine-biotin (see further Gumport and Uhlenbeck, in "Gene Amplification and Analysis," Vol. II: Analysis of Nucleic Acid Structure by Enzymatic Methods, Chirikjian and Papas, eds. Elsevier North Holland, Inc (1980)).

T4 RNA ligase has a greater affinity for RNA than DNA. Although RNA and
DNA are equally reactive as donors, DNA is a less efficient acceptor than RNA. The efficiency of the RNA ligase reaction is also affected by the nucleotide composition of the acceptor and oligo(A) seems to function as the most efficient acceptor. RNA molecules are good acceptors for the T4 RNA ligase.

The 5'-phosphate of yeast tRNA^{Phe} is a very poor donor for T4 RNA ligase,
indicating that secondary or tertiary structures in the RNA donor molecule is inhibiting the ligase reaction. In contrast, DNA restriction fragments are good donors and little difference is observed between DNA restriction fragments with 5'-staggered ends and blunt ends. On the other hand, the presence of a secondary structure of an RNA acceptor molecule has little effect on the reaction. The 5'-cap (m⁷ G^{5'} ppp-^{5'}), which is normally formed through addition of methylated guanosine to the 5' end of eukaryotic mRNA, is neither an acceptor nor a donor for the T4 RNA ligase reaction (Gumport and Uhlenbeck in "Gene Amplification and Analysis," Vol. II: Analysis of Nucleic Acid Structure by Enzymatic Methods, Chirikjian and Papas, eds. Elsevier North Holland, Inc. (1980)).

T4 RNA ligase is a versatile enzyme with new properties continuing to be discovered. T4 RNA ligase has recently been shown to be able-to catalyze the reaction between a 3'-phosphate donor and 5'-hydroxyl acceptor in addition to previously characterized reaction of 5'-phosphate donor and 3'-hydroxyl acceptor (U.S. Patent No. 6,329,177). T4 RNA ligase has also been shown to have template-mediated DNA ligase activity. Reportedly, the T4 RNA ligase can ligate ends of DNA strands hybridized to RNA, even more efficiently than T4 DNA ligase (U.S. Patent No. 6,368,801).

Enzymes having RNA ligase activity, but which are apparently not related to the T4 RNA ligase and other homologous proteins in the small family of viral RNA ligases, have been identified. These enzymes have relatively strict substrate specificity whereas the activity of T4 RNA ligase is the most general RNA joining activity known.

The RNA ligases of T-even bacteriophages apparently belong to a very small family of homologous enzymes. However, it is likely that this is a subfamily of much larger superfamily of ligases including DNA ligases and mRNA capping enzymes (Shuman, S. and B. Schwer, Mol. Microbiol., 17:405-410 (1995); Timson, D.J., et al., Mut. Res., 460:301-318 (2000)). Until recently, the only clearly identifiable relatives of T4 RNA ligase, found through sequence comparisons (ex. with BLAST software), were from bacteriophage RB69 and *Autographa californica nuclearpolyhedrosis* virus. As disclosed in a previous patent applications (U.S. Patent Application No. 09/585,858; PCT Application No. PCT/IB00/00893; European Application No. 00938977.6), the discovery of a bacteriophage from the thermophilic bacterial host *Rhodothermus marinus* and the subsequent genome sequencing identified a new RNA ligase homologous to T4 RNA ligase according to the amino acid sequence of the predicted gene product of a particular open reading frame. Accordingly, these ligases seem to form a family of viral RNA ligase currently only comprising ligases from bacteriophage T4 (and by analogy other T-even bacteriophages), bacteriophage RB69, *Autographa california* nuclearpolyhedrosis virus and the ligases of the instant invention from bacteriophages RM378 and TS2126. The ligases of the instant invention are the only known members of the family from a thermophilic source.

### Summary of the Invention

This publication pertains to thermostable RNA ligases derived from thermophilic microorganisms, such as bacteria, archea and bacteriophage that infect thermophilic bacteria, and their use in various applications including nucleotide labeling, oligonucleotide synthesis, gene synthesis, gene amplification and amplification of mRNA synthesis of cDNA. The publication relates to RNA ligase isolates from bacteriophage that infect the thermophilic bacteria *Rhodothermus marinus* and *Thermus scotoductus.* These thermophilic RNA ligases can replace T4 RNA ligase in methodologies that utilize T4 RNA ligase.

The invention relates to a method of ligating a nucleotide or nucleotide analog or nucleic acid containing nucleotides or nucleotide analogs with another nucleotide or nucleotide analog or a nucleic acid containing nucleotides or nucleotide analogs, the method comprising contacting said nucleotides, nucleotide analogs or nucleic acids with an isolated thermostable RNA ligase, wherein the ligase catalyzes a reaction of ligation of the nucleotides, nucleotide analogs or nucleic acids wherein the ligation is performed at a temperature of at least 50°C. The thermostable RNA ligase can be derived from a thermostable bacteriophage; the nucleic acids can be RNA or DNA; the RNA or DNA can be single stranded; and the nucleotide analogs contain modified bases, modified sugars and/or modified phosphate groups. The RNA ligase is selected from the group comprising: a RNA ligase obtained from a bacteriophage infecting a thermophilic bacteria; a polypeptide comprising the amino acid sequence of SEQ ID NO: 2 or SEQ ID NO: 4; a polypeptide encoded by a nucleic acid comprising the sequence of SEQ ID NO: 1 or SEQ ID NO: 3; a polypeptide having at least 30% sequence identity with the amino acid sequence of SEQ ID NO: 2 or SEQ ID NO: 4; or a fragment or derivative thereof.

A method of forming a phosphodiester bond between a 3' hydroxyl nucleic acid acceptor and a 5' phosphate nucleic acid donor is described, comprising: contacting a 3' hydroxyl nucleic acid acceptor; and a 5' phosphate nucleic acid donor with a thermostable RNA ligase and forming a phophodiester bond between the nucleic acids.

A method of synthesizing an oligonucleotide polymer by repeating cycles of combining a primer oligonucleotide and a blocked oligonucleotide is described, comprising: a) combining the primer oligonucleotide and an oligonucleotide blocked at the 3' or 5' end in the presence of a thermostable RNA ligase, thereby forming an extended primer with a blocked 3' or 5' end; b) enzymatically removing the blocked phosphate group at the 3' or 5' end or enzymatically adding a phosphate group to the 5' end of the extended primer; and c) repeating a) and b) using the extended primer from b) as the primer for a) wherein an oligonucleotide polymer is formed. The formed oligonucleotide polymer comprises a gene or a part of a gene coding for a polypeptide.

The publication additionally pertains to a method for synthesizing a recombinant gene product, comprising: providing an array of immobilized oligonucleotides comprising predetermined areas on a surface of a solid support, each area having immobilized thereon copies of an oligonucleotide; hybridizing to said immobilized oligonucelotides first single stranded terminal regions of first nucleic acid strands to be ligated; and ligating with a thermostable RNA ligase, the hybridized first end of a first nucleic acid and a second nucleic acid.

The publication also pertains to a method of detecting nucleic acids, comprising:
contacting a first probe, a second probe, a target nucleic acid sample and a thermostable RNA ligase, wherein the first probe and the second probe hybridize to the target nucleic acid sample such that the 5' end of the first probe and the 3' end of the second probe are adjacent and can be ligated; and incubating the first probe, second probe, target sample and RNA ligase under conditions that promote hybridization of the probes to the target sequence and that promote ligation of the probes are ligated if the target sequence is present in the target sample. At least the 5' terminal nucleotide of the first probe and the 3' terminal nucleotide of the second probe are deoxyribonucleotides.

The publication further relates to method of amplifying nucleic acids, comprising: a) contacting a nucleic acid containing sample, wherein the sample comprises a pool of mRNAs having a poly-A tail, with i) an oligonucleotide with a 5' end and a 3' end comprising an oligo-dT sequence at the 3' end, a promoter sequence recognized by a RNA polymerase at the 5' end and a transcription initiation region located between the oligo-dT sequence and the promoter sequence wherein the oligonucleotide is blocked at the 3' end to prohibit extension, ii) an enzyme having reverse transcription activity which forms a double stranded promoter-primer sequence, iii) at least one enzyme having RNase H activity, iv) an enzyme having RNA polymerase activity, and v) sufficient amounts of dNTPs and rNTPs; b) maintaining the resulting reaction mixture under appropriate conditions for a sufficient amount of time for enzymatic activity, such that antisense RNA is formed in the absence of cDNA intermediates; c)contacting the multiple copies of RNA with i) a thermostable RNA ligase, ii) a double stranded DNA complex comprising a double stranded DNA promoter sequence, wherein each strand contains a 5' end and a 3' end, the promoter sequence recognizable by a RNA polymerase wherein one strand of said complex has a stretch of RNA attached to the 5' end thereof, iii) an enzyme having RNA polymerase activity, and iv) sufficient amounts of dNTPs and rNTPs; and d) maintaining the resulting reaction mixture under appropriate conditions for a sufficient amount of time for the enzymatic processes to occur.

The publication pertains to a method for selectively isolating total cell mRNA, comprising: contacting a cell lysate comprising total cell mRNA and non-isolated ribosome with a thermostable RNA ligase under conditions wherein the ligase adds a 3' label to the total cell mRNA to form modified total cell mRNA; and isolating the modified total cell mRNA.

The publication relates to a method for synthesizing a repeat region of an oligonucleotide having a defined sequence, the repeat region including a repeated nucleotide that appears more than once in succession, comprising: a) ligating an oligonucleotide primer to a 3'-phosphate-blocked repeated nucleotide to form a 3'-phosphate-blocked primer; b) removing the 3'-phosphate blocking group from the 3'-phosphate-blocked primer using a 3'-phosphatase enzyme, thereby making a deblocked primer without removing the 3'-phosphate blocking group from unreacted 3'-phosphate-blocked repeated nucleotide; and c) repeating steps (a) and (b) using unreacted 3'-phosphate-blocked repeated nucleotide from step (b) as the 3'-phosphate-blocked repeated nucleotide of step (a) and the deblocked primer product of step (b) as the oligonucleotide primer of step (a) without prior separation of the unreacted 3'-phosphate-blocked repeated nucleotide from the deblocked primer product, whereby the cycles are repeated to form an oligonucleotide having a defined sequence.

Additionally, the publication relates to a method for insertion of a single-stranded RNA sequence into a cloning vector, comprising: ligating in the presence of a thermostable RNA ligase, both termini of the single-stranded RNA sequence with a linear double-stranded cloning vector having single-stranded termini complementary to both termini of the single-stranded RNA sequence to form an annealed product, in which the complementary termini of the single-stranded RNA sequence is formed by attaching oligonucleotide linkers to the RNA sequence with a thermostable RNA ligase.

Also described herein is a method for forming a library of DNA sequences, comprising: a) forming a library of target RNA fragments by contacting multiple copies of non-denatured target RNA sequences with a library of random oligonucleotides in the presence of a hydrolytic agent under conditions where a subgroup of the library of random oligonucleotides hybridize to the target RNA, whereupon the hydrolytic agent hydrolyzes the target RNA at a site near the 5' end of each hybridized random oligonucleotide, and wherein the 3' ends of each fragment contains the entire sequence to which a random oligonucleotide in the subgroup hybridized; and b) forming a library of templates for primer extension from the library of target RNA fragments; and forming a library of DNA sequences that are complementary to the target RNA fragments from the library of templates for primer extension by attaching a nucleic acid primer complement sequence to the 3' end of each target RNA fragment with a thermostable RNA ligase.

The publication further pertains to a method for amplifying a 5' end region of target mRNA, comprising: a) dephoshorylating mRNA molecules with a free phosphate group at the 5'-end; b) removing the 5'-cap on full-length mRNAs; c) ligating linkers to the 5'-end of decapped mRNA molecules using a thermostable RNA ligase; d) synthesize cDNA using reverse transcriptase; and e)amplifying the cDNA by PCR.

The publication relates to a method of amplifying mRNA, comprising: a) synthesizing cDNA, wherein the first strand of cDNA is synthesized by reverse transcription of target mRNA using a 5' end-phosphorylated RT-primer that is specific for the target RNA wherein a hybrid DNA-RNA is generated; b) degrading the hybrid DNA-RNA by treatment with at least one enzyme having RNase H activity to remove RNA; c) circularizing the single-stranded cDNA or form concatemers with a thermostable RNA ligase; and d) amplifying DNA by PCR using specific primers that are complementary to known sequences.

Also described herein is a method of amplifying nucleic acids with a single primer, comprising: a) hybridizing a mixture of nucleic acids with a degenerate or non- degenerate primer targeted to a single region in a target sequence; b) synthesizing single-stranded DNA complementary to a region of said target sequence, said synthesis being primed by said degenerate or non-degenerate primer and catalyzed by a DNA polymerase or a reverse transcriptase, thereby performing linear amplification of said target sequence by repeated thermal cycling; c) providing a second primer site to said single-stranded DNA by ligating an oligonucleotide to its 3'end, wherein the ligation is catalyzed by a thermostable RNA; d) amplifying the single-stranded DNA using a primer pair wherein a first primer comprises at least a part of the degenerate or non-degenerate primer sequence, or wherein a first primer is targeted to a region downstream to the degenerate or non-degenerate primer sequence, and the second primer is complementary to the 3' primer site of step (c). The single stranded DNA synthesized in step b) is purified.

Also described is a method for sequencing oligonucleotides, comprising contacting a target oligonucleotide with a thermostable RNA ligase under conditions wherein the ligase adds an auxiliary oligonucleotide to the 3' end of the target oligonucleotide; and sequencing the oligonucleotide.

Methods utilizing the thermophilic RNA ligases are performed at temperatures of at least 50°C, such as at least 55°C, such as at least 60°C, such as at least 65°C, such as at least 70°C. A preferred method of the publication utilizes thermophilic RNA ligases at temperatures in the range of about 50°C to about 75°C.

Although the T4 RNA ligase can be utilized for many useful applications, it is only functional up to about 40°C. The thermostable RNA ligase enzymes as described herein have advantageous properties, such as different substrate specificity, in particular, increased activity to ssDNA as compared to T4 ligase and prevention of undesirable secondary structures due to the enzyme's ability to function at higher temperatures.

### Brief description of the drawings

The foregoing and other objects, features and advantages of the invention will be apparent from the following more particular description of preferred embodiments of the invention, as illustrated in the accompanying drawings.
FIG. 1 is a schematic representation of the single primer method where an adaptor sequence is ligated to the 3' end of the single stranded copy-DNA to provide a second primer site for the second amplification step.
FIG. 2 is a gel depicting screening of amylases with random gene retrieval by single primer method where different RNA ligases were used. Lane 1: 1 Kb ladder (New England Biolabs), lane 2: T4 RNA ligase and PCR with primer Am508, lane 3: T4 RNA ligase and PCR with primer oli11, lane 4: T4 RNA ligase and PCR with primers Am508 and oli11, lane 5: RM378 RNA ligase and PCR with primer Am508, lane 6: RM378 RNA ligase and PCR with primer, oli11, line 7: RM378 RNA ligase and PCR with primers Am508 and oli11 and line 8: 1 Kb ladder (New England Biolabs).
FIG. 3 is a gel which depicts synthesis of IGFA by T4 RNA ligase and RM378 RNA ligase. Lane 1: 100 bp ladder (New England Biolabs), lane 2: T4 RNA ligase and PCR with primer IGFA-r and IGFA-f giving the whole gene, lane 3: T4 RNA ligase and PCR with primer IGFA-r and IGFA-2f giving the oligoA (partial gene), lane 4: RM378 RNA ligase and PCR with primer IGFA-r and IGFA-f giving the whole gene, lane 5: RM378 RNA ligase and PCR with primer IGFA-r and IGFA-2f giving the oligoA (partial gene) and lane 6: 100 bp ladder (New England Biolabs).

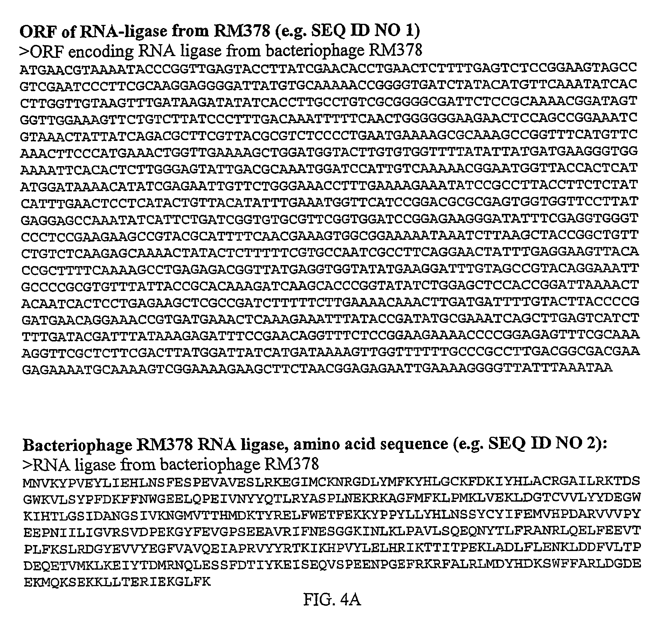

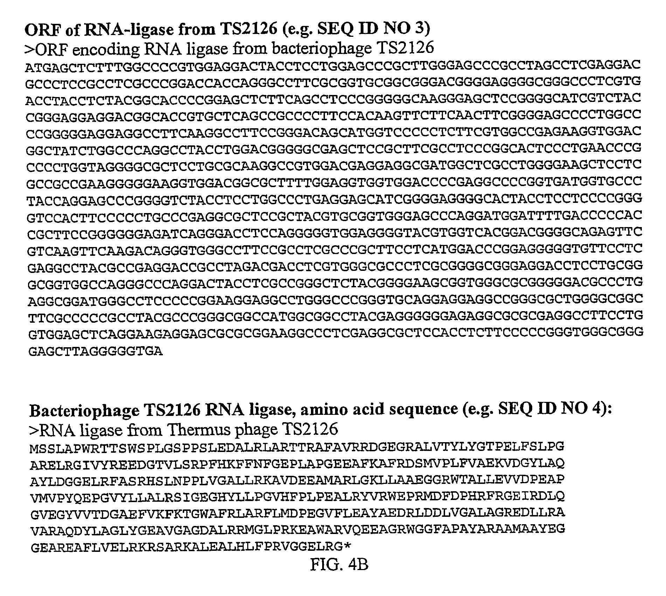

FIG. 4A is a nucleic acid sequence of RM 378 RNA ligase (SEQ ID NO: 1) and the amino acid sequence of RM 378 RNA ligase (SEQ ID NO: 2) and FIG. 4B is the nucleic acid sequence of TS2126 RNA ligase (SEQ ID NO: 3) and the amino acid sequence of TS2126 RNA ligase (SEQ ID NO: 4)
FIG. 5 is a plot which depicts the relative activity of RM378 RNA ligase as a function of pH. MOPS buffer is shown in diamonds and TRIS HCl buffer is shown in squares.
FIG. 6 is a plot of temperature profiles for the activity of RM378 RNA ligase and T4 RNA ligase.
FIG. 7 is a plot which depicts the relative activity of RM378 RNA ligase (squares) and T4RNA ligase (diamonds) after incubation at various temperatures.
FIG. 8 is a plot which depicts the percentage of activity of RM378 RNA ligase at 60°C over time in MOPS buffer without template.
FIG. 9 is a plot depicting a time curve showing RNA ligase activity on rA20 template (10µM) using 0.2 T4 RNA ligase enzyme and 0.2 µg and 0.4 µg RM378 RNA ligase enzyme. The reactions were done at 37°C and 64°C for T4 and RM378 RNA ligase, respectively.
FIG. 10 is a plot which depicts the percent ligation of total DNA substrate 5'[³²P]dA₂₀ versus time for T4 RNA ligase and RM378 RNA ligase.
FIG. 11 is a bar graph which depicts ligation of 90mer ssDNA substrate using RM378 RNA ligase and T4 RNA ligase. As seen the RM 378 RNA ligase ligation is very efficient for long DNA oligos (50-80 %) even without the PEG6000 for RM378 RNA ligase but low for T4 RNA ligase (3% without PEG6000 and 14% with PEG6000).
FIG. 12 is a bar graph which depicts inter-molecular ligation of DNA donors (dA10-dideoxy or -NH₃⁺) to dephosphorylated ssDNA (dA10) oligo or dephosphorylated 17 n.t. RNA (agcgtttttttcgctaa oligo; SEQ ID NO: 5). RM378 RNA ligase shows 20 and 25 % ligation when ligating ³²PdA10dd to dA10 without PEG, with PEG, respectively. Ligation dA10-NH₃⁺to DNA and RNA oligos shows 27% and 28% ligation, respectively. T4 RNA ligase shows little activity ligating dA10-NH3 to DNA acceptor but high activity to RNA acceptor (6% and 60% ligation, respectively).
FIG. 13 is a gel depicting the results from control PCR in RLM-RACE using RM378 RNA ligase, and using primers GeneRacer 5' nested primer and B1 control primer. Lane 1: PCR product; Lane 2: Lambda HindIII marker. A PCR product of about 800-900 bp in size is seen, the expected product is 858 bp.
FIG. 14 is an activity profile of the TS2126 RNA ligase as a function of pH. The pH optimum of the ligase is approximately 7.5.
FIG. 15 is an activity profile of TS2126 profile as a function of temperature. The ligase shows over 90% activity in the 55 - 70°C range.
FIG. 16 is a thermostability profile of the TS2126 ligase. After incubation for 1 hour at various temperatures, the activity was determined. The enzyme is stable at 50°C, loses 25% activity at 60°C, and is inactivated by incubation at 70°C.
FIG. 17 shows the effect of divalent cations on the ligation reaction. Divalent cations are necessary for the reaction, and Mn²⁺ gives slightly more activity than Mg²⁺.
FIG. 18 shows the effect of ATP on TS2126 ligase activity. The enzyme shows over 90% activity in 0.1 - 2.5 mM ATP, but is inhibited by high concentrations of ATP.
FIG. 19 is a phosphatase resistance test comparing TS2126 and T4 RNA ligases. The TS2126 ligase shows over 10-fold higher specific activity than the T4 ligase.
FIG. 20 is a profile of the effect of protein concentration on TS2126 ligase activity. The concentration of the ligase was varied and the phosphatase resistance determined. Over 90% ligation was reached at 0.1 mg/ml protein concentration.
FIG. 21 shows results from an experiment wherein TS2126 was used in an RLM-RACE application. (A) Lane 3: TS RNA ligase; Lane 4: T4 RNA ligase; Lane 5: cDNA control; Lane 6: PCR control. (B) Lanes 9-12: Beta actin cDNA controls.
FIG. 22 shows results from an inverse beta actin PCR. Lane 1: control; Lane 2: TS ligase; Lane 3: T4 ligase.
FIG. 23 shows results of an intra-molecular ligation using a 5'P-d(N₂₂) oligonucleotide, varying the protein concentration from 0.1 - 0.3 mg/ml and the oligonucleotide concentration from 0.5 - 5 µM.
FIG. 24 shows results of a comparison experiment in which T4 ligase was used for intramolecular ligation of the same 5'P-d(N₂₂) oligonucleotide as in FIG. 23, at a protein concentration of 0.28 mg/ml in 0.02 mM ATP, 1 mM hexamine cobalt chloride and 10, 20 and 30% PEG6000 at 17°C and 22°C.
FIG. 25 shows results of an internal PCR reaction of ligation products from IGFA synthesis performed as in Example 9. PCR was performed using Fwd2 and Rev primers, and the ligation reactions were performed using I3 and I2 oligonucleotides using 1µg TS2126 RNA ligase and 3µg RM378 ligase. Lane 1: Markers; Lane 2: TS2126 ligase; Lane 3: RM378 ligase; Lane 4: negative control; Lane 5: Markers.

### Detailed description of the Invention

A description of preferred embodiments of the invention follows.

This invention pertains to methods of using thermostable RNA ligases, in particular ligases that are derived from bacteriophages which infect thermophilic bacteria. In certain aspects, RNA ligases derived from bacteriophages that infect the bacteria, *Rhodothermus marinus* (RM378 RNA ligase) and *Thermus scotoductus* (TS2126 RNA ligase) are utilized in methods of manipulating nucleic acids.

These enzymes are functionally analogous to the widely used T4 RNA ligase yet provide several advantageous properties over conventional mesophilic enzymes. These include, but are not limited to:
- different substrate specificity, in particular, increased activity to DNA;
- prevention of undesirable secondary structures due to the enzymes ability to function at high temperatures;
- more reliable applications, due to fewer complications with respect to competing enzymatic activities, undesirable substrate structure, etc.

Interestingly, these ligases have about 30% sequence identity to each other and to the T4 RNA ligase. The various applications disclosed herein illustrate the wide applicability of thermostable RNA ligases, which benefit from the advantageous properties of these enzymes.

Thus, the publication is directed to methods and processes using thermostable RNA ligases, in particular RM378 RNA ligase and TS2126 RNA ligase and other substantially similar enzymes for ligation of nucleic acids, such as ribonucleic acids, deoxyribonucleic acids and nucleic acid analogs. The present publication is directed to specific processes including synthesis of oligonucleotides, gene synthesis, gene shuffling, amplification of RNA including amplification of full-length mRNA through cDNA synthesis, labeling of nucleic acids, sequencing, analysis of single-nucleotide polymorphism, gene amplification, mutation analysis and processing of detector molecules and others. In particular, the methods and processes include catalysis of a chemical reaction by thermostable RNA ligases at high temperatures such as above 40°C, for example, at temperatures of about 50°C to about 75°C, even more preferably at temperatures of about 55°C to about 70°C.

The publication pertains to processes using a thermostable RNA ligase such as RM378 RNA ligase and TS2126 RNA ligase to catalyze the ATP-dependent formation of a phosphodiester bond between a 3'-hydroxyl nucleic acid and a 5'-phosphate nucleic acid donor. These processes includes ligation of two oligonucleotides as well as the circularization of a single oligonucleotide.

An underlying concept is the use of thermostable RNA ligases. A common theme is manifested by a ligation step in which a thermostable RNA ligase of the invention is utilized.

As used herein, "Nucleobase" refers to a nitrogen-containing heterocyclic moiety, *e.g*., a purine, a 7-deazapurine, or a pyrimidine. Typical nucleobases are adenine, guanine, cytosine, uracil, thymine, 7-deazaadenine, 7-deazaguanine, and the like.

"Nucleoside" as used herein refers to a compound consisting of a nucleobase linked to the C-1' carbon of a ribose sugar.

"Nucleotide" as used herein refers to a phosphate ester of a nucleoside, as a monomer unit or within a nucleic acid. Nucleotides are sometimes denoted as "NTP," or "dNTP" and "ddNTP" to particularly point out the structural features of the ribose sugar.

"Nucleotide 5'-triphosphate" refers to a nucleotide with a triphosphate ester group at the 5' position. The triphosphate ester group can include sulfur substitutions for the various oxygens, *e.g.*, alpha-thio-nucleotide 5'-triphosphates.

As used herein, the term "nucleic acid" encompasses the terms "oligonucleotide" and "polynucleotide" and means single-stranded and double-stranded polymers of nucleotide monomers, including 2'-deoxyribonucleotides (DNA) and ribonucleotides (RNA). The nucleic acid can be composed entirely of deoxyribonucleotides, entirely of ribonucleotides, or chimeric mixtures thereof, linked by internucleotide phosphodiester bond linkages, and associated counter-ions, *e.g.*, H⁺, NH₄⁺, trialkylammonium, Mg²⁺, Na⁺ and the like. Nucleic acids typically range in size from a few monomeric units, *e.g.*, 5-40 when they are commonty referred to as oligonucleotides, to several thousands of monomeric units. Unless denoted otherwise, whenever an oligonucleotide sequence is represented, it will be understood that the nucleotides are in 5' to 3' order from left to right and that "A" denotes deoxyadenosine, "C" denotes deoxycytosine, "G" denotes deoxyguanosine, and "T" denotes deoxythymidine, unless otherwise noted.

"Nucleotide analog" as used herein can be a modified deoxyribonucleoside; a modified ribonucleoside; a base-modified, sugar-modified, a phosphate-modified phosphate group, a phosphorothioate group, a phosphonate group, a methyl-phosphonate group, a phosphoramidate group, a formylacetyl group, a phosphorodithioate group, a boranephosphate group, or a phosphotriester group.

The term "primer" normally refers herein to an oligonucleotide used, for example in amplification of nucleic acids such as PCR. The primer can be comprised of unmodified and/or modified nucleotides, for example modified by a biotin group attached to the nucleotide at the 5' end of the primer.

"Label" as used herein refers to any moiety covalently attached to a nucleotide that is detectable or imparts a desired functionality or property in the ligation extension product.

"Ligation" is the enzymatic joining by formation of a phosphodiester bond between nucleic acids.

"Peptide nucleic acid" (PNA) refers to synthetic oligomers containing any backbone of acyclic, achiral, and neutral polyamide linkages to which nucleobases are attached.

"Thermostable" or "thermophilic" is defined herein as having the ability to withstand temperatures above 50°C for a length of time in minutes without becoming irreversibly denatured and maintaining the ability to catalyze a chemical reaction, such as the formation of a phosphodiester bond, at preferred temperatures above 50°C, such as between 50°C and 100°C, at preferred temperatures of about 50°C to about 75°C and at even more preferred temperatures of about 55°C to about 70°C.

"Thermophilic bacteria", also referred to as "thermophiles", are defined as bacteria having optimum growth temperature above 50°C. "Thermophilic bacteriophages" or "thermostable bacteriophages" are defined as bacteriophages having thermophilic bacteria as hosts.

"Thermophilic microorganisms" are defined herein as thermophilic microorganisms selected from the group consisting of archea, bacteria and bacteriophage.

Methods of producing replicate copies of the same polynucleotide, such as PCR or gene cloning, are collectively referred to herein as "amplification" or "replication." For example, single or double stranded DNA can be replicated to form another DNA with the same sequence. RNA can be replicated, for example, by RNA directed RNA polymerase, or by reverse transcribing the RNA and then performing a PCR. In the latter case, the amplified copy of the RNA is a DNA with the correlating or homologous sequence.

The polymerase chain reaction ("PCR'') is a reaction in which replicate copies are made of a target polynucleotide using one or more primers, and a catalyst of polymerization, such as a DNA polymerase, and particularly a thermally stable polymerase enzyme. Generally, PCR involves repeatedly performing a "cycle" of three steps: "melting," in which the temperature is adjusted such that the DNA dissociates to single strands, "annealing," in which the temperature is adjusted such that oligonucleotide primers are permitted match their complementary base sequence using base pair recognition to form a duplex at one end of the span of polynucleotide to be amplified; and "extension" or "synthesis," which can occur at the same temperature as annealing, or in which the temperature is adjusted to a slightly higher and more optimum temperature, such that oligonucleotides that have formed a duplex are elongated with a DNA polymerase. The cycle is then repeated until the desired amount of amplified polynucleotide is obtained. Methods for PCR amplification can be found in U.S. Patent Nos. 4,683,195 and 4,683,202.

When referring to a particular protein such as a RNA ligase, the term "isolated" refers to the preparation of the protein which is substantially free of contaminants.

"Reverse transcription" or "reverse transcribing" refers to the process by which RNA is converted into cDNA through the action of a nucleic acid polymerase such as reverse transcriptase. Methods for reverse transcription are well known in the art and described for example in Ausubel, F.M., et al., Short Protocols in Molecular Biology, John Wiley and Sons (1995); and Innis, M.A. et al., PCR Protocols, Academic Press (1990).

The methods disclosed herein involving the molecular manipulation of nucleic acids are known to those skilled in the art. See generally Ausubel, F.M. et al., "Short Protocols in Molecular Biology," John Wiley and Sons (1995); and Sambrook, J., et al., "Molecular Cloning, A Laboratory Manual," 2nd ed., Cold Spring Harbor Laboratory Press (1989). The T4 RNA ligase has been utilized for a great variety of applications involving the manipulation of nucleic acids.

After the discovery and early characterization of T4 RNA ligase, it was realized that the enzyme could be used for synthesis of oligonucleotides including oligonucleotides with a defined sequence, even complete genes of DNA or their RNA equivalents (Gumport and Uhlenbeck, in "Gene Amplification and Analysis," Vol. II: Analysis of Nucleic Acid Structure by Enzymatic Methods, Chirikjian and Papas, eds. Elsevier North Holland, Inc.; McCoy and Gumport, Biochemistry, 19:635-642 (1980); Sugino, et al., J. Biol. Chem., 252:1732-1738 (1980)).

T4 RNA ligase has been used for the synthesis of circular hammer head ribozymes (Wang, L., and D.E. Ruffner, Nucleic Acids Res., 26:2502-2504 (1998)), synthesis of dinucleoside polyphosphates (Atencia, E.A., et al., Eur. J. Biochem., 261:802-811 (1999)), and for the production of composite primers (Kaluz, S., et al, BioTechniques, 19:182-186 (1995)).

The particular limitations of the T4 RNA ligase can hinder the practical use of applications being designed and prevent development of new applications. An enzyme with similar activity but substantially different properties such as a high working temperature and more equal activity on DNA and RNA substrates can make certain applications more practical and increase possibilities for the development of new methods and applications.

A thermostable RNA ligase can be utilized for improvements of various methods previously utilizing T4 RNA ligase such as methods described above. For example, amplification of mRNA and synthesis of cDNA often involve the use of a complex mixture of RNA containing RNA molecules with various stable secondary structures, which inhibits the action of T4 RNA ligase. The negative influence of secondary structure has been shown using well-defined substrates, both RNA and DNA. An additional heating step prior to ligation is often added to processes to reduce the undesirable secondary structures. The limited efficiency of T4 RNA ligase using natural RNA substrates has also been demonstrated (Gumport and Uhlenbeck, in "Gene Amplification and Analysis," Vol. II: Analysis of Nucleic Acid Structure by Enzymatic Methods, Chirikjian and Papas, eds. Elsevier North Holland, Inc. (1980); Bruce and Uhlenbeck, Nucleic Acids Res., 5:3665-3677 (1978); McCoy and Gumport, Biochemistry, 19:635-642 (1980)). Some protocols for mRNA amplification, recommended for example by manufactures of commercial kits for rapid amplification of cDNA ends (RACE), include a preheating step and subsequent cooling prior to ligation in an effort to decrease secondary structures of the substrate RNA. The possibility to carry out ligation reactions at higher temperatures using a thermostable RNA ligase will limit the formation of secondary structures and thus improve the amplification of the RNA by increasing the proportion of RNA molecules available for ligation by the enzyme. Similarly, other applications involving ligation of nucleic acids may benefit from decreased formation of secondary structures at higher temperatures. T4 RNA ligase originates from bacteriophage T4 that has a mesophilic host (*E. coli*) and is not thermostable. The present publication demonstrates the thermostability of RNA ligases analogous to the T4 RNA ligase. The discovery of thermostable homologues of T4 RNA ligase, originating from the thermophilic bacteriophage RM378 having thermophilic a bacterial host *Rhodothermus marinus,* represents the first known thermostable RNA ligase comparable to T4 RNA ligase. Further evidence for various other properties of this enzyme in comparison to T4 RNA ligase are described therein demonstrating important similarities and differences in properties of these homologous enzymes. A second thermostable RNA ligase enzyme has also been characterized from a another bacteriophage, TS2126 which infects the bacterial host *Thermus scotoductus.* It should be noted that *Thermus scotoductus* is thermophilic like *Rhodothermus marinus* but the two bacterial species are not closely related. The isolated enzyme, originating from bacteriophage TS2126, was predicted to be related to T4 RNA ligase based on a database sequence similarity search but the sequence similarity is low. TS2126 has nucleic acid ligase activity similar to T4 RNA ligase and RM378 RNA ligase. It is also shown that the TS2126 RNA ligase is active at high temperatures.

RM378 RNA ligase, TS2126 RNA ligase and T4 RNA ligase belong to a family of RNA ligases with very few known members, all of viral origin. The discovery of thermostable RNA ligases and the characterization of its activities is a significant contribution to the knowledge and further utilization of a small group of enzymes with versatile and useful activities for the manipulation of nucleic acids. The various uses of T4 RNA ligase already apparent by the many applications currently utilizing this enzyme indicates the utility of other enzymes having similar activities. The present publication provides characterization of RNA ligases with activities generally comparable with that of RNA ligase from bacteriophage T4 but with distinctly different properties making the use of the RM378 RNA ligase for various applications advantageous.

"RM378 RNA-ligase" refers to a polypeptide having the amino acid sequence of SEQ ID NO: 2. RM378 RNA ligase originates from bacteriophage RM378 having a thermophilic bacterial host *Rhodothermus marinus.*

"TS2126 RNA ligase" refers a polypeptide having the amino acid sequence of SEQ ID NO: 4. TS2126 RNA ligase originates from bacteriophage TS2126 having a thermophilic host *Thermus scotoductus.*

The term "RNA ligase" is used herein according to the conventional name of the homologous enzyme from bacteriophage T4 usually referred to as "RNA ligase" (Gumport and Uhlenbeck, in "Gene Amplification and Analysis," Vol. II: Analysis of Nucleic Acid Structure by Enzymatic Methods, Chirikjian and Papas, eds. Elsevier North Holland, Inc. (1980)). Alternatively, the enzyme can be described by terms such as "nucleic acid ligase" or "oligonucleotide ligase".

The terms "RM378 RNA ligase, TS2126 RNA ligase or any substantially similar enzyme" and "thermostable RNA ligases of the present publication or any substantially similar enzyme" refers to any polypeptide belonging to a group consisting of:
a) a RNA ligase obtained from a bacteriophage infecting a thermophilic bacteria; *i.e.*, bacteriophage having a thermophilic bacterial host;
b) a polypeptide comprising the amino acid sequence of SEQ ID NO: 2 or SEQ ID NO: 4;
c) a polypeptide encoded by a nucleic acid comprising the sequence of SEQ ID NO: 1 or SEQ ID NO: 3;
d) a polypeptide having at least 30% sequence identity with the amino acid sequence of SEQ ID NO: 2 or SEQ ID NO: 4; or
e) a fragment or derivative of (a), (b), (c), or (d).

The thermostable RNA ligases of the present publication or any substantially similar enzyme can be partially or substantially purified (*e.g.*, purified to homogeneity), and/or are substantially free of other polypeptides. Accordingly, the amino acid sequence of the polypeptide can be-that of the naturally-occurring polypeptide or can comprise-alterations therein. Polypeptides comprising alterations are referred to herein as "derivatives" of the native polypeptide. Such alterations include conservative or non-conservative amino acid substitutions, additions and deletions of one or more amino acids; however, such alterations should preserve at least one activity of the polypeptide, *i.e.*, the altered or mutant polypeptide should be an active derivative of the naturally-occurring polypeptide. For example, the mutation(s) can preferably preserve the three-dimensional configuration of the binding site of the native polypeptide, or can preferably preserve the activity of the polypeptide (*e.g.*, any mutations preferably preserve the ability of the enzyme to catalyze the ligation of nucleic acids). The presence or absence of activity or activities of the polypeptide can be determined by various standard functional assays including, but not limited to, assays for binding activity or enzymatic activity.

Additionally, the RNA ligases are directed to active fragments of the thermostable RNA ligases of the present invention or any substantially similar enzyme, as well as fragments of the active derivatives described above. An "active fragment," as referred to herein, is a portion of polypeptide (or a portion of an active derivative) that retains the polypeptide's activity, as described above.

Appropriate amino acid alterations can be made on the basis of several criteria, including hydrophobicity, basic or acidic character, charge, polarity, size, the presence or absence of a functional group (*e.g*., -SH or a glycosylation site), and aromatic character. Assignment of various amino acids to similar groups based on the properties above will be readily apparent to the skilled artisan; further appropriate amino acid changes can also be found in Bowie, et al., Science, 247:1306-1310 (1990). For example, conservative amino acid replacements can be those that take place within a family of amino acids that are related in their side chains. Genetically encoded amino acids are generally divided into four families: (1) acidic=aspartate, glutamate; (2) basic=lysine, arginine, histidine; (3) nonpolar=alanine, valine, leucine, isoleucine, proline, phenylalanine, methionine, tryptophan; and (4) uncharged polar=glycine, asparagine, glutamine, cystine, serine, threonine, tyrosine. Phenylalanine, tryptophan and tyrosine are sometimes classified jointly as aromatic amino acids. For example, it is reasonable to expect that an isolated replacement of a leucine with an isoleucine or valine, an aspartate with a glutamate, a threonine with a serine or a similar conservative replacement of an amino acid with a structurally related amino acid will not have a major effect on activity or functionality.

The thermostable RNA ligases of the present publication or any substantially similar enzyme can also be fusion polypeptides comprising all or a portion (*e*.*g*., an active fragment) of a native polypeptide fused to an additional component, with optional linker sequences. Additional components, such as radioisotopes and antigenic tags, can be selected to assist in the isolation or purification of the polypeptide or to extend the half life of the polypeptide; for example, a hexahistidine tag would permit ready purification by nickel chromatography. The fusion protein can contain, *e.g.*, a glutathione-S-transferase (GST), thioredoxin (TRX) or maltose binding protein (MBP) component to facilitate purification; kits for expression and purification of such fusion proteins are commercially available.

Also included in the thermostable RNA Ligases are polypeptides which are at least about 30% identical (*i.e.*, polypeptides which have substantial sequence identity) to the RM378 RNA ligase or the TS2126 RNA ligase. However, polypeptides exhibiting lower levels of identity are also useful, particular if they exhibit higher identity over one or more particular domains of the polypeptide. For example, polypeptides sharing high degrees of identity over domains necessary for particular activity, such as RNA ligase activity, are included herein. Thus, polypeptides which are at least about 10%, preferably at least about 20%, more preferably at least about 30%, more preferably at least about 40%, even more preferably at least about 50%, yet more preferably at least about 70%, still more preferably at least about 80%, and even more preferably at least about 90% identity, yet more preferably at least about 95% identity, are encompassed by the publication.

The thermostable RNA ligases of the present publication or any substantially similar enzyme can be isolated from naturally-occurring sources (*e.g.*, isolated from host cells infected with bacteriophage RM378, bacteriophage TS2126 or other bacteriophage infecting a thermophilic bacterium). Alternatively, the polypeptides can be chemically synthesized or recombinantly produced. For example, PCR primers can be designed to amplify the ORFs from the start codon to stop codon, using DNA of RM378 or TS2126 or related bacteriophages or respective recombinant clones as a template. The primers can contain suitable restriction sites for an efficient cloning into a suitable expression vector. The PCR product can be digested with the appropriate restriction enzyme and ligated between the corresponding restriction sites in the vector (the same restriction sites, or restriction sites producing the same cohesive ends or blunt end restriction sites).

The thermostable RNA ligases used in the present publication or any substantially similar enzyme can be isolated or purified (*e.g.*, to homogeneity) from cell culture (*e.g.*, from culture of host cells infected with bacteriophage RM378 or bacteriophage TS2126) by a variety of processes. These include, but are not limited to, anion or cation exchange chromatography, ethanol precipitation, affinity chromatography and high performance liquid chromatography (HPLC). The protein can be isolated by conventional means of protein biochemistry and purification to obtain a substantially pure product, *i.e.*, 80, 95 or 99% free of cell component contaminants, as described in Jacoby, Methods in Enzymology, Volume 104, Academic Press, New York (1984); Scopes, Protein Purification, Principles and Practice, 2nd Edition, Springer-Verlag, New York (1987); and Deutscher (ed.), Guide to Protein Purification, Methods in Enzymology, Vol. 182 (1990).

The publication pertains to processes using a thermostable RNA ligases such as RM378 RNA ligase, TS2126 ligase or a substantially similar enzyme, to catalyze the ATP-dependent formation of a phosphodiester bond between a 3'-hydroxyl nucleic acid acceptor and a 5'-phosphate nucleic acid donor. This includes ligation of two oligonucleotides as well as the circularization of a single oligonucleotide. The invention is thus directed to processes involving ligation of nucleic acids at relatively high temperatures. For example, the Examples demonstrate the ability of RM378 RNA ligase to catalyze the formation of bond between the 3'-hydroxyl end of a nucleic acid and a 5'-phosphate nucleic acid through circularization of a single nucleic acid substrate. Furthermore, it is shown in that the RM378 RNA ligase of the present publication is able to ligate two separate oligonucleotides thus demonstrating its ability to ligate two oligonucleotides as well as circularize a single oligonucleotide. It is also demonstrated that the RM378 RNA ligase of the present publication is able to catalyze reaction of this type using both RNA and DNA substrates.

The present publication demonstrates that, like the T4 RNA ligase, the RM378 RNA ligase of the publication does not require complementary template strands to align donor phosphates with acceptor hydroxyls. The enzyme is able to use single-stranded nucleic acids as substrates, an activity that is fundamental to various utilities of the enzyme. Importantly, the publication shows that the RM378 RNA ligase is able to catalyze these reactions at elevated temperatures, such as about 40°C to about 80°C. The publication thus shows for the first time the ability of any enzyme to optimally carry out catalysis of these types of reactions at high temperatures.

The publication also pertains to processes using thermostable RNA ligases such as RM378 RNA ligase or TS2126 RNA ligase to catalyze the ATP-independent formation of a phosphodiester bond between a 3'-hydroxyl nucleic acid acceptor and a 5'-phosphate nucleic acid donor. This can be accomplished by using an ADP-derivative (Ado-5'pp-X) as donor in enzymatically catalyzed joining of the extra moiety (p-X) to a nucleic acid substrate acceptor with the elimination of AMP.

Further, the invention is directed to processes using the thermostable RNA ligases of the present invention to catalyze the ligation of nucleic acids wherein the nucleic acid can be made of ribonucleotides (RNA), deoxyribonucleotides (DNA) or nucleotide analogs. The nucleic acids or nucleic acid analogs can be oligonucleotides or polynucleotides or single nucleotides such as a nucleoside 3',5'-biphosphate (pNp). The nucleic acids can be synthetic or natural. The natural nucleic acids can be obtained from biological samples and can be crude samples, or they can be substantially purified. The nucleic acids can further be made through previous amplification of natural nucleic acids. The nucleotide analogs can be analogs such as dideoxyribonucleotides or any base-modified, sugar-modified or phosphate group modified nucleotide. The nucleic acids can be made of natural nucleic acids or synthetic nucleic acids and can contain chimera of natural nucleic acids such as RNA-DNA chimeras. The substrates for the ligase can also contain chimeras of nucleic acids and non-nucleic acids such as chimeras of PNA and DNA, *i.e.* PNA-DNA (U.S. Patent No. 6,297,016).

Example 6 shows that the RM378 RNA ligase is able to use a nucleotide analog as a substrate. In the example, the RM378 RNA ligase uses 3'NH₂-3'dATP instead of ATP for adenylation of a nucleic acid.

The publication includes processes involving ligation of nucleic acids at temperatures of at least about 40°C, such as at least 50°C, such as at least 55°C, such as at least 55°C, such as at least 60°C, such as at least 65°C, such as at least 70°C. Especially preferred aspects include processes involving ligations at temperatures in the range of about 50°C to about 75°C, such as about 55°C to about 70°C. The elevated temperatures can limit formation of secondary structures which limit the use of the conventional T4 RNA ligase which has been shown to less effective on substrates containing secondary structure such as the yeast tRNA^{Phe} (Gumport and Uhlenbeck, in "Gene Amplification and Analysis,"Vol. II: Analysis of Nucleic Acid Structure by Enzymatic Methods (1980); Chirikjian and Papas, eds. Elsevier North Holland, Inc; Bruce & Uhlenbeck, Nucleic Acids Res., 5:3665-3677 (1978); McCoy and Gumport, Biochemistry, 19:635-642 (1980)). Protocols for amplification of mRNA include a heating step prior to ligation with T4 RNA ligase to limit secondary structures of substrate nucleic acid. Furthermore, the utility of a thermostable RNA ligase has been suggested for the synthesis of oligonucleotides (Hyman, U.S. Patent No. 5,514,569). As shown in Example 3, the RM378 RNA ligase has a temperature profile clearly distinct from T4 RNA ligase. The RM378 RNA ligase is active at higher temperatures than the T4 RNA ligase, such as about 40°C to about 80°C. The RM378 enzyme has an optimum temperature between 60°C and 65°C, at a temperature where the T4 enzyme is practically inactive. Example 13 shows that the enzyme of the present invention is active at higher temperatures than the T4 RNA ligase, such as at about 40°C to about 80°C, such as temperatures of about 50°C to about 75°C, and even more preferable at temperatures of about 55°C to about 70°C. Thus, the thermostable RNA ligases, such as RM378 RNA ligase and TS2126 RNA ligase, can be used for ligation of nucleic acids at temperatures outside the working temperatures of the T4 RNA ligase. Elevated temperatures will limit the formation of secondary structures among substrate nucleic acids and any process involving ligation of nucleic acids containing substantial tertiary or secondary structures can preferably be carried out at higher temperatures, such as above 40°C. For example, natural RNA substrates such as ribosomal RNA and mRNA can contain substantial secondary structure which can limit ligation such as by limiting access to 5'-phosphate groups in the ligation reaction.

The present invention is directed to processes involving ligation of DNA molecules. The T4 RNA ligase has been shown to be much less active on DNA substrates than RNA substrates and reactions involving ligation of DNA catalyzed by T4 RNA ligase have required large amount of enzyme and long reaction times (Gumport and Uhlenbeck, "Gene Amplification and Analysis," Vol. II: Analysis of Nucleic Acid Structure by Enzymatic Methods, Chirikjian and Papas, eds. Elsevier North Holland, Inc (1980); Gumport, et al., Nucleic Acids Symp. Ser., 7:167-171 (1980)). As shown in Example 5, the publication clearly demonstrates that the RM378 RNA ligase is far more active on DNA substrates than the T4 RNA ligase but under different conditions, optimized for the T4 enzyme, the activity of the two enzymes is more comparable. However, it is shown that the RM378 RNA ligase is able to catalyze these types of reactions at elevated temperatures and determination of optimal conditions for the RM378 enzyme, such as was done for the T4 RNA ligase, can lead to a substantial increase activity. In comparison to T4 RNA ligase, the RM378 RNA Ligase has much more similar activity on DNA and RNA under the conditions detailed in the Examples. From these results, the RM378 prefers DNA over RNA as a substrate which is in contrast to the T4 RNA ligase.

Examples 12-18 show that the TS2126 ligase is stable and active under a variety of conditions of temperature, cation concentration, ATP concentration and protein concentration. Further, Example 17 shows that the TS2126 ligase is far more active on an RNA substrate than the T4 enzyme

The publication is directed to processes involving ligation of nucleic acids including single stranded DNA and double stranded DNA such as restriction fragments including DNA restriction fragments with 5'-staggered ends or blunt ends.

The publication is also directed to processes utilizing thermostable RNA ligases as described herein or any substantially similar enzyme to catalyze a reaction between a 3'-phosphate donor and 5'-hydroxyl acceptor. The publication is further directed to processes involving template-directed ligation of nucleic acids such as template-directed ligation of DNA.

The publication is directed to processes using thermostable or substantially similar enzyme to synthesize oligonucleotides. The synthetic oligonucleotides have widespread use in various fields and can be used for example in applications in molecular biology, including genetic engineering and PCR, in therapeutics, for example for antisense oligonucleotides, for diagnostics and to make catalysts such as ribozymes. The synthetic oligonucleotides can be used as primers for amplification of genetic material. PCR technology, for example, routinely employs oligonucleotides as primers for amplification of genetic material and synthetic genes are made for various purposes including optimization of codon usage for efficient expression. Useful synthetic oligonucleotides include polymers containing natural ribonucleotides and deoxynucleotides as well as polymers containing modified nucleotides such as base-modified, sugar-modified and phosphate-group modified nucleotides.

The invention further includes processes for making oligonucleotides or polynucleotides constituting genes or parts thereof, made of DNA oligonucleotides or their RNA analogs. The synthetic genes can be for inclusion in vectors for expression of a particular gene product. The synthetic genes can comprise codons not used in naturally occurring genes for example for the purpose of optimization of codon usage for efficient expression in a particular host. In the synthesis of oligonucleotides catalyzed by a thermostable RNA ligase, the efficiency of the reactions can be enhanced by blocking the 3'-terminus of donor molecules or de-phosphorylating the 5'-terminus of acceptor molecules; thus driving the reaction to yield products containing a defined order of the oligonucleotide sequences. The use of a thermostable ligase can be preferable to the use of other ligases such as the T4 RNA ligase due to its distinct properties such as its ability to work at high temperatures. This can increase the efficiency of the reaction as well as limit the amount of enzyme and the time needed for synthesis.

The synthesis of oligonucleotides can be performed by repeated cycles of combining a primer oligonucleotide and a blocked oligonucleotide using a thermostable RNA ligase, such as such as RM378 RNA ligase or TS2126 RNA ligase.

In a series of patents (U.S. Patents Nos. 5,516,664; 5,629,177; 5,514,569 and 5,602,000), Hyman describes the synthesis of oligonucleotides by repeated cycles of combining a primer oligonucleotide and a blocked oligonucleotide using RNA ligase. The method involves few steps: i) combining the primer and a nucleotide having a 3'-end blocked by a phosphate group in the presence of RNA ligase thereby forming an extended primer with a blocked 3'-end; ii) enzymatically removing the blocking phosphate group at the 3'-end of the extended primer using a phosphatase; and iii) repeating the previous steps using the primer-nucleotide from previous cycle (ii) as the primer in the first step (i) in the next cycle. Using Hyman's method, the primer in each step functions as the acceptor with a free 3'-OH group and the activated adenylated nucleotide to be added as the donor with a 5'-phosphate group. This way, the enzymatic procedure proceeds in the 5' to 3' direction. However, Havlina describes the surprising discovery of the capability of RNA ligase to link a 3'-phosphate donor and a 5'-hydroxyl acceptor (U.S. Patent No. 6,329,177). This allows for the synthesis of oligonucleotides in the 3' to 5' direction using RNA ligase, in opposite direction compared to the above procedure described by Hyman. In Havlinaś method, RNA ligase can be used to ligate an oligonucleotide primer to a carrier molecule with a protecting group at the 5'-position or lacking a protecting group at the 3'-position.

The above procedure has been carried out previously using T4 RNA ligase, which is not thermostable, and a phosphatase that was also not thermostable. As pointed out in U.S. Patent No. 5,516,664, the procedure could benefit from the use of a thermostable RNA ligase and thermal cycling, *i.e.,* performing the ligase reaction at high temperature and then turn off the activity of the ligase by lowering the temperature for subsequent incubation with the phosphatase. Elevating the temperature again in the next cycle would then activate the RNA ligase again.

Using the method outlined above, the synthesis of oligonucleotides proceeds in the 5' to 3' direction with the primer in each step functions as the acceptor with a free 3'-OH group and the activated adenylated nucleotide (adenylated 3',5'-bisphosphate) to be added as the donor with a 5'-phosphate group. The invention also pertains to processes involving synthesis of oligonucleotides in the 3' to 5' direction catalyzed by the RNA ligase by linking a 3'-phosphate donor and a 5'-hydroxyl acceptor. The RNA ligase can be used to ligate an oligonucleotide primer to a carrier molecule with a protecting group at the 5'-position or lacking a protecting group at the 3'-position. This method for preparing a nucleotide polymer in the 3' to 5' direction can essentially involve the following steps: i) providing a nucleic acid primer and a nucleic acid carrier molecule; ii) ligating the 3' end of the carrier molecule to the 5' position of the primer with RNA ligase; thereby obtaining a ligation product; iii) de-protecting the ligation product by removing a protecting group of the carrier molecule; iv) transferring a natural or modified phosphate group to the 5' position of the ligation product; and v) optionally repeating steps (i) to (iv).

The methods of the invention are also directed the synthesis of oligonucleotides in the 3'-to 5'- direction catalyzed by the RNA ligase by linking a 5'-phosphate donor of a first oligonucleotide and a 3'-hydroxyl acceptor of a second oligonucleotide. The circularization of the individual oligonucleotides can be blocked such as by attaching a chemical group such as biotin to the 3'-end of the oligonucleotide or by having a hydroxyl group at both the 5' end and the 3' end. The method involves: i) combining the first oligonucleotide with a 5'-phosphate group and the second oligonucleotide having a 3'-hydroxyl and 5'-hydroxyl group in the presence of the RNA ligase thereby forming an extended oligonucleotide formed through ligation of the 5'-phosphate of the first oligonucleotide and the 3'-hydroxyl group of the second oligonucleotide; ii) adding a phosphate group to the 5'-end of the extended oligonucleotide such as by using polynucleotide kinase; iii) optionally blocking the 5' end of the unreacted first oligonucleotide; and iv) repeating steps i) to iii) using the extended oligonucleotide from the previous cycle ii) as the first oligonucleotide i) in the next cycle.

The methods of the invention are also directed the synthesis of oligonucleotides in the 5'-to 3'- direction catalyzed by the RNA ligase by linking a 3'-hydroxyl donor of a first oligonucleotide and a 5'-phosphate acceptor of a second oligonucleotide. The circularization of the individual oligonucleotides can be blocked such as by attaching a chemical group such as biotin to the 5'-end of the oligonucleotide or by having a hydroxyl group at both the 5' end and the 3' end or by having a phosphate group at both the 5'end and the 3' end. The method involves: i) combining the first oligonucleotide with a 3'-hydroxyl group and the second oligonucleotide having a 3'-phosphate and 5'-phosphate group in the presence of the RNA ligase thereby forming an extended oligonucleotide formed through ligation of the 3'-hydroxyl of the first oligonucleotide and the 5'-phosphate group of the second oligonucleotide; ii) removing the phosphate group at the 3'-end of the extended oligonucleotide such as by using a phosphatase; iii) optionally blocking the 3' end of the unreacted first oligonucleotide; and iv) repeating steps i) to iii) using the extended oligonucleotide from the previous cycle ii) as the first oligonucleotide i) in the next cycle.

The oligonucleotides used for synthesis catalyzed by the ligase can be performed using methods such as chemical synthesis. The RNA ligases described herein can be used to ligate single-stranded oligonucleotides of variable length, such as about 50 to about 100 bases. The blocking of the 5' end of oligonucleotides can be done for example through chemical modification of the 5'-phosphate group. The extended oligonucleotide can comprise a synthetic gene coding for a gene product comprising the amino acid sequence of a naturally occurring protein or a modification thereof. The oligonucleotide synthesis can further be carried out with a plurality of first oligonucleotides of variable sequence and/or a plurality of second oligonucleotides of variable sequence to produce a plurality of extended oligonucleotides of variable sequence produced by different combinations of various first oligonucleotides with various second oligonucleotides. The procedure can be repeated for additional cycles. The method can thus be used as a method for gene shuffling such as by having the plurality of oligonucleotides comprising the sequence of corresponding fragments of genes of naturally occurring sequences from different sources. Example 9 below describes the synthesis of a gene coding for a protein with the amino acid sequence of a naturally occurring protein. The example demonstrates the synthesis of gene using the RM378 RNA ligase. In the example, the experiment was designed to produce a synthetic gene suitable for cloning and expression of a protein equivalent to the active human insulin-like growth factor. The example demonstrates the synthesis of genes optimized for the production of specific protein having several potential advantages over other methods for producing the same protein. The protein is of human origin and can be difficult to obtain directly. The active protein is only a part of a precursor protein which *in vivo* requires processing to produce the mature protein chain. Production of the active protein through expression cloning would require genetic engineering, such of a subcloning of a corresponding gene fragment from cDNA and the insertion of a initiator methionine codon to produce an expression vector expressing only the active protein. The composition of the sequence of the human gene, such as codon usage and GC content can not be optimal for expression in a heterological host such as *E. coli.*

The publication also pertains to methods using the RNA ligase for amplification of RNA, such as methods for amplification of mRNA including synthesis of the corresponding cDNA. The methods can benefit from the use of the thermostable RNA ligase such as a thermostable RNA ligase in favor of the conventional RNA ligase fromT4. For example, amplification of mRNA can preferably be carried out at high temperatures such as about 60°C to limit formation of secondary structures in the nucleic acid substrates that can inhibit the ligase reaction.

Amplification methods can be used for identifying the 5'and 3' untranslated regions of genes, studying heterogeneous transcriptional start sites, characterizing promoter regions, obtaining the complete cDNA sequence of a gene and amplifying the full-length cDNA for downstream cloning and expression.

Several methods have been disclosed involving amplification of RNA, especially mRNA through synthesis of the corresponding cDNA. Kempe, *et al.,* U.S. Patent No. 4,661,450, describes a method for molecular cloning of RNA. In this method the use of T4 RNA ligase is fundamental for the process wherein the RNA ligase is used to attach oligonucleotide linkers to the single-stranded molecule to be cloned. The attached oligonucleotides can be composed of RNA, DNA or mixture of each and facilitate the insertion of the RNA species into a cloning vector. Multiple DNA copies can then be obtained after transformation of the cloning vector into a suitable host. One disadvantage of this particular method is the requirement of having a ribonucleotide at the 3'-terminus of the linker which is attached to the 5'-terminus of the single-stranded RNA molecule to be cloned. This requirement is based on the properties of conventional RNA ligase from bacteriophage T4 which does not effectively use deoxynucleotide with the 3'-hydroxyl group of the acceptor. The use of T4 RNA ligase is thus limited by its substrate specificity.

More recently, methods for amplification of mRNA have mostly been based on synthesis of cDNA with the use of reverse transcriptase and amplification using PCR. Various variations and improvements on the general method of synthesizing cDNA have appeared including methods to obtain cDNA of full-length RNA such as RACE (Rapid amplification of cDNA ends, Maruyama, et al., Nucleic Acids Res., 23:3796-7 (1995)). The methods described often involve the use of RNA ligase for ligation of nucleic acids such as for ligation of oligonucleotide to the 5'-ends of the mRNA or circularization of single-stranded cDNA. One problem associated with traditional RACE methods is the amplification of truncated cDNA (Schaefer, B.C., Anal. Biochem., 227:255-273 (1995)). Ligation-mediated amplification of RNA uses RNA ligase to increase reliability of the process by preserving the termini of the RNA molecules (Volloch, et al., Nucleic Acids Res., 22:2507-2511 (1994)). The presence of the cap structure on the 5'-end of full-length mRNA can be used to selectively produce cDNAs with complete length. First, a phosphatase is used to dephoshorylase mRNA molecules with a free phosphate group at the 5'-end, *i.e.*, degraded and incomplete RNA molecules. After enzymatic removal of the cap on full-length mRNAs, linkers can be added to decapped mRNA molecules which now have a free 5'-phosphate group and can function as substrates for RNA ligase in contrast to the molecules lacking a 5'-phosphate group. A specific oligonucleotide can thus be ligated to the 5'-end of the full-length RNA molecules and cDNA can be produced using reverse transcriptase with for example, a primer containing a poly(T) region complementary to the poly(A) region of eukaryotic mRNA. The cDNA can then be amplified using PCR with primers complementary to the previously ligated oligonucleotide and a gene specific primer or a primer complementary to the poly(A) region (Maruyama & Sugano, Gene, 138:171-174 (1994)).

U.S. Patent No. 5,597,713 describes a method of producing cDNAs with complete length by ligation of DNA or DNA-RNA chimeric oligonucleotide to the 5'-end of intact mRNAs after decapping. PCT Patent No. WO 01/04286 describes optimization of a method for constructing full-length cDNA libraries, by minimizing mRNA degradation and increase fullness ratio, through optimization of reaction conditions including the RNA ligase reaction. U.S. Patent No. 6,242,189 discloses a method for selective isolation of bacterial mRNA after enzymatic modification of the mRNA such as by using RNA ligase. Merenkova, et al. (U.S. Patent No. 6,022,715) describe a method for specific coupling of the 5'-cap of the mRNA, using chemical modifications, with subsequent isolation of mRNA and preparation of complete cDNA and Zohlnhöfer and Klein (PCT Patent No. WO 01/71027) describe a method for amplification of mRNA involving ligation of poly(C) and poly(G) flanks to cDNA.

Recently identified applications of T4 RNA ligase are based on a target-mediated ligation of DNA by RNA ligase (U.S. Patent No. 6,368,801). Accordingly, T4 RNA ligase can, more efficiently than T4 DNA ligase, ligate DNA ends hybridized to RNA. This property of T4 RNA ligase can be used for the detection and/or amplification of nucleic acids. Thus, known techniques based on ligation of DNA can be improved using T4 RNA ligase. These methods include ligase chain reaction (LCR), ligation-mediated PCR (LD-PCR), reverse transcription PCR combined with ligation, PCR/ligation detection reaction (PCR/LDR), oligonucleotide ligation assay (OLA), ligation-during-amplification (LDA), iterative gap ligation (IGL) and ligation of padlock probes, open circle probes and other circularizable probes.

A method for amplification of mRNA but not encompassing cDNA synthesis has been described (U.S. Patent No. 6,338,954). This method uses RNA polymerase for amplification from an attached promoter sequence. RNA ligase is used to attach double-stranded DNA with a promoter sequence to RNA molecules.

Thermostable RNA ligase is used for molecular cloning of RNA wherein the RNA ligase is used to attach oligonucleotide linkers to single-stranded RNA molecule to be cloned. The attached oligonucleotides can be composed of RNA, DNA or mixture of each and facilitate the insertion of the RNA species into a cloning vector. Multiple DNA copies can then be obtained after transformation of the cloning vector into a suitable host.

Preferentially amplification of mRNA can be based on synthesis of cDNA with the use of reverse transcriptase and amplification using PCR. This includes methods to obtain cDNA of full-length RNA such as methods for rapid amplification of cDNA ends (RACE, Maruyama, et al., Nucleic Acids Res., 23:3796-7 (1995)). These methods involve the use of RNA ligase for ligation of nucleic acids such as for ligation of oligonucleotide to the 5'-ends of the mRNA or circularization of single-stranded cDNA. The thermostable RNA ligase can be used for ligation-mediated amplification of RNA by preserving the termini of the RNA molecules. The presence of the cap structure on the 5'-end of full-length mRNA can be used to selectively produce cDNAs with complete length. As an example, the process essentially comprises the following: i) a phosphatase, such as alkaline phosphatase, is used to dephoshorylase mRNA molecules with a free phosphate group at the 5'-end, *i.e.*, degraded and incomplete RNA molecules which lack a 5'-cap; ii) the 5'-cap on full-length mRNAs is removed such as by enzymatic removal such as by using the enzyme tobacco acid pyrophosphatase (TAP); iii) the thermostable RNA ligase, such as RM378 RNA ligase or TS2126 RNA ligase or any substantially similar enzyme is used to add linkers to the 5'-end of decapped mRNA molecules; iv) cDNA is synthesized using reverse transcriptase such as by using a primer containing a poly(T) region complementary to a poly(A) region of the mRNA; and v) the cDNA is amplified such as by using PCR such as with primers complementary to the previously ligated oligonucleotide and a gene specific primer or a primer complementary to a poly (A) region.

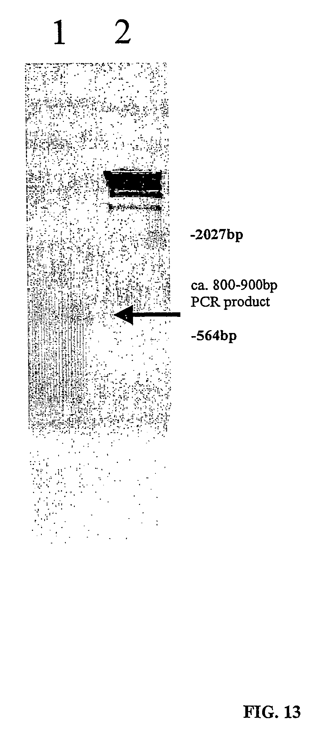

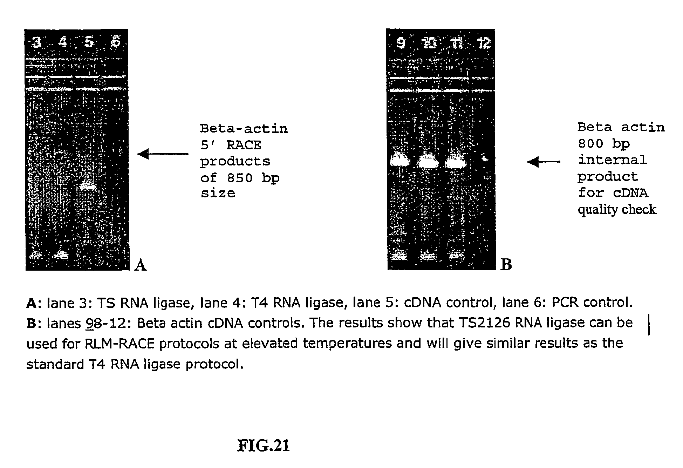

Example 10 provides an example of how the RM378 ligase can be used in 5'-RACE (RLM-RACE) applications. Thus, FIG. 13 shows a PCR product band in the expected size range. Further, Example 19 shows results using TS2126 and T4 ligases in an RLM-RACE application. The results (FIG. 21) clearly show that the TS2126 ligase is suitable for applications of this nature. These examples therefore illustrate the feasibility of using thermostable ligases of the present publication in RACE reactions, further illustrating the wide variety of novel applications for these thermostable enzymes.

The ligation step is preferably carried out at high temperatures, such as about 40°C to about 80°C, more preferably at temperatures of about 50°C to about 75°C, and even more preferably at about 55°C to about 70°C. The linkers added to the 5'-end of RNA molecules can comprise oligonucleotides composed of RNA, DNA, DNA-RNA chimeric oligonucleotides or nucleotide analogs.

Variations on this technique for the rapid amplification of cDNA end (RACE) are also contemplated. For example, a 5'end region of target mRNA can be amplified by: 1) synthesizing the cDNA, by synthesizing the first strand of cDNA by reverse transcription of target mRNA using a 5' end-phosphorylated RT-primer that is specific for the target RNA; 2) degrade the hybrid DNA-RNA by treatment with RNase H to remove RNA; 3) circularize the single-stranded cDNA to form concatemers with a thermostable RNA ligase, such as RM378 RNA ligase or TS2126 RNA ligase or any substantially similar enzyme; and 4) amplify DNA by PCR using specific primers that are complementary to known sequences.

In another method, mRNA is treated with a phosphatase to eliminate the 5' phosphates from truncted mRNA and non-mRNA, the dephosphorylated RNA is treated the pyrophosphatase to remove the 5'cap structure from intact, full-length mRNA, which leaves a 5' phosphate required for ligation to a RNA oligonucleotide. An oligonucleotide is ligated using a thermostable RNA ligase, such as RM378 RNA ligase or TS2126 RNA ligase or any substantially similar enzyme, to the 5'end of the full-length decapped mRNA. The RNA oligonucleotide provides a priming site for 3' primers. The ligated mRNA is reverse-transcribed. mRNA is degraded by RNAse H. cDNA generated is then amplified by PCR.

Generally, PCR amplification procedure is based on the application of two specific primers. Therefore, in PCR screening, two conserved target sites with favorable length of interval sequence are required. Although, the method can be adapted in a high throughput manner, most of these single gene PCR methods have only been used on DNA samples from single species harboring limited number of genes.

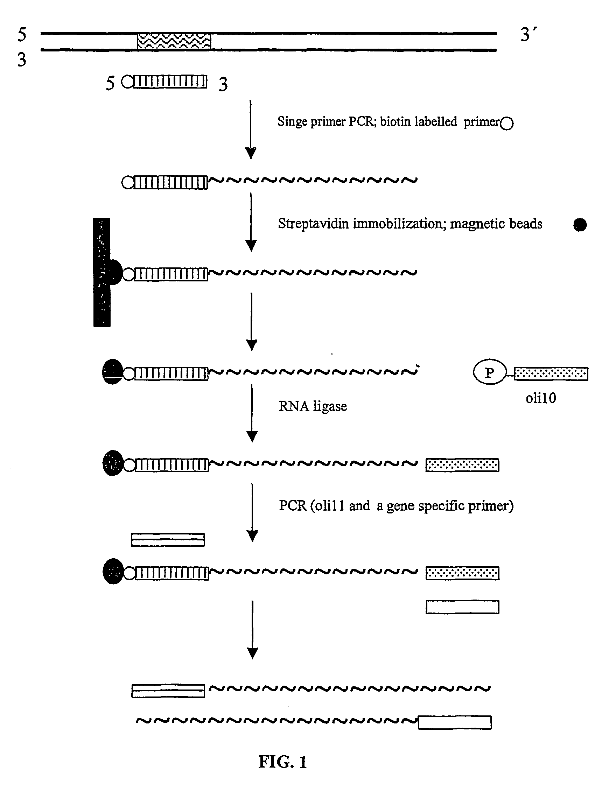

One approach for single primer PCR (linear PCR) can be performed for example, by using one gene specific primer in each PCR and then ligating an adaptor sequence to the 3' end of the single stranded copy-DNA to provide a second primer site for the second amplification step. The designed gene specific primers are affinity labeled at the 5' end (such as preferably labeled with biotin), which allows the separation of the first single stranded DNA product from the complex DNA. After a number copies of the single stranded DNA have been produced by linear amplification, a second reverse priming site can be made available by ligating a single stranded oligonucleotide of known sequence to the 3' end of the single stranded DNA by means of a ligase such as T4 RNA ligase. The modified templates are then re-amplified by using the gene specific primer (unlabelled) and a reverse primer complementing the adapter sequence primer to make double-stranded DNA that can then be amplified.by PCR for further cloning and/or sequencing (FIG. 1).

The publication also pertains to processes for using a thermostable RNA ligase, such as RM378 RNA ligase or TS2126 RNA ligase in single primer PCR (linear PCR and Inverse PCR). The RNA ligase may be applied in a PCR method where only one specific primer is used. The second downstream reverse primer (unspecific) binds to an anchor sequence, which is added to the polymerase extension product from the specific primer with RNA ligase. The double stranded DNA generated in this way is then cloned and sequenced. This single specific primer PCR method can be used for screening of family wide gene fragments and further for retrieval of complete genes from various DNA sources such as from pure or mixed cultures and environmental samples or DNA libraries.

The screening method consists of DNA or the mixture of the nucleic acids extracted from the said sample and then the DNA is hybridized with a degenerate primer targeted to a single region in said target sequence. The gene specific primer is degenerate for a highly conserved amino acid sequence region, which is identified by analyzing multiple alignments of proteins from the protein family that is targeted. The degenerate gene specific primer can be designed by a number of methods, including the CODEHOP method [Consensus-Degenerate Hybrid Oligonucleotide Primer] (Rose *et al.,* 1998). The target region of the protein family being targeted should preferably contain at least 3-4 conserved amino acid. Following hybridization, the single stranded copy-DNA-molecules (extension products) are produced with a polymerase or reverse transcriptase by repeated thermal cycling (a linear amplification). The single stranded DNA is then separated from unused primers and template DNA with column purification. A further purification step of the single stranded DNA can be included to reduce background caused by unspecific hybridization of primers and degraded DNA. This is done with specific primers, affinity labeled at the 5' end (preferably labeled with biotin). Thus, the single stranded DNA product can be immobilized to a solid support with the binding to the corresponding ligand molecule (preferably streptavidin, *e.g.* streptavidin coded beads or wells) and non-immobilized DNA can be washed away. Then a second primer site is provided to the 3' end of the single stranded copy-DNA with the ligation of a defined 5'-phosphorylated oligonucleotide linker (anchor-molecule) to the 3' end of the previously produced single stranded DNA molecules with RNA ligase. Following the ligation the single stranded copy-DNA's can be amplified with a primer pair, which comprises part of the 5' degenerate primer sequence and a primer complementary to the 3' anchor molecule. The PCR product can then be cloned and sequenced or directly sequenced.

Additionally, the method for accessing natural diversity and recovering unknown genes and gene fragments from complex DNA isolated from mixed populations of natural microorganisms is an alternative to other conventional methods based on the construction and screening of DNA libraries (Woo, et al., Nucleic Acid Res., 22:4922-4931 (1994); Dalboge H., FEMS Microbiol Rev., 21:29-42 (1997); Rondon et al., PNAS USA, 96:6452-6455 (1999); U.S. Patent No. 5,958,672; Henne, et al., Appl. Environ. Microbiol., 66:3133-316 (2000)) or PCR amplification of environmental samples with two specific degenerate primers (forward and reverse) (U.S. Patent No. 5,849,491). In fact, the method described above has several advantages over these methods. First of all, it is simple, less time consuming and more targeted to discover new diversity of homologous gene than the library construction methods. Secondly, this method can be used for all gene families, as long as at least one common conserved region is found, containing at least 3 well conserved amino acids and that one specific gene family specific degenerate primer can be designed. This is in contrast to library expression screening where different screening assay, which can be very complicated in procedure, must be employed for different enzyme activities. The requirement of only one conserved region in a protein family instead of two such sites, as are required for the application of the conventional two primer methods, obviously allows for the retrieval of much greater diversity of more distantly related genes with the present publication.

The single primer method has additional advantages over PCR screening methods where two specific primers (forward and reverse) are used. The first step requires the hybridization of only one specific primer before the polymerization starts, and is therefore kinetically more favorable than when hybridization of two primers is required.
Consequently greater diversity is obtainable with the single primer method. With the use of only one gene family specific primer in each reaction as in the invented method, fewer reaction are needed (no matrix of forward and reverse primers). This not only saves time and expensive reagents but also the source DNA. Another significant advantage of the single specific primer method compared to the two primer method is that longer sequences can be obtained in the first reaction since the fragment length is not dependent on the interval between two conserved sites. Few methods based on the application of only one gene specific primer have been described (Jones and Winistorfer, Nucleic Acids Res. 20:595-600 (1992); Jones and Winistorfer, Biotechniques, 15:894-904 (1993); Megonigal et al., PNAS USA, 97: 9597-9602 (2000); Riley, et al., Nucleic Acids Res., 18:2887-2890 (1990); Rubie, et al., Biotechniques, 27:414-418 (1999); Morris et al. Appl. Environ. Microbiol., 1998; Rosenthal and Jones, Nucleic Acids Res., 18:3095-3096 (1990); Kilstrup and Kristiansen, Nucleic Acid Res., 28 E55 (2000); Stokes, et al., Appl. Environ Microbiol., 67:5240-5246 (2001), and Laging, et al., Nucleic Acid Res., 29:E8 (2001)). However these methods have only been described for the purpose of isolation of unknown sequences in a single genome DNA or genome library DNA. Furthermore, in the method described above, one polymerase reaction takes place as the first step, wherein single stranded polynucleotides are produced. Since no restriction or ligation of the source DNA takes place the demands for high quality DNA are not as stringent as for the library-based methods.

The single specific primer - PCR method can be applied for the isolation of sequences flanking a known sequence. Thus the method can be used for the retrieval of complete genes by gene walking from the fragment isolated with the single specific primer PCR screening. In this case two forward specific non-degenerate primers are used in two PCR reactions. In the first reaction, an outer primer labeled with immobilization-ligand (*e.g.*, biotin labeled) is used to generate single stranded DNA molecules, which are purified as described above. In the second reaction the single stranded DNA produced in the first round is used as a template in the second round. A second nested forward primer is then used against the reverse anchor primer in a PCR reaction to improve the specificity of the amplification products. The products can then be cloned and sequenced. Example 8 describes in detail an experiment of PCR with single specific primer using T4 RNA ligase as well as RM378 RNA ligase. The publication is directed to methods involving single primer PCR wherein the ligation step using RNA ligase is preferably carried out at high temperatures, such as at temperatures about 40°C to about 80°C, more preferably at temperatures of about 50°C to about 75°C, even more preferable at temperatures of about 55°C to about 70°C.

The publication further pertains to methods for labeling of nucleic acids using a thermostable RNA ligase, such as RM378 RNA ligase or TS2126 RNA ligase or any substantially similar enzyme. RNA ligase can be used for the labeling of oligonucleotide probes, primers or template molecules or polynucleotide probes or template molecules with nucleotide or oligonucleotide labeled with a chemical group. T4 RNA ligase has been used in fluorescence-, isotope or biotin-labeling of the 5'-end of DNA/RNA molecules (Kinoshita, et al., Nucl. Acid Res., 26:2502-2504 (1997)).

Labeling of the nucleic acid (probe or primers) with the RNA ligase can be carried out prior to or following hybridization (cf. PCT WO97/27317). The chemical group can immobilize the hybrid probe/template molecule on a solid surface (see for example U.S. Patent No. 5,595,908) or it can serve as a ligand which binds to a molecule (antibody) coupled with an enzymatically active group, thus allowing measuring of enzymatic activity and thereby achieving quantitative measure of the specific nucleotide acid in said sample.

Labeled DNA or RNA molecules can be used in various methods of quantitatively detecting nucleic acids and for detection of polynucleotide hybridization. The hybridization of DNA or RNA template molecules with the labeled nucleic acid probes can be carried out in a solution (see for example U.S. Patent No. 6,136,531) or on a solid surface. If the hybridization takes place on a solid surface, either the nucleic acid probes or the template DNA can be immobilized prior the hybridization. Further, the different probes can be immobilized and organized in an array. The hybrid template/probe molecules can be detected in solution or immobilized on a solid surface.

The methods described herein pertain to the use of thermostable RNA ligases in detection assays for nucleic acids such as in diagnostics assays. This includes detection in various samples such as the detection of DNA contamination in biopharmaceuticals or detection of rare nucleic acids in clinical samples. Template nucleic acid molecules to be detected can be hybridized with binary nucleotide probes complementary to adjacent portions of the target sequence. Following hybridization the probes can be ligated with the RNA ligase in a template dependent manner. The template nucleic acid can be DNA or RNA and the primer molecules can be DNA or RNA. The ligation product can be detected with PCR amplification using appropriate primers, nucleotides and polymerases. The ligation chain reaction (LCR) can also be used for the detection of the ligation product. One of the primers or both can be labeled with radioactive, fluorescent, or electrochemiluminescent molecule, or ligand, which can bind to a molecule (antibody) coupled with an enzymatically active group, thus allowing quantitative measure of the specific nucleotide acid in said sample. Another method is to use a probe, which is complementary on 5' and 3' ends to the target nucleic acid. The ends hybridize to adjacent portions of the target DNA and can be ligated with RNA ligase in a template dependent manner thus circularizing the probe. Following ligation, one complementary primer can be added to the circular template and subsequently primer extension can be performed. Also, two primers can be added to the circular template, one reverse complementary and another forward primer, to amplify the circular template. Either the primer or the dNTPs in the PCR reaction can be labeled with radioactive, fluorescent, or electrochemiluminescent molecule, or ligand, which can bind to a molecule (antibody) coupled with an enzymatically active group, thus allowing detection and quantitative measure of the amplification product.

The invention is further directed to methods wherein a thermostable RNA ligase is used in a process of sequencing short oligonucleotides. The method can essentially comprise the following steps: an auxiliary oligonucleotide is ligated to the 3'-end of a target oligonucleotide with the RM378 RNA ligase. A labeled primer complementary to the auxiliary oligonucleotide is hybridized to the ligation product. The auxiliary oligonucleotide can be sequenced with the Sanger dideoxy method (PNAS USA, 74:5463-5467 (1977)).

The publication is further directed to processes utilizing a thermostable RNA ligase such as RM 378 RNA ligase or TS2126 RNA ligase for analysis of single nucleotide polymorphisms and detection of mutations. The enzyme can be used in ligase-polymerase mediated genetic bit analysis of single nucleotide polymorphisms. Essentially, two oligonucleotide primers are hybridized to adjacent portions of a target molecule, separated by one nucleotide. One of the primers can be immobilized to a solid support such that the hybridization products will be immobilized. Following immobilization, polymerase extension with corresponding nucleoside triphosphate species, complementary to the nucleotide of said pre selected site, is performed to fill the space between the primers. RNA ligase is then used in the ligation of the extended primer with the downstream primer. Either one of the primers or the dNTP can be labeled for the detection of the extension-ligation product.

In another method of the publication, the RNA ligase can be used for detection of mutations, *i.e.,* in direct sequence identification of mutations by cleavage and ligation associated mutation-specific sequencing. The DNA molecule, containing mutations (single base substitutions, insertions, deletions) is immobilized to a solid support. Oligos which do not contain the alteration, are hybridized to the immobilized DNA molecule. Thus, heteroduplex is formed at the mismatch site. In the next step, the hybrids are treated with enzymes such as resolvases, mismatch repair proteins, nucleotide excision repair proteins or combinations thereof so that one or both DNA strands are cleaved within, or in the vicinity of the mismatch region. Example of a resolvase is endonuclease VII from bacteriophage T4. Examples of mismatch proteins are MutY from *E. coli* and the MutS, MutL, and MutH system in *E. coli.* Examples of nucleotide excision repair proteins are UvrA, B, C and D. The hybrids formed between the wild-type DNA and the altered DNA (with mutations) are then dissociated by denaturation, and the wild-type DNA and any cleavage product of the target DNA are removed by washing. Then the immobilized remaining target DNA is ligated with the RNA ligase to an oligonucleotide linker of predetermined sequence. This linker serves as a binding site for a sequencing primer. The sequence of the DNA immediately adjacent to the ligated oligonucleotide is then determined by sequence analysis such as by using the Sanger dideoxy method.

The publication further pertains to processes wherein a thermostable RNA ligase is used in the processing of detector molecules, such as in a SELEX process and for Q-beta technology (Ellington and Szostak, Nature, 346:818-22 (1990)). The detector molecules can be used for the detection of any analytes with RNA affinity such as proteins, nucleotides or amino acids, vitamins, antibiotics, carbohydrates, to which they form a complex through non-nucleic acid base pairing interactions. They can be used in the diagnosis of cancer, infectious and inherited diseases. Each detector molecule consists of three functional parts, each serving a special purpose: one is ligand with high affinity to the target analyte, one is ligated to the corresponding part in the second molecule, one is a template that can be amplified by Q-beta replicase after the ligation of two RNA molecules by RNA ligase. To select specific detector molecules against a defined analyte a library of RNA molecules consisting of the three functional parts are added to a sample with pure analyte. RNA-molecules containing functional part with high affinity to the analyte, bind and form a RNA-target molecule complex. RNA-ligase is then used to ligate two RNA molecules in the complex. Consequently a template for the Q-beta replicase is formed, which enables it to replicate the detector molecule. The molecule can be amplified further by reverse transcriptase and polymerase and cloned and sequenced to analyze the composition of the detector molecule. The RNA molecules contain recognition sites for ribozymes. Following ligation, the sample is treated with ribozymes, which digests all unbound ligated RNA molecules. The specific RNA detector molecules can be produced by transcription of complement DNA sequences in plasmid downstream from promotor such as the T7 promotor. In detection assays they are added to the sample. Then RNA ligase is added for the ligation of the two RNA molecules to form template for the Q-beta replicase. Then the molecule is amplified with Q-beta-replicase, and further with reverse transcriptase and polymerase.

The following Examples are offered for the purpose of illustrating the present invention and are not to be construed to limit the scope of this invention.

### Examples

### Example 1: Expression and purification of RM378 RNA ligase

A clone containing the gene for RM378 RNA ligase (pBTac1expression vector in Topo cells) was cultivated at 37° C and induced with IPTG at OD₆₀₀ = 0.4. The cells were harvested and disrupted by sonication. The crude cell extract was centrifuged at 10,000 r.p.m. for 1 hour in JA 25.50 rotor. The supernatant was collected and ammonium sulfate added to 30%. The solution was stirred at 4°C for 40 minutes and centrifuged 10,000 g for 1 hour in JA 25.50 rotor. The protein pellet was dissolved in 20mM Tris pH 8 and centrifuged in JA 25.50 at 20.000 rpm for 1 hour. The supernatant was run through Hiprep 26/10 desalting column (Pharmacia) in 20mM Tris pH 8. The protein was then run on ResQ column (Pharmacia) and eluted with KCl. The majority of the ligase eluted in a single peak, wich was collected. The ligase was concentrated with ammonium sulfate precipitation and dialysed against 20mM Tris pH 8. The protein was stored in 10mM Tris pH 8, 50mM KCl, 1mM DTT, 0,1mM EDTA and 50% glycerol.

### Example 2: pH optimum of RM378 RNA ligase

A pH activity profile was determined using two different buffers: Tris HC1 and MOPS, 500 mM stocks. Both buffers were made over the pH range 4-11. MOPS stock buffers were calibrated at room temperature but Tris at 55° C due to the drastic effect of temperature on its pH. The reaction mixtures (10 µl) were prepared as follows:

| | |
|---|---|
| MOPS or Tris HCl | 50 mM at pH 4-11 |
| ATP | 1 mM |
| MgCl | 10 mM |
| BSA | 25 µg/mL |
| DTT | 10 mM |
| ³²P 5' labeled rA20 oligo | 10 µM |
| RM378 RNA ligase | 0.45 µM concentration |

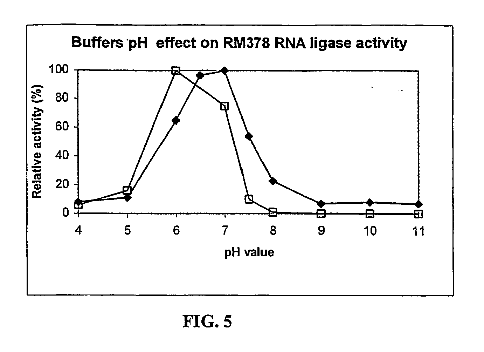

Each mixture was incubated at 55° C for 1 hour, and the reaction terminated by heating at 95°C for 5 minutes. 30 µl SAP cocktail which includes 5U Shrimp alkaline phosphatase (SAP) in 1× SAP buffer (20mM Tris-HCl (pH 8.0), 100mM MgCl₂) (USB Corp. Cleveland, Ohio) was then added and incubation continued for 3 hours at 37°C. After the incubation period, 10 µl where spotted on DE81 filters (Whatman plc. Kent, UK), washed twice in 500mM Phosphate buffers (pH 7) and dried. The filters were transferred to liquid scintillation counter vials, 5 ml OptiGold cocktail added and the filters counted for radioactivity in a liquid scintillation counter (Packard-Tricarb). The results are indicated in FIG.5. The optimum pH is 6.5 and 7 for Tris and MOPS, respectively. Due to temperature tolerance, MOPS was used in subsequent experiments. FIG. 5 depicts the relative percentage activity of RM378 RNA ligase as a function of pH using MOPS Buffer and Tris HCl buffer.

### Example 3: Temperature optimum of RM378 RNA ligase and T4 RNA ligase

Reactions were incubated at different temperatures as follows: 4°C, 20°C, 30°C, 37°C, 50°C, 55°C, 60°C, 65°C, 70°C, 80°C and 90°C for RM378 RNA ligase and 4°C, 20°C, 37°C, 45°C, 55°C and 65°C for T4 RNA ligase (New England Biolabs, Bedford, MA). The reaction mixtures (10 µl) were as follows:
For RM 378 RNA ligase:

| | |
|---|---|
| MOPS | 50 mM (pH 7.0) |
| ATP | 1 mM |
| MgCl | 10 mM |
| BSA | 25 µg/ml |
| DTT | 10 mM |
| ³²P 5' labeled rA20 oligo | 10 µM |
| | |
| RM378 RNA ligase | 0.45 µM (0.2µg) µM concentration |

For T4 RNA ligase:

| | |
|---|---|
| MOPS | 50 mM (pH 7.8) |
| ATP | 1 mM |
| MgCl | 10 mM |
| BSA | 25 µg/ml |
| DTT | 10 mM |
| ³²P 5' labeled rA20 oligo | 10 µM |
| RM378 RNA ligase | 0.45 µM (0.2µg) µM concentration |

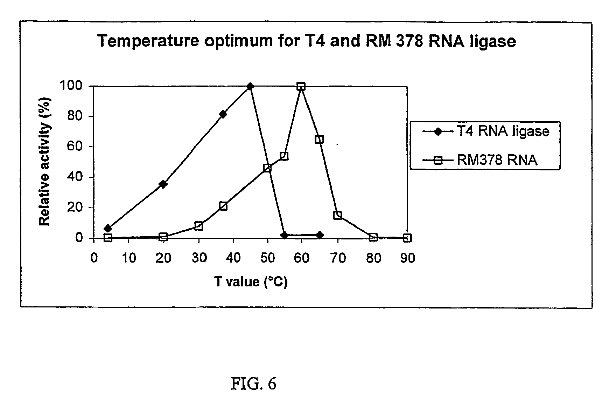

Each mixture was incubated at given temperature for 1 hour, and the reaction was terminated by heating at 95°C for 5 minutes. 30 µl SAP cocktail which includes 5U Shrimp alkaline phosphatase (SAP) in 1x SAP buffer (20mM Tris-HCl (pH 8.0), 100mM MgCl₂) (USB Corp. Cleveland, Ohio) and was then added and incubation continued for 3 hours at 37°C. Then, 10 µl were spotted on DE81 filters, washed twice in 500 mM Phosphate buffers (pH 7) and dried. The filters were transferred to liquid scintillation counter vials, 5 ml OptiGold cocktail added and the filters counted for radioactivity in a liquid scintillation counter (Packard-Tricarb). The results are indicated in FIG. 6. The RM378 RNA ligase (squares) has temperature optimum at 60°C but is active from 40-70° C, whereas the T4 RNA ligase (diamonds) has temperature optimum at 37-45°C but loses all activity at 55°C. The T4 RNA ligase is not thermostable and has a different temperature profile compared to the RM378 RNA ligase. The T4 RNA ligase has no activity at the optimum temperature for the RM378 enzyme. By optimizing the buffer, by lowering the DTT to 1 mM and MgCl₂ to 5 mM (data not shown) and 1 hour incubation times, we were able to get temperature optimum up to 64°C but the enzyme is not stable at that temperature and looses activity when the incubation time is longer than 1 hour (data not shown).

### Example 4: Thermostability of RM378 RNA ligase

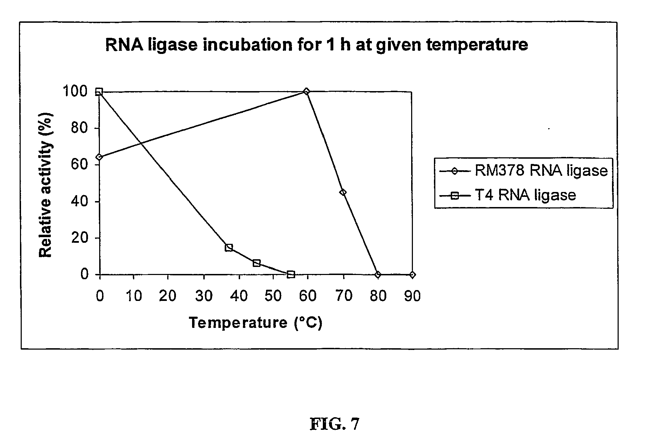

Thermostability of the RM378 RNA ligase was determined by investigating RM378 RNA ligase ability to withstand denaturation after incubation at different temperatures. The reaction mixtures (as is described in Example 3) without the template were incubated for 1 hour at 60-90°C. The rA20 template was then added and incubated at 60°C for 30 minutes. Each reaction was then terminated by heating at 95°C for 5 min, washed and counted for radioactivity as described previously. To study the stability of T4 RNA ligase, the same experiment was done, but the reactions were incubated at 37°C, 45°C and 55°C for 1 hour and then the template was added and incubated for 60 minutes at 37°C. The reaction was terminated by heating at 95°C for 5 minutes, washed and counted for radioactivity. The results are shown in FIG. 7.

RM378 RNA ligase maintains stability at 60°C but starts to lose activity at 70°C. Only 40% activity remains after 1 hour at 70°C. All activity was lost at 80°C. The starting point (zero point) on the graph corresponds to the activity of the enzyme measured directly after the enzyme was stored in a -20°C freezer. As is shown in FIG. 7, the T4 RNA ligase loses 80% of its activity after being incubated at 37°C for 1 hour in the reaction buffer. Note that the enzymes are without template which may affect their stability. However, under the given conditions, the RM378 enzyme becomes more active whereas theT4 enzyme rapidly loses activity at the same temperature. The MOPS buffer was also subjected to these conditions and the enzyme and template added afterwards. There was less than a 10% decrease in activity after the buffer had been heated at 90°C for 1 hour (data not shown).

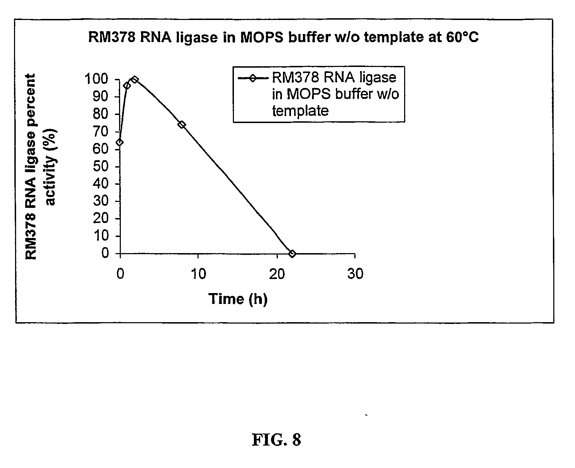

Thermostability at 90°C over time was also monitored but at 5 minutes at 90°C, the RM378 RNA ligase enzyme lost all activity (data not shown). Stability at 60°C over longer time was also monitored. As shown in FIG. 8, the enzyme is stable at 60°C for at least few hours and shows clear signs of thermoactivation. Note that the enzyme is incubated without template in these experiments. Data from dA20 oligo ligation over time indicates that the enzyme is stable in the presence of DNA template for at least 8 hours.

### Example 5: Ligation of oligonucleotides using RM378 RNA ligase and T4 RNA ligase

The ability of RM378 RNA ligase and T4 RNA ligase to catalyze ligation of defined oligoribonucleotide (5' phosphorylated rA20 mer) and deoxyribonucleotide (5' phosphorylated dA 20 mer)) substrates was tested.

### Activity measurements on ribonucleotide (RNA) substrate

The ligation of a rA20 oligoribonucleotide (RNA) or a 17 n.t. RNA oligoribonucleotide (5'P-agcgtttttttcgctaa (SEQ ID NO: 5) was measured using RM378 RNA ligase and T4 RNA ligase. This corresponds to an end-to-end ligation and consequently circularization of the substrate. The reaction was followed by taking samples at multiple time periods and terminate by heating at 95°C for 5 minutes.

The reactions (10 µl) were as follows:

| | |
|---|---|
| MOPS | 50 mM (pH 7.0) |
| ATP | 1 Mm |
| MgCl₂ | 5 mM |
| BSA | 25µg/ml |
| DTT | 1mM |
| ³²P 5' labeled RNA oligo | 10 µM |
| RM378 RNA ligase | 0.45 µM concentration |

The T4 RNA ligase (New England Biolabs; Bedford, MA) reactions were done according to the manufacturer's instructions, with supplied buffer (50 mM Tris-HCl pH 7.8, 10 mM MgCl₂, 1 mM ATP, and 10 mM DTT) at 37°C with the same amount of ³²P 5' labeled rA20 oligo and protein concentration (measures with Bradford assay as 2 mg/ml, after running 3 µl of the T4 RNA ligase on 10 % PAGE gel to observe one major band about 50 kDa). After the heating, 30 µl SAP cocktail (5 U) was added and the mixture incubated at 37°C for 3 hours. After the incubation, 10 µl were spotted on DE81 filters, washed twice in 500 mM Phosphate buffers (pH 7) and dried. The filters were transferred to Liquid scintillation counter vials, 5 ml OptiGold cocktail added and the filters counted for radioactivity as described previously.

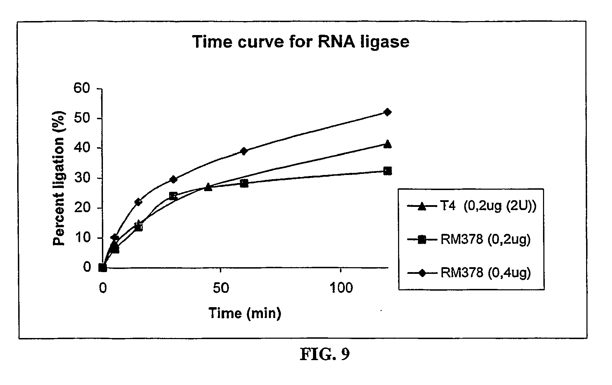

The results shown in FIG. 9 using ribonucleotide (RNA) substrates demonstrate that the RM378 RNA and T4 RNA ligase have similar activity under the given assay, ligating about 30-40 % of the total RNA in 1 hour. T4 RNA ligase looks more active, but the RM378 can increase its ligation when protein concentration is increased. The less specific activity could be a result of the thermoactivity of the RM 378 RNA ligase enzyme.

### Activity measurements on deoxyribonucleotide (DNA) substrates

Samples were removed from a reaction mixture at multiple time periods and terminated by heating at 95°C for 5 minutes.

The reaction mixture (10µL) was prepared as follows:

| | |
|---|---|
| MOPS | 50 mM (pH 7.0) |
| ATP | 1 mM |
| MgCl | 10 mM |
| BSA | 25 µg/ml |
| DTT | 10 mM |
| ³²P 5' labeled dA20 oligo | 10 µM RM378 |
| RNA ligase | 1.0 µM concentration |
| H20 to 10 µl volume. | |

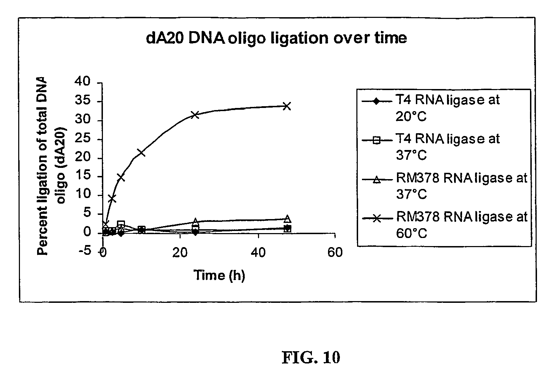

RM378 RNA ligase was assayed at 37 and 60°C. The T4 reactions were done according to the manufacturer's instructions, with supplied buffer at 20°C and 37°C with the same amount of ³²P 5' labeled dA20 oligo and protein concentration. The processing of the samples were done as described in the rA20 activity assay. As shown in FIG. 10, the results with deoxyribonucleotide (DNA) substrates show that the RM378 RNA ligase is much more active on DNA than the T4 RNA ligase. Note that some chemicals have been reported to increase the T4 RNA ligase activity on DNA, this includes 10-25% PEG6000 and hexamine cobalt chloride.

To demonstrate that RM378 RNA ligase could ligate larger ssDNA molecules a ligation of a (5'
cggcgaattctttatgggtccggaaaccctgtgcggtgctgaactggttgatgctctgcaattcgtttgcggtgatcgtggtttctac ttcaa-3)', (SEQ ID NO: 6) was done under the following reaction condition:

| | |
|---|---|
| RM378 RNA ligase | 2.0 µM (1µg) |
| 5 x MOPS buffer | 2µl (50 mM MOPS pH 7, 5 mM MgCl₂, 1mM DTT, 1 mM ATP and 25 µg/ml BSA) |
| ³²P 5' labeled ssDNA oligo | 10 µM |
| PEG6000 | 0-30% w/v |
| H₂O to 10 µl | |

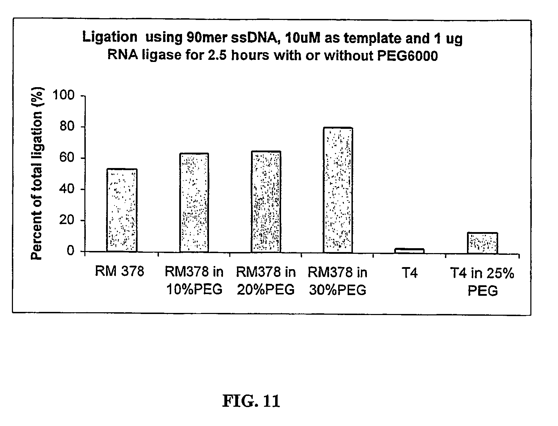

The reaction was incubated for 2.5 hours at 60°C, and subjected to phosphatase resistance assay as described above. The same was done for T4 (same amount of enzyme) RNA ligase at 22°C with 0 and 25% PEG 6000. As seen in FIG. 11 the RM378 RNA ligase have good activity on the ssDNA, and PEG6000 helps the ligation as known for T4 RNA ligase but the activity is still over 50% of total ssDNA without PEG6000. The T4 DNA ligation activity is low and even only about 15% with PEG6000. Note that T4 RNA ligase have been reported to have higher activity when hexamine cobalt cloride is added to DNA ligation mixture.

To demonstrate that the RM378 RNA ligase could ligate two DNA molecules together a ³²P-5'-dA10dd (dideoxy dA, lacking C3 -OH group) or ³²P-5'-dA10- Amino modifier (C3-NH₃⁺) oligo (that can not self ligate) was ligated to dA10 with and without PEG6000 as described:

| | |
|---|---|
| RM378 RNA ligase | 3.3 µM (1,45µg) |
| 5 x MOPS buffer | 2µl (50 mM MOPS pH 7, 5 mM MgCl2, 1mM DTT, 1 mM ATP and 25 µg/ml BSA) |
| ³²P 5' labeled ssDNA oligo | 10 µM (donor) |
| 5' defosforylated oligo | 10 µM (acceptor, RNA or DNA) |
| PEG6000 | 0 or 25% w/v |
| H₂O to 10 µl | |

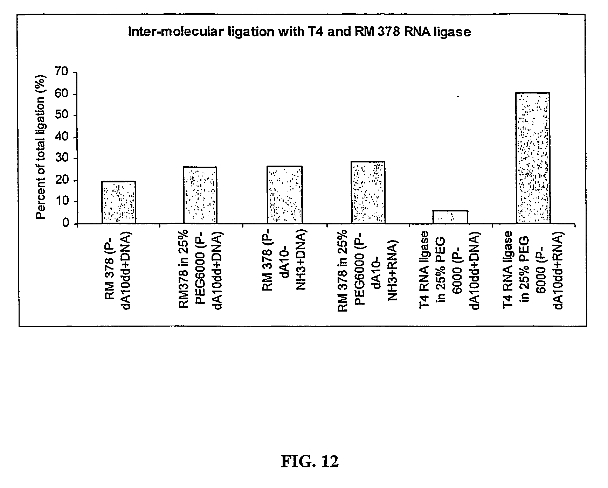

The reaction was incubated for 1 hour at 64°C, and subjected to phosphatease resistance assay as described above. The same reaction was prepared for T4 (same protein amount) RNA ligase at 22°C with 25% PEG 6000. As seen in FIG. 12 the RM378 RNA ligase has good activity on the ssDNA, and PEG6000 helps the ligation as known for T4 RNA ligase. The T4 DNA ligation activity is low when ligating to a DNA acceptor but is ore active when ligating to a RNA acceptor. The RM378 RNA ligase does not show discrimination of this kind, resulting in very similar ligation to RNA or DNA acceptor. Note that T4 RNA ligase has been reported to have higher activity when hexamine cobalt cloride is added to DNA ligation mixture.

### Example 6: DNA adenylation using modified nucleotides

RM 378 RNA ligase was used with modified nucleotides to determine its use in modification of nucleic acids such as labeling. ATP was substituted with 3'NH₂-3' dATP. The reaction was done in the MOPS buffer using 2.0 µM RM378 RNA ligase and 10 µM rA20 or dC14-ddC template in 10 ml reaction volumes, incubated for 8 hours at 60°C, and then put on ice. The samples were desalted using ZipTip (Millipore, Bedford, MA) and resuspended in 50% acidonitrile. The samples were spotted onto an AnchorChip^{™} (Bruker Daltonics) with a 400 µm anchor. The matrix employed was 7 mg/mL of 3-hydroxypicolinic acid (3-HPA) and 0.7 mg/mL ammonium citrate (dibasic). Mass spectra were recorded and analyzed on a Bruker Reflex III (Bruker Daltonics) that was operated in the linear mode.

RM378 RNA ligase can use modified nucleotides instead of ATP for adenylation of a nucleic acid (Mass spectra results not shown). DNA is less efficient as a substrate, compared to RNA, but adenylation of DNA is still clearly detectable as (note that this C15 substrate has a dideoxynucleotide on its 3' end and possible interference of this on the adenylation reaction is unknown).

The mass spectra after enzyme-catalyzed adenylation of the 5'P-dC14ddC (DNA) substrate using 3'NH₂-3'-dATP was also done (results not shown).

### Example 7: DNA ligation

DNA ligation was done for MALDI - TOF as described in Example 5, using 2.0 µM RM378 RNA ligase and 25 mM 5'P-dC15 and 5'P-dC5 oligodeoxyribonucleotides as substrates. The reaction was carried out at 60°C for 8 hours. After incubation, the mixture was desalted and analyzed as described in Example 6.

The results demonstrate that the RM378 RNA ligase can be used for single-stranded DNA ligation (data not shown). The mass spectra indicated that the oligos are ligated in three ways: i) 5'P-dC5-dC15; ii) 5'P-dC5-dC5-dC15; and iii) 5'P-dC15-dC15. It is also demonstrated that the ligase can do multiple ligations, *i.e.*, the product of one ligation reaction can be used as substrate for a subsequent reaction. The ligation reaction can also result in circularization of one of the substrates (rather 5'P-dC15 than 5'P-dC5). Although not seen in the mass spectra, this substrate can compete with the linear ligations.

### Example 8: Single primer gene retrieval by using T4 and RM378 RNA ligases

The purpose of this study was to analyze the RM378 RNA ligase and compare it to the T4 RNA ligase for random gene retrieval by the single primer method as described in the schematic of FIG. 1. This method is based on the principle of using a RNA ligase to ligated a short oligonucleotide to long single stranded PCR products, obtained by single primer PCR.

### Environmental sample collection and DNA extraction

An environmental sample (microbial biomass and water sample) was collected from a basin of an alkaline hot spring (pH 8.5) at 80°C. In order to extract the microorganisms from the sediment and biomass, the biomass and the spring water were vigorously mixed together before the DNA isolation. Genomic DNA from the hot spring biomass was extracted as described by Marteinsson, et al., Appl. Environ. Microbiol., 827-833 (2001b).

### Construction of a degenerated primer

A random degenerated primer (degeneracy of 32) was constructed. The primer was degenerate at the 3' core region of length 11 bp but it was non-degenerate at the 5' region (consensus clamp region) of 29 bp. The primer was Am508 (5'-
GATATTTAATATGT1TAGCTGCATCAATTckraanccrtc-3'; (SEQ ID NO: 7). Letters in lower case correspond to the core region, and upper case letters to the consensus clamp region.

### Linear PCR with a single degenerate random primer

The DNA from the environmental sample was used as a template for the primer Am508. The primer was biotin labeled at the 5 end (MWG Biotech, Ebersberg, Germany). The PCR was carried out in 50 µl reaction mixture containing 1-100 ng of genomic DNA (dilutions used), 0.2 µM Am508, 200 µM of each dNTP in 1X DyNAzyme DNA polymerase buffer and 2.0 U DyNAzyme DNA polymerase (Finnzymes) with a MJ Research thermal cycler PTC-0225. The reaction mixture was first denatured at 95°C for 5 min, followed by 40 cycles of denaturing at 95°C (50 seconds), annealing at two different temperatures (44°C and 50°C) for 50 seconds and extension at 72°C (2 minutes). Samples were loaded on 1% a TAE agarose gel to identify high priming. The samples with no PCR products from the different annealing temperatures were selected for re-amplification. PCR purification and immobilization of single stranded PCR products. To remove excess of biotin labeled primers, nucleotides and polymerase, the PCR samples were passed through QIAquick PCR purification spin columns (QIAGEN, Germany) by following the manufacturers instructions. Before the purification, samples from the different annealing temperatures were pooled. The samples were eluted with 30 µl of H₂O and then the biotin labeled PCR products were immobilized by using 150 µg of streptavidin-coated magnetic beads (Dynal, Oslo, Norway) according to the instructions of the manufacturer. The captured biotin labeled PCR products were resuspended in 5 µl of dH₂O. The immobilized single stranded DNA was then subjected to different ligation reactions as described below. Ligation of an adaptor (oli10) to the single stranded biotin labeled PCR products was done using T4 RNA ligase and RM378 ligase.

In the presence of 20 U of T4 RNA ligase (New England BioLabs, Beverly, MA, USA),1X T4 RNA ligation buffer (50 mM Tris-HCl, pH 7.8, 10 mM MgCl 2, 10 mM DTT and 1 mM ATP), 10% PEG8000, 50 nM of the adaptor 5'-phosphorylated oligodeoxyribonucleotide oli10 (5'-AAGGGTGCCAACCTCTTCAAGGG-3') (SEQ ID N0:8) was added to the captured DNA in a final volume of 20 µl. The mixture was incubated at 22°C for 20 hours. Before the ligation reaction, the immobilized DNA was heated for 1 minute at 90°C.

In the presence of 0.5 µg of RM378 RNA ligase (in 50% glycerol), 1X MOPS ligation buffer (50 mM MOPS, pH 7.0, 10 mM MgCl₂, 10 mM DTT, 0.5 µg BSA and 1 mM ATP), 10 % PEG8000, 50 nM of the adaptor 5'-phosphorylated oligodeoxyribonucleotide oli10 (5'-AAGGGTGCCAACCTCTTCAAGGG-3') (SEQ ID NO: 8) was added to the captured DNA in a final volume of 20 µl. The mixture was incubated at 60°C for 20 hours. Before the ligation reaction, the immobilized DNA was heated for 1 minute at 90°C.

### Re-amplification PCR from the ligation reaction

The exponential re-amplification PCR was carried out in 50 µl reaction mixture containing 2 µl ligation mixture, 1.0 µM unlabelled primer Am508, 1.0 µM oli11 (5'-CTrGAAGAGGTTGGCACCCT-31 (SEQ ID NO: 9) which is complementary to oli10, 200 µM of each dNTP in 1X DyNAzyme DNA polymerase buffer and 2.0 U DyNAzyme DNA polymerase (Finnzymes, Espoo, Finland) with a MJ Research thermal cycler PTC-0225. The reaction mixture was first carried out by denaturing at 95°C for 5 minutes, followed by 30 cycles of denaturing at 95°C (0:50 minutes), annealing at 55°C for 50 seconds and extension at 72°C (2 minutes). This was then followed with a final extension for 7 minutes at 72°C to obtain A overhangs. Control PCRs were also performed with only primer Am508 or only oli11 under the conditions given above.

### Analyzing, purification and cloning of the PCR product

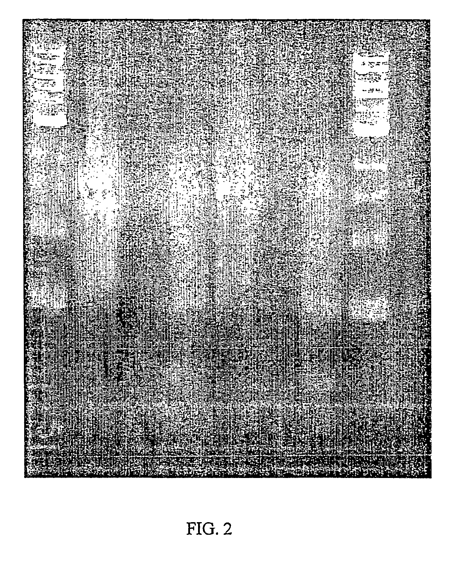

Seven microliters of the PCR re-amplification products were taken for 1% TAE agarose gel electrophoresis to confirm the identity of the PCR products and the patterns compared between the control PCRs (primer Am508 or primer oli11) and the main PCRs (primer pair oli11 and Am508). Before cloning, twenty microliters of the PCR products were loaded on thick 1% TAE agarose electrophoresis gels. The same PCR pattern was obtained from the T4 and RM378 RNA ligase ligations (FIG. 2). No amplification was obtained in the oli11 control PCR. Visible amplification DNA products of 0.5- 3.0 kb (mostly smears) were observed on agarose gels in the control PCR where only the primer Am508 was used, giving a thick band at approximately 1500 bp. The main PCR (primer pair oli11 and Am508) gave amplification products of 0.2- 3.0 kb whereas four bands were-visible. Their sizes were approximately 1.5 kb, 0.5 kb and two below 0.5 kb. Compared to the control PCR of the primer Am508, three extra bands are visible, supporting that the oli10 ligation was successful. Bands and smears from the main PCR (primer pair oli11 and Am508) were purified by using spin columns, GFX PCR DNA and Gel Band Purification kit according to the manufacturer (Amersham Biosciences, H? rsholm, Denmark). The samples were eluted with 20 µl of H₂O. Then the purified PCR products (4 µl) were cloned by the TA cloning method (Zhou and Gomes-Sanchez, Curr. Issues Mol. Bio. 2:1-7(2000)). Clones were grown overnight and their inserts were amplified with M13 reverse and M13 forward primers. The PCR amplification was carried out in 15 µl, containing 0.8 µl overnight culture, 0.5 µM M13 reverse primer, 0.5 µM M13 forward primer, 200 µM of each dNTP in 1X DyNAzyme DNA polymerase buffer and 0.75 U DyNAzyme DNA polymerase (Finnzymes, Espoo, Finland) with a MJ Research thermal cycler PTC-0225. The reaction mixture was first carried out by denaturing at 95°C for 2 min, followed by 30 cycles of denaturing at 95°C (0:50 minutes), annealing at 50°C for 50 seconds and extension at 72° C (2 minutes). Prior to sequencing, the PCR products were purified with PCR Product Pre-Sequencing Kit according to the instructions of the producer (USB). Inserts in a total of 79 clones were sequenced from samples ligated with T4 RNA ligase and a total of 85 clones were sequenced from samples ligated with RM378 RNA ligases. The gene inserts were sequenced with M13 reverse and M13 forward primers on ABI 3700 DNA sequencers by using a BigDye terminator cycle sequencing ready reaction kit according to the instructions of the manufacturer (PE Applied Biosystems, Foster City, CA). All sequences were analyzed in Sequencer 4.0 for Windows (Gene Codes Cooperation, Ann Arbor, MI) and XBLAST searched (Altschul, *et al.,* 1990; Altschul, *et al.,* 1997). For both of the RNA ligases the single primer method with primer Am508 retrieved different proteins, in various lengths. Their lengths were from 150-850 bp. The sequences showed the highest sequence identity to variable proteins, *e.g.* dehydrogenases, amylases, endoglucanases, carboxylases, a kinase and an oxidase.

The main purpose of the study was to analyze the ligation efficiency of the RM378 RNA ligase, that is its efficiency to ligate a short oligonucleotide to longer single stranded PCR products, variable in length. Of the 85 sequences obtained by the RM378 ligation , 66 sequences had ligated the short oli10 oligonucleotide to its end, corresponding to 78% of the sequences. This is according to the FIG. 2 were three new bands are obtained in the PCR by the main Am508/oli11 PCR versus the control Am508 PCR. The results for the T4 RNA ligase showed that 47 out of the 79 sequences (60%) had the oli10 oligonucleotide ligated to its end.

### Example 9 Gene synthesis using RM378 RNA ligase

The purpose of the study was to determine if the RM378 RNA ligase could be used for gene synthesis where single stranded oligos of 60-150 bases in length are ligated one after another. Using this approach for the gene synthesis, thermostable enzyme can be important due to the undesirable formation of secondary structures at lower temperatures, which increases with sequence length. Therefore, gene synthesis of this kind is useful. Furthermore, the method can be used to optimize codon usage because a host's preferred codons for amino acids seem to dramatically improve gene expression. Additionally, the method described herein can be used to modify transcription promoters or translation factor sites or add or remove protein functional domains. The human insulin-like growth factor (IGFA) gene is used as a model for the gene synthesis.

### IGFA oligonucleotides synthesis

Human insulin-like growth factor (IGFA) gene (ECBI accession number P01343) was synthesized. The codon usage of the IGFA gene was changed to *E. coli* codon usage and then the gene was split up and three oligonucleotides were designed. The 5' and 3' end oligonucleotides where designed with 13-14 bases long linkers. The oligonucleotides were I1 (5'-cggcgaattctttatgggtccggaaaccctgtgcggtgctgaactggttgatgctctgcaattcgtttgcggtgatcgtggtttctac ttcaa-3), (SEQ ID NO: 6) I2 (5'-caaaccgaccggttacggttcttcttctcgtcgtgctccgcaaaccggtatcgttgatgaa-3) (SEQ ID NO: 10) and I3 (5'-tgctgcttccgttcttgcgatctgcgtcgtctggaaatgtactgcgctccgctgaaaccggctaaatctgcttaaggatcccggcg-3) (SEQ ID NO:11). The I3 oligonucleotide was biotin labeled at the 3'-end and phosphorylated at the 5 end. The oligonucleotides were manufactured by Transgenomics, Cruachem Limited (Glasgow, Scotland).

### Immobilization of biotin labeled oligonucleotide I3 with Dynabeads M-280 Streptavidin

The biotin labeled oligonucleotide I3 (10 pmoles) was immobilized by using 150 µg of streptavidin-coated magnetic beads (Dynal, Oslo, Norway) according to the instructions of the manufacturer. The captured biotin labeled olionucleotide I3 was resuspended in 5 µl of dH₂O. Before the immobilization procedure, the oligonucleotide was heated to 90°C for 1 minute. The immobilized oligonucleotide 13 was then subjected to different ligation reactions as described below.

### Ligation between oligonucleotides I3 and I2 using T4-RNA ligase or RM378 ligase

Prior to ligation, the immobilized oligonucleotides I3 and I2 were heated at 90°C for 1 minute. In the presence of 20 U of T4 RNA ligase (New England BioLabs, Beverly, MA),1X T4 RNA ligation buffer (50 mM Tris-HCl, pH 7.8, 10 mM MgCl 2, 10 mM DTT and 1 mM ATP), 10% PEG8000, 50 pmole of oligonucleotide I2 was added to the captured oligonucleotide I3 (10 pmole) in a final volume of 20 µl. The mixture was incubated at 22°C for 20 hours. The new ligation product was called oligoA-T4.

In the presence of 0.5 µg of RM378 RNA ligase (in 50% glycerol) and 1X MOPS ligation buffer (50 mM MOPS, pH 7.0, 10 mM MgCl₂, 10 mM DTT, 0.5 µg BSA and 1 mM ATP), 50 pmole of oligonucleotide I2 was added to the captured oligonucleotide 13 (10 pmol) in a final volume of 20 µl. The mixture was incubated at 60°C for 20 hours. The new ligation product was called oligoA-RM.

After the ligation and the formation of oligoAT4 and oligoA-RM, the rest of oligonucleotide I2 that was not ligated to oligonucleotide 13 was removed by washing the solution twice with 100 wl of dH₂O. After the washing, the samples were resuspended in 17 µl of dH₂O.

*Phosphorylation of the 5'end of oligoA (former 5' end of* oligonucleotide *I2) with polynucleotide kinase*

In order to subject oligoA-T4 and oligo-RM to further ligation, their 5 ends were phosphorylated in the presence of 10 U of I polynucleotide kinase (New England BioLabs, Beverly, MA) and 1X PNK buffer (70 mM Tris-HCl, pH 7.6, 10 mM MgCl₂ and 5 mM dithiothreitol) in a final volume of 20µl. The mixture was incubated at 37°C for 30 minutes. Inactivation of the enzyme was done be heating at 65µC for 20 minutes.

After the phosphorylation the solutions were washed twice with 100 µl of dH₂O as described above. After the washing, the samples were resuspended in 5 µl of dH₂O,

### Ligation between oligonucleotide I1 and oligoA-T4 using T4-RNA ligase and ligation between oligonucleotide I1 and oligoA-RM using RM378 ligase

Prior to the next ligation step, the ligation products oligoA-T4 and oligoA-RM were heated at 90°C for 1 minute as well as oligonucleotide I1 (50 pmol) in order to minimize secondary structures. The ligation reactions were as described above whereas the ligation between oligonucleotide I1 and oligoA-T4 was done with T4-RNA ligase giving the product oligoB-T4 and ligation between oligonucleotide I1 and oligoA-RM by using RM378 ligase, giving the product oligoB-RM. After the ligation, the solutions were washed twice with 100 µl of dH₂O as described above. After the washing, the samples were resuspended in 20 µl of dH₂O.

### PCR of the synthesized IGFA gene (oligoB) by two primers

Different PCR amplifications from the ligation solutions allowed to detect if the ligations were successful. This analysis was done by three different PCRs. First to check if the whole gene was formed by using primers complementary for oligonucleotides I1 and I3 (primers IGFA-r and IGFA-f which should give a band of approximately 240 bp) . The other two PCRs were to check the ligation reactions. One of the control ligation PCR was to check if the first ligation was successful by using primers complimentary for 13 and 12 (formation of oligoA: primers IGFA-r and IGFA-2f giving a band of approximately 150 bp). The other control ligation PCR was to check if the second ligation was successful by using primers complimentary for I1 and I2 (formation of oligoB: primers IGFA-2r and IGFA-f giving a band of approximately 150 bp). The PCRs were carried out in 50 µl reaction mixture containing 2 µl ligation mixture, 1.0 µM reverse primer IGFA-r (5'-CCGGGATCCTTAAGCAGATT-3)' (SEQ ID NO: 12): complementary to I3) or IGFA-2r (5-' TCATCAACGATACCGGTTTGC-3)' (SEQ ID NO: 13): complementary to I2), 1.0 µM primer IGFA-f (5'-GGCGAATTCTTTATGGGTCCGGAAAC-3)' (SEQ ID NO: 14: complementary to I1) or 1.0 µM primer IGFA-2f (5'-ACCGACCGGTTACGGTTCTTC-3)' (SEQ ID NO: 15): complementary to 12), 200 µM of each dNTP in 1X DyNAzyme DNA polymerase buffer and 2.0 U DyNAzyme DNA polymerase (Finnzymes, Espoo, Finland) with a MJ Research thermal cycler PTC-0225. The reaction mixture was first carried out by denaturing at 95°C for 5 minutes, followed by 30 cycles of denaturing at 95°C (0:50 min), annealing at 55°C for 50 seconds and extension at 72°C (2 minutes). This was then followed with a final extension for 7 minutes at 72°C to obtain A overhangs.

### Analyzing, purification and cloning of the PCR products

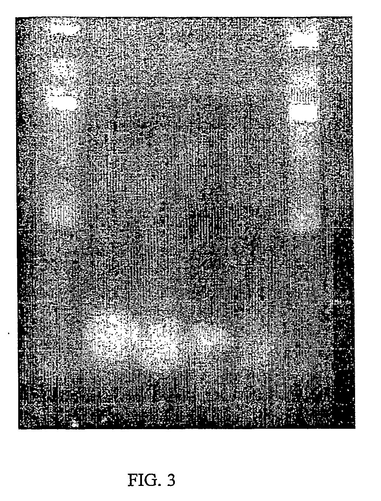

Seven microliters of the PCR reamplification products were taken for 1% TAE agarose gel electrophoresis to confirm that the two ligation reactions had occurred. Visible amplification DNA products of approximately 200 b were observed on agarose gels for both of the ligation reactions and the whole gene PCR (FIG.3). The bands were cloned to confirm the ligation reactions and the gene synthesis. Before cloning, twenty microliters of the PCR products were loaded on thick 1% TAE agarose electrophoresis gels. The bands were purified by using spin columns, GFX PCR DNA and Gel Band Purification kit according to the manufacturer (Amersham Biosciences, Hörsholm, Denmark). The samples were eluted with 20 µl of H₂O. The purified PCR products (4 µl) were then cloned by the TA cloning method (Zhou and Gomez-Sanchez, Curr. Issues Mol, Biol. 2:1-7 (2000)).

Clones were grown overnight and their inserts were amplified with M13 reverse and M13 forward primers. The PCR amplification was carried out in 15 µl, containing 0.8 µl overnight culture, 0.5 µM M13 reverse primer, 0.5 µM M13 forward primer, 200 µM of each dNTP in 1X DyNAzyme DNA polymerase buffer and 0.75 U DyNAzyme DNA polymerase (Finnzymes, Espoo, Finland) with a MJ Research thermal cycler PTC-0225. The reaction mixture was first carried out by denaturing at 95°C for 2 minutes, followed by 30 cycles of denaturing at 95°C (0:50 min), annealing at 50°C for 50 seconds and extension at 72°C (2 minutes). Prior to sequencing, the PCR products were purified with PCR Product Pre-Sequencing Kit according to the instructions of the producer (USB). The gene inserts were sequenced with M13 reverse primer on ABI 3700 DNA sequencers by using a BigDye terminator cycle sequencing ready reaction kit according to the instructions of the manufacturer (PE Applied Biosystems, Foster City, CA). All sequences were analyzed in Sequencer 4.0 for Windows (Gene Codes Cooperation, Ann Arbor, MI) and XBLAST searched (Altschul et al., J. Mol. Biol., 215:403-410 (1990); Altschul et al., Biotechniques, 15:894-904 (1997)).

The sequencing result for the first ligation reaction (ligation between 13 and 12) were according to the PCR results, that is, the RM378 ligation was successful and a correct oligoA sequence was obtained. Wrong ligation products were also obtained (see below). The sequencing results for the second ligation reaction (ligation between I2 and I1) were according to the PCR results, that is, the ligation was successful. However, not a correct sequence was obtained for this ligation with the RM378 RNA ligase, that is oligonucleotide I1 was right but it was ligated to a truncated sequence of I2. This may be explained by the fact that in the synthesis of long oligonucleotides, different forms of truncated oligonucleotides is a common artifact. For the T4 RNA ligase, right I1-I2 and I2-I3 products were formed as well as trunctated ones. The result that PCR product of I1-I2 was seen (although truncated for the RM378 RNA ligase), demonstrates that product I1-I2-I3 was actually formed. This is due to the fact that if I1-I2 was only formed but not I1-I2-I3, the I1-I2 or the I2 oligonucelotide product would have been washed away in one of the many washing steps if not ligated to 13, and therefore not observed. This means that only products ligated to I3 stay in the solution, as that is the only biotin labeled oligonucleotide.

The sequencing of the whole gene PCRs gave only I1-I3 products for both T4 and RM378 RNA ligases. However, as observed for the I1-I2 ligation, not a correct sequence was obtained for this ligation in all cases. About 40% of the I1-I3 products had a wrong sequence that is oligonucleotide I1 was normally right but ligated to a variable truncated sequences of I3. The cloning results indicate that I1-I3 products dominate the IGFA-r/IGFA-f PCR and we cannot see the Il-12-13 product, although, the 11-12 ligations show that the 11-12-13 product was actually formed as mentioned above.

Although, the I1-I2-I3 product was not obtained in sequencing, this experiment shows that with the PCRs and sequencing results, the RM378 RNA ligase (as well as T4) can be used in sequential single stranded DNA ligations to ligate long oligonucleotides together, for the purpose of gene synthesis. In order to retrieve the whole gene, different PCR methods can be used, like the known gene splicing overlap (SOE) method (Lefebvre et al., 1995, Biotechniques 19:186-8).

IGFA gene with a *E. coli* codon usage and linkers (italic and bold) at the 3'- and 5'-ends.

### RM ligations

Sequence for I1 and I2 ligation (truncated form of I2) (SEQ ID NO:21):
Sequence for 12 and 13 ligation (correct ligation) (SEQ ID NO:22-):
Sequence for I1 and I3 ligation (correct ligation) (SEQ ID NO:23):

### Example 10: RLM-RACE (RNA Ligase Mediated Rapid Amplification of cDNA Ends)

Generally, this method can be used for example to obtain 5' ends of mRNA molecules if only a part of the sequence is known. In this example, a RACE experiment was done using some components from the GeneRacer core kit (Invitrogen Inc.) plus additional components. The RNA sample used contained the control RNA provided with the GeneRacer kit.

The RNA was dephosphorylated with calf intestial phosphatase (CIP) which dephosphorylates all RNA except capped mRNA. The reaction conditions were as follows:

| | |
|---|---|
| Total RNA | 5 µg (Total RNA from HeLa cell line) |
| 10x CIP buffer | 1 µl |
| RnaseOUT(40U/ml) | 1 µl |
| CIP (10U/ml) | 1 µl |
| DEPC treated water to | 10µl |

The solution was mixed and incubated for 1 hour at 50°C, then centrifuged and put on ice. The RNA was purified using the RNeasy kit (QIAgen Inc.) according to the manufacturers instructions and resuspended in 30 µl DEPC (to remove RNAse contamination) treated water.

Decapping was done on the full length mRNA with Tobacco Acid Pyrophosphatase (TAP). The reaction conditions were as follows:

| | |
|---|---|
| CIP treated RNA | 7µl |
| 10x TAP buffer | 1µl |
| TAP(0.5U/ml) | 1µl |
| RNAseOUT (40U/ml) | 1µl |
| Total | 10µl |

The solution was mixed and incubated at 37°C for 1 hour. The RNA was then purified using the RNeasy kit (QIAgen Inc.) and resuspended in 30 µl DEPC treated water.

The GeneRacer RNA Oligo was ligated onto the decapped mRNA with RNA ligase using using T4 RNA ligase (5U per reaction) and RM378 ( 5 U per reaction), respectively. During this step, the RNA solution (7µl) was mixed with pre-aliquoted, lyophilized GeneRacer RNA oligonucelotide (0.25mg) and a second oligonucleotide 5'-CGACUGGAGCACGAGGACACUGACAUGGACUGAAGGAGUAGAAA-3') (SEQ ID NO: 17).

The RNA solution for the T4 RNA ligase ligation was heated to 65°C for 5 minutes and then spun down and put on ice, in order to minimize secondary structure. This was not done for the RNA solution for the RM378 RNA ligase reaction. The reaction solution was as follows:

| | |
|---|---|
| Decapped RNA | 6 µl |
| 10x RNA ligase buffer | 1 µl (MOPS buffer for the RM378 RNA ligase, and the supplied buffer with the GeneRacer kit for T4 RNA ligase) |
| ATP (10 mM) | 1µl |
| RNAseOUT | 1 µl (RNAse inhibitor from CHIMERx (50U) wasused at 60°C since it is stable below 70°C) |
| RNA ligase | 1 µl |
| Total | 10 µl |

The reaction was incubated at 37°C for 1 hour for the T4 RNA ligase and 1 hour at 60°C for the RM 378 RNA ligase. The RNA was then purified using the RNeasy kit (QIAgen Inc.) and resuspended in 30 ml DEPC treated water.

cDNA synthesis was performed in few steps. First, an annealing step with the following conditions:

| | |
|---|---|
| Ligated RNA | 18.4 µl |
| dT20 oligo (50 µ M) | 1.6 µl |
| Total | 20 µl |

The solution was incubated at 70°C for 10 minutes and cooled on ice.

Second, a first strand synthesis in a solution made of:

| 5x First strand synthesis buffer 6 µl | |
|---|---|
| PowerScript RT (Clontech) | 1.5 µl |
| dNTP mix (10m each) | 3 µl |
| DTT (100mM) | 3µl |
| RNAase OUT (40U/ml) | 1.5 µl |
| RNA and dT20 mixture | 15 µl |

The solution was incubated 42°C for 70 minutes and the reaction terminated by heating at 70°C for 15 minutes. The solution was then centrifuged and put on ice.
1.5 ml RNAseH solution (2U/ml, Stratagene Inc.) was then added, the solution mixed and incubated for 20 minutes at 37°C and put on ice.

Third, a polymerase chain reaction (PCR) was done (Using AmpliTaq Gold^{™} DNA polymerase (Applied Biosystems), see manufacturer instructions for details) with 0.1-1.0 µ I of the previous solution for 30 ml PCR reaction. The primers used were GeneRacer 5' Primer (SEQ: 5'-CGACTGGAGCACGAGGACACTGA-3') (SEQ ID NO: 18) or GeneRacer 5' nested primer (5'-GGACACTGACATGGACTGAAGGAGTA-3') (SEQ ID NO: 19) and GeneRacer 5' control primer B1 (Beta actin gene specific primer) (5'-GACCTGGCCGTCAGGCAGCTCG-3') (SEQ ID NO: 20). The reaction solution was made of:

| | |
|---|---|
| cDNA | 1µl |
| 10x Gold buffer | 3µl |
| MgCl2 solution | 3µl |
| AmpliTaq (5U/ml) | 0,3µl |
| dNTPs (2 mM) | 3µl |
| Water | 19.7µl |

The PCR was done according to the following program:

| Temperature | Time | Cycles |
|---|---|---|
| 94°C | 12 min | 1 |
| 94°C | 30 sec | 4 |
| 72 | 2 min | |
| 94°C | 30 sec | 4 |
| 70°C | 2 min | |
| 94°C | 30 sec | 30 |
| Gradient 55-70°C | 30 sec. | |
| 72°C | 2 min | |
| 4°C | for length of storage | |

After the reaction, 5 ml of the PCR product were run on a 0,8% agarose gel.

### Results:

A PCR product with a size similar to the expected size was generated (Figure 13). These results demonstrate that the RM378 RNA ligase can be used in a RLM-RACE procedure.

### Example 11. Cloning, expression and purification of the TS2126 RNA ligase

A clone containing the TS2126 gene was used to amplify the TS2126 gene by conventional PCR-methods. PCR products were run on a 1% agarose gel and DNA fragments of the right size cut out. The fragments were purified using GFX columns (Amersham Biosciences), according to manufacturer's instructions, and cut with restriction enzymes (BamHI and NdeI). The fragments were ligated into expression vector pET-23b (Novagen), cut with the same restriction enzymes, which was designed to add a histidine-tag to the C-terminus of the protein. The vector was then transformed into E. coli BL21-(DE3)-RIL (Strategene) and a clone selected after verification of the correct sequence by DNA sequencing. The cells were grown in a 10 L fermenter and production of the enzyme induced by IPTG in log phase. The cells were harvested and disrupted by sonication. After removal of cell debris by centrifugation was the enzyme purified using standard chromatographic techniques, first with affinity chromatography (HiTrap Chelating, Amersham Biosciences) and subsequently with gel filtration (HiPrep 26/10 Sephacryl S200 HR Amersham Biosciences) to give the final protein sample. The gene was also expressed using a Thermus host-vector system (O.H. Fridjonsson, Prokaria Ltd, personal communication) and the corresponding protein purified for comparison with the protein expressed in E. coli.

### Example 12: pH optimum of TS2126 RNA ligase:

The activity assay for RNA activity is based on the Phosphatase resistant assay developed by Silber et al (Proc.Natl. Acad Sci. U.S.A. 69:3009-3013 (1972)).

### Reaction conditions:

| | |
|---|---|
| 2 µl | 5x ligase buffer (250 mM MOPS (pH 4-11), 25mM MgCl₂, 5mM DTT, 50 mM KCl, 125 µg/ml BSA). |
| 0.04 µg | TS2126 RNA ligase. |
| 2 µl | ³²P-5'-rA20 RNA substrate (25 µM). |
| 1 µl | ATP (10mM). |
| H₂O | to 10 µl. |

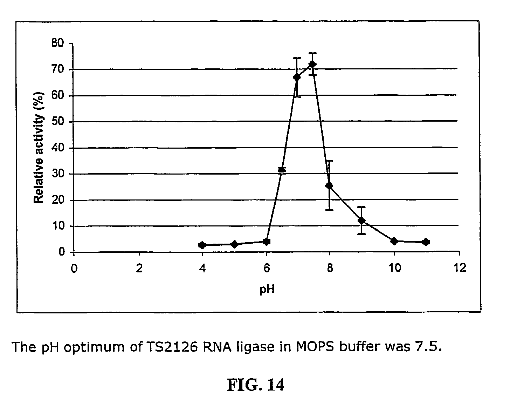

Each mixture was incubated at 60°C for 30 minutes, and the reaction then terminated by heating at 95°C for 5 minutes. 30 µl SAP cocktail, which includes 5U Shrimp alkaline phosphatase (SAP) in 1x SAP buffer (20mM Tris-HCl (pH 8.0), 100mM MgCl₂) (USB Corp. Cleveland, Ohio), was then added and incubation continued for 3 hours at 37°C. After the incubation period, 10µl where spotted on DE81 filters [Whatman plc. Kent, UK), washed twice in 500mM Phosphate buffers (pH 7) and dried. The filters were transferred to liquid scintillation counter vials, 5 ml OptiGold cocktail added and the filters counted for radioactivity in a liquid scintillation counter (Packard-Tricarb). The results are shown in Figure 14.

### Example 13: Temperature optimum of TS2126 RNA ligase:

To examine the temperature optimum of the TS2126 RNA ligase, was the ligation reaction carried out at different temperatures for 30 minutes and then the activity determined by the phosphatase resistant assay (Silber et al).

### Reaction conditions:

| | |
|---|---|
| 2 µl | 5x ligase buffer (250 mM MOPS (pH 7.5), 25mM MgCl₂, 5mM DTT, 50 mM KCl, 125 µg/ml BSA). |
| 0.04 µg | TS2126 RNA ligase. |
| 2 µl | ³²P-5'-rA20 RNA substrate (25 µM). |
| 1 µl | ATP (10mM). |
| H₂O | to 10 µl. |

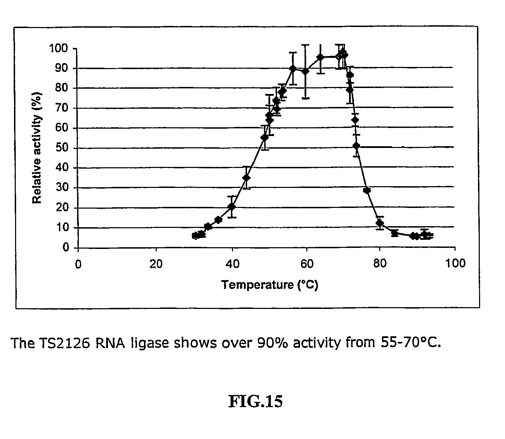

The enzymatic activity of the enzyme as function of temperature is shown in Figure 15.

### Example 14: Thermostability of the TS2126 RNA ligase:

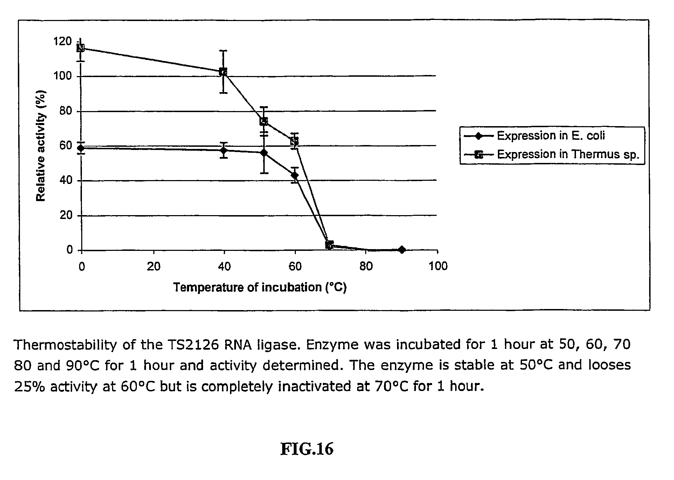

The enzyme was incubated in the reaction solution without substrate for 1 hour at 50, 60, 70, 80 and 90°C and then substrate was added and incubated at 60°C for 1 hour, and the samples processed as described above for the phosphatase resistant assay. The results are shown in Figure 16.

### Example 15: Effect of cations on the TS2126 RNA ligase activity.

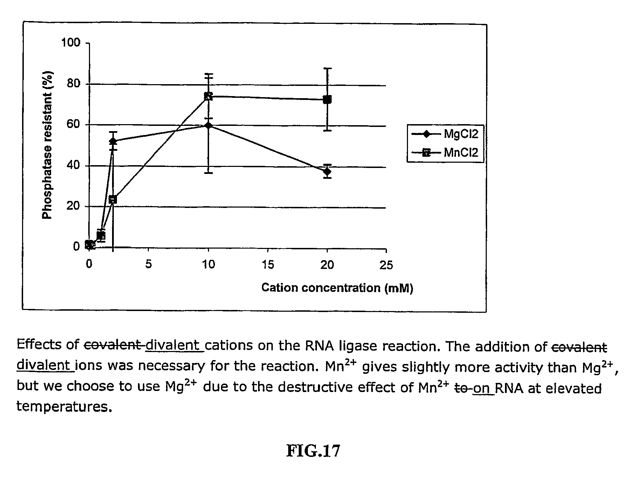

The effects of varying the concentration of divalent cations, Mn2+ or Mg2+, was studied using different concentration of respective cation in the reaction buffer and ligation reaction done as described. The results are shown in Figure 17.

### Reaction conditions:

| | |
|---|---|
| 2 µl | 5x ligase buffer (250 mM MOPS (pH 7.5), 5mM DTT, 50 mM KCl, 125 µg/ml BSA). |
| 0.04 µg | TS2126 ligase. |
| 2 µl | ³²P-5'-rA20 RNA substrate (25 µM). |
| 1 µl | ATP (10mM). |
| MgCl₂ or MnCl₂ | 0-10 mM final concentration |
| H₂O | to 10 µl. |

### Example 16: Effects of ATP concentration on the activity of TS2126 RNA ligase.

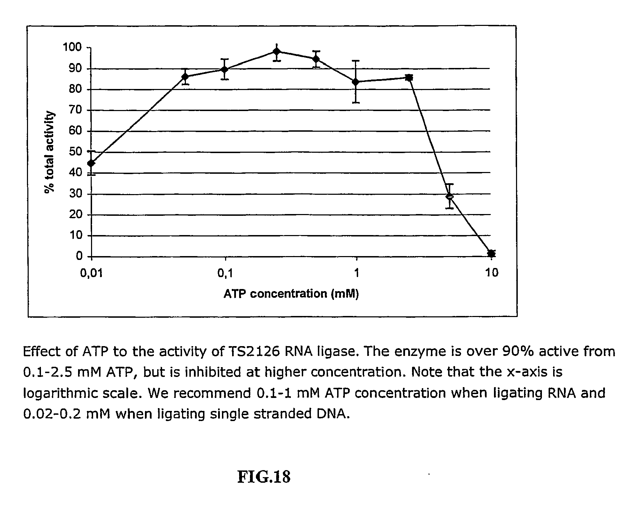

Effect of ATP concentration was studied by determining the activity of TS2126 RNA ligase with different amount of ATP. The results are shown in Figure 18. We recommend 0.1-1 mM ATP concentration to be used when ligating RNA and 0.02-0.2 mM when ligating single stranded DNA.

### Reaction conditions:

| | |
|---|---|
| 2 µl | 5x ligase buffer (250 mM MOPS (pH 7.5), 25mM MgCl₂, 5mM DTT, 50 mM KCl, 125 µg/ml BSA). |
| 0.04 µg | TS2126 ligase. |
| 2 µl | ³²P-5'-rA20 RNA substrate (25 µM). |
| 0.01-10mM | ATP (final concentration). |
| H₂O | to 10µl. |

### Example 17; Specific activity of TS2126 RNA ligase:

The specific activities of TS2126 RNA ligase and T4 RNA ligase were compared.

### Reaction conditions:

| | |
|---|---|
| 2 µl | 5x ligase buffer (250 mM MOPS (pH 7.5), 25mM MgCl₂, 5mM DTT, 50 mM KCl, 125 µg/ml BSA). |
| 0.1 µg | TS2126 ligase. |
| 2 µl | ³²P-5'-rA20 RNA substrate (25 µM). |
| 1 µl | ATP (10mM). |
| H₂O | to 10 µl. |

The samples were incubated at 60°C for 0, 2.5, 5, 15, 30,60 and 120 minutes before determining the activity.

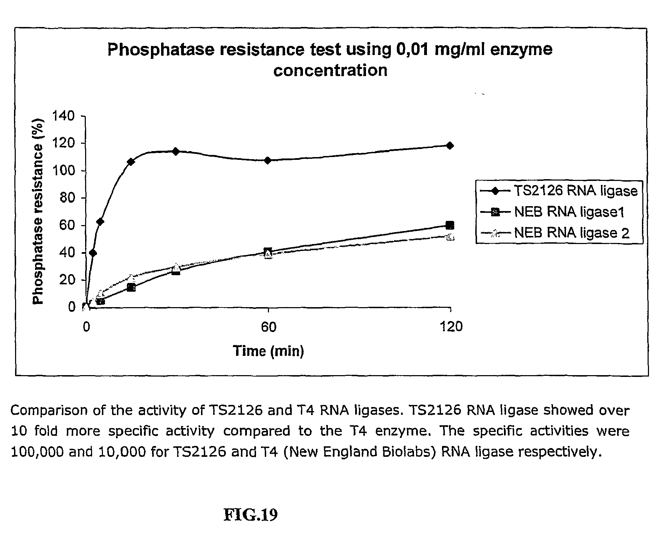

The same procedure was done with T4 RNA ligase from NEB (New England Biolabs) as described by the manufacturer, using the same amount of protein (0.1 µg) and incubating at 37°C for the same time periods. The results are shown in Figure 19.

### Example 18: Effects of protein concentration on TS2126 RNA ligase activity.

The standard RNA ligase assay was used to monitor the effect of protein concentration on the ligation of RNA substrate.

### Reaction conditions:

| | |
|---|---|
| 2 µl | 5x ligase buffer (250 mM MOPS (pH 7.5), 25mM MgCl₂, 5mM DTT, 50 mM KCl, 125 ? g/ml BSA). |
| 0.001-0.4 µg | TS2126 RNA ligase. |
| 2 µl | ³²P-5'-rA20 RNA substrate (25 µM). |
| 1 µl | ATP (10mM). |
| H₂O | to 10 µl. |

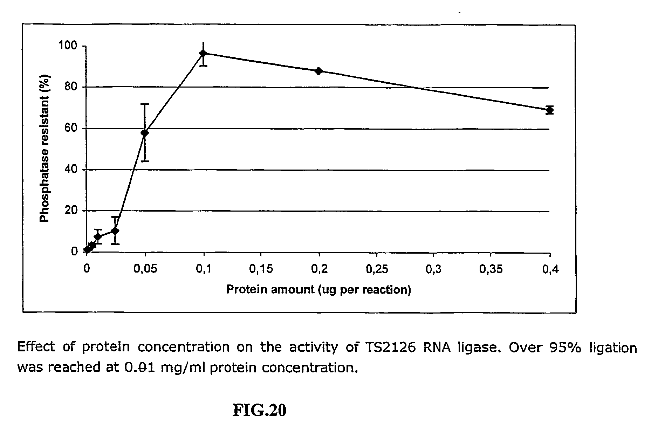

Incubated for 30 minutes at 60°C before determining the activity.The results are shown in Figure 20.

### Example 19. TS2126 RNA ligase in RLM-RACE application

RLM-RACE (RNA Ligase Mediated Rapid Amplification of cDNA Ends) is one of the major application for RNA ligase in Molecular Biology. It is used to obtain 5' ends of mRNA molecules, if only a part of the sequence is known. This experiment was done using some components from the GeneRacer core kit (Invitrogen Inc.) and additional components.

Substrate for this experiment was 100ng human testis mRNA (Ambion Inc.).

### Step 1. Defosforylation with calf intestial phosphatase (CIP) which defosforylates all RNA except capped mRNA:

### Reaction conditions:

| | |
|---|---|
| Total RNA | 100ng mRNA |
| lOx CIP buffer | 1 µl |
| RnaseOUT(40U/µl) | 1 µl |
| CIP (10U/µl) | 1 µl |
| DEPC treated water to | 10µl |

The reaction mixture was incubated for 1 hour at 50°C and then centrifuged and put on ice. The mRNA was purified with Phenol/Cloroform extraction and ethanol precipitation before resuspending in 10 µl water.

### Step 2: Decapping the full length mRNA with Tobacco Acid Pyrophosphatase (TAP):

### Reaction conditions:

| | |
|---|---|
| CIP treated RNA | 7µl |
| 10x TAP buffer | 1µl |
| TAP(0.5U/µl) | 1µl |
| RNAseOUT (40U/µl) | 1µl |
| Total | 10µl |

Mixed and incubated at 37°C for 1 hour. The RNA was purified with phenol/chloroform extraction and ethanol precipitation before resuspending in 20 µl water

### Step 3: Ligation of the Generacer RNA Oligo onto the decapped mRNA with TS2126 RNA ligase:

The ligation was done with both T4 RNA ligase (5U per reaction) and TS2126 RNA ligase (5U per reaction):
RNA (7µl) was mixed with pre-aliquoted, lyophilized Generacer RNA oligo (0.25? g), mix carefully (SEQ: 5'-CGACUGGAGCACGAGGACACUGACAUGGACUGAAGGAGUAGAAA-3').
The mRNA for the T4 RNA ligase ligation was heated to 65°C for 5 min and then spun down and put on ice in order to minimize secundary structure. This was not done for the decapped mRNA for the TS2126 RNA ligase reaction.

### Reaction conditions:

| | |
|---|---|
| Decapped RNA | 6µl |
| 10× RNA ligase buffer | 1 µl (MOPS buffer for the TS2126 RNA ligase, and the supplied buffer with the Generacer kit for T4 RNA ligase) |
| ATP (10 mM) | 1 µl |
| RNAseOUT | 1 µl |
| RNA ligase (5U/ µl) | 1µl |
| Total | 10µl |

The reaction mixture was incubated at 37°C for 1 hour forT4 RNA ligase and 1 hour at 60°C for TS2126 RNA ligase. The RNA was purified with Phenol/Cloroform extraction and ethanol precipitation and resuspended in 10 µl water.

### Step 4: cDNA synthesis

| | |
|---|---|
| Ligated RNA | 18,4µl |
| dT20 oligo | 1,6 µl (1µg) |
| Total | 20 µl |

Incubated at 70°C for 10 min and cooled on ice.

| | |
|---|---|
| 5x First strand synthesis buffer | 6 µl |
| PowerScript RT (Clontech) | 1.5 µl |
| dNTP mix (10 µ each) | 3 µl |
| DTT (100mM) | 3 µl |
| RNAaseOUT (40U/ µl) | 1.5µl |
| RNA and dT₂₀ mixture | 15µl |

After incubation at 42°C for 70 min was the reaction terminated by heating at 70°C for 15 min and then centrifuged and put on ice. Wethen used 0.1-1.0 µl for 30 µl PCR reaction using generacer 5' Primer (SEQ: 5'-CGACTGGAGCACGAGGACACTGA-3') or GeneRacer 5' nested primer (SEQ: 5'-GGACACTGACATGGACTGAAGGAGTA-3') and GeneRacer 5' control primer B1 (Beta actin gene specific primer) (SEQ: 5'- GACCTGGCCGTCAGGCAGCTCG-3'). The PCR protocol (Using AmpliTaq Gold ® (Applied Biosystems)was as follows, see manufacturer instructions for details):

| | |
|---|---|
| cDNA | 1µl |
| 10× Gold buffer | 3µl |
| MgCl₂ solution | 3µl |
| AmpliTaq (5U/µl) | 0,3µl |
| dNTPs (2 mM) | 3µl |
| Water | 19,7µl |

### PCR program:

| Temperature | Time | Cycles |
|---|---|---|
| 94°C | 12 min | 1 |
| 94°C | 30 sec | 4 |
| 72 | 2 min | |
| 94°C | 30 sec | 4 |
| 70°C | 2 min | |
| 94°C | 30 sec | 30 |
| Gradient 55-70°C | 30 sec | |
| 72°C | 2 min | |
| 4°C | forever | |

5 µl of the PCR product were run on a 0,8% agarose gel after the PCR.
As seen by the results in Figure 21, we obtained a PCR product of similar size as expected.
We therefore conclude that we can use the TS2126 RNA ligase in a RLM-RACE procedure.

### Example 20: The use of TS2126 RNA ligase in inverse RACE protocol

DNA circularisation of large templates for inverse RACE on the cDNA level was done by making beta actin cDNA from 500 ng testis mRNA (Ambion Inc.). Phosphorylated internal primer 5'P-B1 (5'P-GACCTGGCCGTCAGGCAGCTCG) was used in the cDNA synthesis using AMV first strand synthesis kit (Invitrogen Inc) as recommended by the manufacturer and the RNA digested with RNAseH (Ambion Inc.) as recommended by the manufacturer. The ca. 800 base long beta actin specific cDNA was then purified on PCR purification column (Qiagen Inc.), as recommended by the manufacturer, and resuspended in 30 µl water. 10 µl of the beta actin cDNA were used for each ligation.

The samples were then ligated using both 50U T4 RNA ligase and TS2126 RNA ligase in standard buffers (with PEG6000 and 1 mM hexamine cobalt chloride for T4 RNA ligase) and 20 µ M ATP in a 20 µl volume at 22°C and 60°C respectively, for 12 hours.
The ligated samples were amplified using internal inverse primers (InvA:
CTGGACTTCGAGCAAGAGATG and invB: GCCGTTGTCGACGACGAGC) over the ligation border. The reaction mixture was made as follows:

| | |
|---|---|
| Ligated cDNA | 3µl |
| 10× Gold buffer | 3µl |
| MgCl₂ solution | 3µl |
| AmpliTaq (5U/µl) | 0,3µl |
| dNTPs (2 mM) | 3µl |
| Water | 17,7µl |

### PCR program:

| Temperature | Time | Cycles |
|---|---|---|
| 94°C | 12 min | 1 |
| 94°C | 30 sec | 35 |
| 55 | 30 min | |
| 72°C | 1 min | |
| 4°C | forever | |

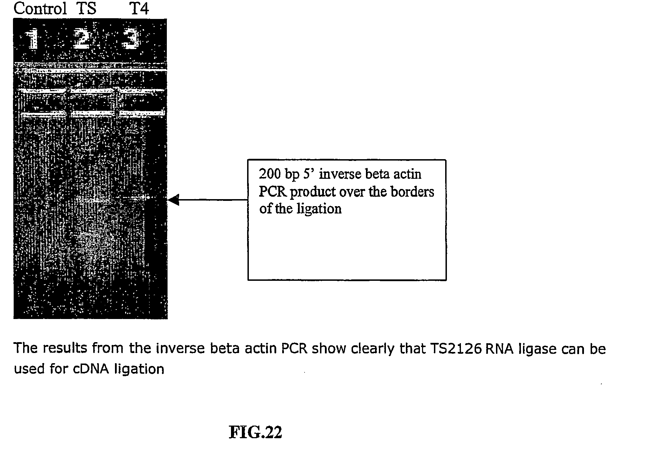

8 µl of the PCR product were run on a 2% agarose gel. The results are shown in figure 22.

### Example 21: DNA intra-molecular ligations

RNA ligases also show ligation activity on ssDNA. To measure the activity on ssDNA was 5'P-d(N₂₂) oligonucleotide ligated (circularised) under the following conditions.

### Reaction conditions:

| | |
|---|---|
| 4 µl | 5x ligase buffer (250 mM MOPS (pH 7.5), 25mM MgCl₂, 5mM DTT, 50 mM KCl, 125 µg/ml BSA). |
| 2 µg | TS2126 RNA ligase (0.1 mg/ml final concentration). |
| 2 µl | ssDNA substrate (1,5 or 50µM) |
| 1µl | ATP (1mM). |
| H₂O | to 20 µl. |

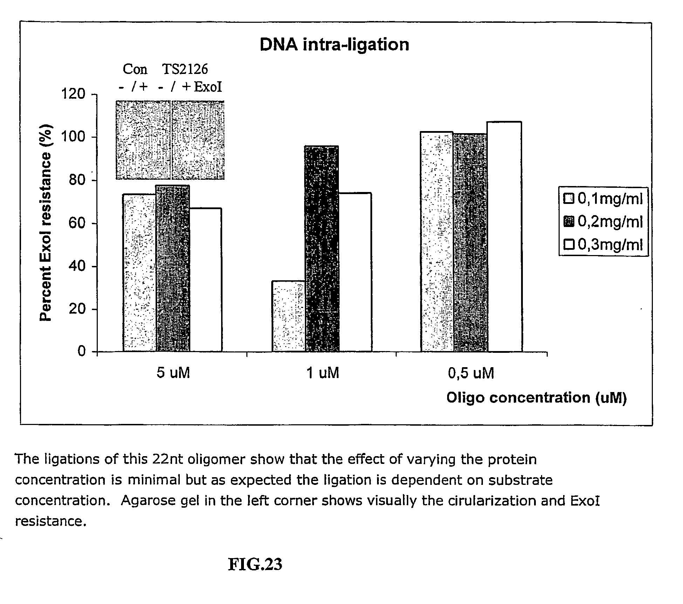

The reaction mixture was incubated for 2 h at 60°C. 10 µl of each sample was digested with 10 U Exonuclease I, mixed with Oligreen reagent (Molecular Probes), and measured with Oligreen Ex/Em 490-520 nm. The remaining of the sample was measured as total and unligated samples (Exo I digested) were used for background subtraction. The samples with and without ligation were run on 4 % agarose gel. The results are shown in Figure 23.

The DNA circularisation experiments routinely give over 50% ligations after 2 hours at 60°C using 20-100 uM ATP and 0,1mg/ml TS2126 RNA ligase.
The addition of Hexamine cobalt chloride or PEG6000 does not significantly enhance the activity.

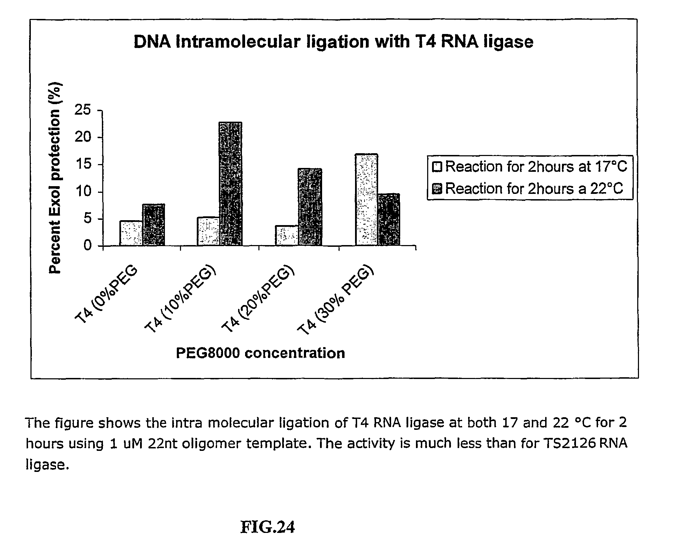

For comparison T4 RNA ligase was also used for ligation of the same substrate in 0,28mg/ml conc. (1U/ul) in 0.02 mM ATP, with 1 mM hexamine cobalt chloride and 10, 20 and 30% PEG6000 at 17 and 22°C. The results are shown in Figure 24.

### Example 22. End-to-end ligation with RM378 RNA ligase and TS2126 RNA ligase.

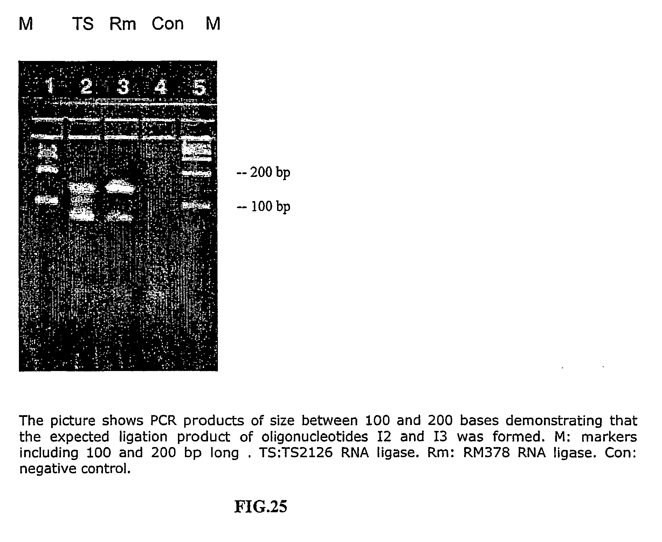

Internal PCR of ligations from IGFA gene synthesis was performed with Fwd2 and Rev primers as described in Example 9. Reactions were the standard protocol we used for the IGFA gene synthesis with oligonucleotides I3 and I2 (Example 9) using 1 µg of TS2126 RNA ligase and 3µg RM378 RNA ligase per reaction. The PCR products were run on an agarose gel. The results shown in Figure 25 demonstrate that both RNA ligases are able to ligate different oligonucleotides end-to-end.

## Claims

1. A method of ligating a nucleotide or nucleotide analog or a nucleic acid containing nucleotides or nucleotide analogs with another nucleotide or nucleotide analog or a nucleic acid containing nucleotides or nucleotide analogs, the method comprising contacting said nucleotides, nucleotide analogs or nucleic acids with an isolated thermostable RNA ligase, wherein the ligase catalyzes a reaction of ligation of the nucleotides, nucleotide analogs or nucleic adds, wherein the ligation is performed at a temperature of at least 50°C.

2. The method of claim 1 wherein the ligation is performed at a temperature in the range of 50°C to 75°C.

3. The method of any of the claims 1-2 wherein the RNA ligase is derived from a thermophilic microorganism.

4. The method of any of the claims 1-3 wherein the nucleotides are RNA or DNA.

5. The method of any of the claims 1-4 wherein the nucleotide analogs contain modified bases, modified sugars and/or modified phosphate groups.

6. The method of any of the claims 1-5 wherein the nucleic acids are single-stranded RNA or DNA.

7. The method of any of the claims 1-6 wherein the thermostable RNA ligase is an isolated RNA ligase selected from the group consisting of:
a) a RNA ligase obtained from a themophilic microorganism;
b) a polypeptide comprising the amino add sequence of SEQ ID NO: 2 or SEQ ID NO: 4;
c) a polypeptide encoded by a nucleic acid comprising the sequence of SEQ ID NO: 1 or SEQ ID NO: 3;
d) a polypeptide having RNA ligase activity and having at least 70% sequence identity with the amino acid sequence of SEQ ID NO: 2 or SEQ ID NO: 4; and
e) a fragment or derivative of a), b), c), or d) having RNA ligase activity.

8. The method of any of the claims 1-7, wherein the microorganism is selected from the group consisting of bacteria, bacteriophage and archea.

9. The method according to claim 1 for forming a phosphodiester bond between a 3' hydroxyl nucleic add acceptor and a 5' phosphate nucleic acid donor, comprising:
a) contacting a 3' hydroxyl nucleic acid acceptor; and
b) a 5' phosphate nucleic acid donor with a thermostable RNA ligase,
wherein a phophodiester bond is formed between the nucleic acids.

10. The method of claim 9 wherein the ligation is performed at a temperature in the range of 50°C to 75°C.

11. The method according to claim 1 for synthesizing an oligonucleotide polymer by repeating cycles of combining a primer oligonucleotide and a blocked oligonucleotide, comprising:
a) combining the primer oligonucleotide and an oligonucleotide blocked at the 3' or 5' end in the presence of a thermostable RNA ligase, thereby forming an extended primer with a blocked 3' or 5' end;
b) enzymatically removing the blocked phosphate group at the 3' or 5' end or enzymatically adding a phosphate group to the 5' end of the extended primer; and
c) repeating a) and b) using the extended primer from b) as the primer for a) wherein an oligonucleotide polymer is formed.

12. The method of Claim 11 wherein the formed oligonucleotide polymer comprises a gene or a part of a gene coding for a polypeptide.

13. The method of claim 12 wherein the ligation is performed at a temperature in the range of 50°C to 75°C.

14. The method according to claim 1 for synthesizing a recombinant gene product, comprising:
a) providing an array of immobilized oligonucleotides comprising predetermined areas on a surface of a solid support, each area having immobilized thereon copies of an oligonucleotide;
b) hybridizing to said immobilized oligonucleotides first single stranded terminal regions of first nucleic add strands to be ligated; and
c) ligating with a thermostable RNA ligase, the hybridized first end of a first nucleic acid and a second nucleic add.

15. The method of claim 14 wherein the ligation is performed at a temperature in the range of 50°C to 75°C.

16. The method according to claim 1 for detecting nucleic acids, comprising:
a) contacting a first probe, a second probe, a target nucleic acid sample and a thermostable RNA ligase, wherein the first probe and the second probe hybridize to the target nucleic acid sample such that the 5' end of the first probe and the 3' end of the second probe are adjacent and can be ligated; and
b) incubating the first probe, second probe, target sample and RNA ligase under conditions that promote hybridization of the probes to the target sequence and that promote ligation of the probes are ligated if the target sequence is present in the target sample.

17. The method of claim 16 wherein the ligation is performed at a temperature in the range of 50°C to 75°C.

18. The method according to claim 1 for amplifying nucleic acids, comprising:
a) contacting a nucleic acid containing sample, wherein the sample comprises a pool of mRNA having a poly-A tail, with i) an oligonucleotide with a 5' end and a 3' end comprising an oligo-dT sequence at the 3' end, a promoter sequence recognized by a RNA polymerase at the 5' end and a transcription initiation region located between the oligo-dT sequence and the promoter sequence wherein the oligonucleotide is blocked at the 3' end to prohibit extension, ii) an enzyme having reverse transcription activity which forms a double stranded promoter-primer sequence, iii) at least one enzyme having Rnase H activity, iv) an enzyme having RNA polymerase activity, and v) sufficient amounts of dNTPs and rNTPs;
b) maintaining the resulting reaction mixture under appropriate conditions for a sufficient amount of time for enzymatic activity, such that antisense RNA is formed in the absence of cDNA intermediates;
c) contacting the multiple copies of RNA with i) a thermostable RNA ligase, ii) a double stranded DNA complex comprising a double stranded DNA promoter sequence, wherein each strand contains a 5' end and a 3' end, the promoter sequence recognizable by a RNA polymerase wherein one strand of said complex has a stretch of RNA attached to the 5' end thereof, iii) an enzyme having RNA polymerase activity, and iv) sufficient amounts of dNTPs and rNTPs; and
d) maintaining the resulting reaction mixture under appropriate conditions for a sufficient amount of time for the enzymatic processes to occur.

19. The method of claim 18 wherein the ligation is performed at a temperature in the range of 50°C to 75°C.

20. The method according to claim 1 for synthesizing a repeat region of an oligonucleotide having a defined sequence, the repeat region including a repeated nucleotide that appears more than once in succession, comprising:
a) ligating an oligonucleotide primer to a 3'-phosphate-blocked repeated nucleotide to form a 3'-phosphate-blocked primer;
b) removing the 3'-phosphate blocking group from the 3'-phosphate-blocked primer using a 3'-phosphatase enzyme, thereby making a deblocked primer without removing the 3'-phosphate blocking group from unreacted 3'-phosphate-blocked repeated nucleotide; and
c) repeating steps (a) and (b) using unreacted 3'-phosphate-blocked repeated nucleotide from step (b) as the 3'-phosphate-blocked repeated nucleotide of step (a) and the deblocked primer product of step (b) as the oligonucleotide primer of step (a) without prior separation of the unreacted 3'-phosphate-blocked repeated nucleotide from the deblocked primer product, whereby the cycles are repeated to form an oligonucleotide having a defined sequence.

21. The method of claim 20 wherein the ligation is performed at a temperature in the range of 50°C to 75°C.

22. The method according to claim 1 for insertion of a single-stranded RNA sequence into a cloning vector, comprising: ligating in the presence of a thermostable RNA ligase, both termini of the single-stranded RNA sequence with a linear double-stranded cloning vector having single-stranded termini complementary to both termini of the single-stranded RNA sequence to form an annealed product, in which the complementary termini of the single-stranded RNA sequence is formed by attaching oligonucleotide linkers to the RNA sequence with a thermostable RNA ligase.

23. The method of claim 22 wherein the ligation is performed at a temperature in the range of 50°C to 75°C.

24. The method according to claim 1 for forming a library of DNA sequences, comprising:
a) forming a library of target RNA fragments by contacting multiple copies of non-denatured target RNA sequences with a library of random oligonucleotides in the presence of a hydrolytic agent under conditions where a subgroup of the library of random oligonucleotides hybridize to the target RNA, whereupon the hydrolytic agent hydrolyzes the target RNA at a site near the 5' end of each hybridized random oligonucleotide, and wherein the 3' ends of each fragment contains the entire sequence to which a random oligonucleotide in the subgroup hybridized; and
b) forming a library of templates for primer extension from the library of target RNA fragments; and forming a library of DNA sequences that are complementary to the target RNA fragments from the library of templates for primer extension by attaching a nucleic acid primer complement sequence to the 3' end of each target RNA fragment with a thermostable RNA ligase.

25. The method of claim 24 wherein the ligation is performed at a temperature in the range of 50°C to 75°C.

26. The method according to claim 1 for amplifying a 5' end region of target mRNA, comprising :
a) dephoshorylating mRNA molecules with a free phosphate group at the 5'-end;
b) removing the 5'-cap on full-length mRNAs;
c) ligating linkers to the 5'-end of decapped mRNA molecules using a thermostable RNA ligase;
d) synthesizing cDNA using reverse transcriptase; and
e) amplifying the cDNA by PCR.

27. The method of claim 26 wherein the ligation is performed at a temperature in the range of 50°C to 75°C.

28. The method according to claim 1 for amplifying mRNA, comprising:
a) synthesizing cDNA, wherein the first strand of cDNA is synthesized by reverse transcription of target mRNA using a 5' end-phosphorylated reverse transcription-primer that is specific for the target RNA wherein a hybrid DNA-RNA is generated;
b) degrading the hybrid DNA-RNA by treatment with at least one enzyme having RNase H activity, to remove RNA;
c) circularizing the single-stranded cDNA to form concatemers with a thermostable RNA ligase; and
d) amplifying DNA by PCR using specific primers that are complementary to known sequences.

29. The method of claim 28 wherein the ligation is performed at a temperature in the range of 50°C to 75°C.

30. The method according to claim 1 for amplifying nucleic acids with single primer, comprising:
a) hybridizing a mixture of nucleic acids with a degenerate or non-degenerate primer targeted to a single region in a target sequence;
b) synthesizing single-stranded DNA-complementary to a region of said target sequence, said synthesis being primed by said degenerate or non-degenerate primer and catalyzed by a DNA polymerase or a reverse transcriptase, thereby performing linear amplification of said target sequence by repeated thermal cycling;
c) providing a second primer site to said single-stranded DNA by ligating an oligonucleotide to its 3' end, wherein the ligation is catalyzed by a thermostable RNA ligase;
d) amplifying the single-stranded DNA using a primer pair wherein a first primer comprises at least a part of the degerate or non-degenerate primer sequence, or wherein a first primer is targeted to a region downstream to the degenerate or non-degenerate primer sequence, and the second primer is complementary to the 3' primer site of step (c).

31. The method of Claim 30 wherein the single stranded DNA synthesized in step b) is purified.

32. The method of claim 31 wherein the ligation is performed at a temperature in the range of 50°C to 75°C.

33. The method according to claim 1 for sequencing oligonucleotides, comprising:
a) contacting a target oligonucleotide with a thermostable RNA ligase under conditions wherein the ligase adds an auxilary oligonucleotide to the 3' end of the target oligonucleotide; and
b) sequencing the oligonucleotide.

34. The method of claim 33 wherein the ligation is performed at a temperature in the range of 50°C to 75°C.

## Patentansprüche

1. Verfahren des Ligierens eines Nukleotids oder Nukleotidanalogs oder einer Nukleinsäure, welche Nukleotide oder Nukleotidanaloga enthalten, mit einem anderen Nukleotid oder Nukleotidanalog oder einer Nukleinsäure, welche Nukleotide oder Nukleotidanaloga enthalten, wobei das Verfahren das Kontaktieren der Nukleotide, Nukleotidanaloga oder Nukleinsäuren mit einer isolierten thermostabilen RNA-Ligase umfasst, wobei die Ligase eine Ligationsreaktion der Nukleotide, Nukleotidanaloga oder Nukleinsäuren katalysiert, wobei die Ligation bei einer Temperatur von mindestens 50°C durchgeführt wird.

2. Verfahren nach Anspruch 1, wobei die Ligation bei einer Temperatur im Bereich von 50°C bis 75°C durchgeführt wird.

3. Verfahren nach einem der Ansprüche 1-2, wobei die RNA-Ligase von einem thermophilen Mikroorganismus stammt.

4. Verfahren nach einem der Ansprüche 1-3, wobei es sich bei den Nukleotiden um RNA oder DNA handelt.

5. Verfahren nach einem der Ansprüche 1-4, wobei die Nukleotidanaloga modifizierte Basen, modifizierte Zucker und/oder modifizierte Phosphatgruppen enthalten.

6. Verfahren nach einem der Ansprüche 1-5, wobei es sich bei den Nukleotiden um einzelsträngige RNA oder DNA handelt.

7. Verfahren nach einem der Ansprüche 1-6, wobei es sich bei der thermostabilen RNA-Ligase um eine isolierte RNA-Ligase handelt, die aus der Gruppe ausgewählt ist, die aus Folgenden besteht:
a) einer RNA-Ligase, die von einem thermophilen Mikroorganismus stammt;
b) einem Polypeptid, welches die Aminosäuresequenz von SEQ ID Nr: 2 oder SEQ ID Nr: 4 aufweist;
c) einem von einer Nukleinsäure kodiertes Polypeptid, welches die Sequenz von SEQ ID Nr: 1 oder SEQ ID Nr: 3 aufweist;
d) einem Polypeptid, das RNA-Ligaseaktivität und eine Sequenzidentität von mindestens 70% zu der Aminosäuresequenz von SEQ ID Nr: 2 oder SEQ ID Nr: 4 aufweist; und
e) einem Fragment oder Derivat von a), b), c) oder d), das RNA-Ligaseaktivität aufweist.

8. Verfahren nach einem der Ansprüche 1-7, wobei der Mikroorganismus aus der Gruppe ausgewählt ist, die aus Bakterien, Bakteriophage und Archaea besteht.

9. Verfahren nach Anspruch 1 zum Ausbilden einer Phosphodiesterbindung zwischen einem 3'-Hydroxylnukleinsäureakzeptor und einem 5'-Phosphatnukleinsäuredonor, umfassend:
a) Kontaktieren eines 3'-Hydroxylnukleinsäureakzeptors und
b) eines 5'-Phosphatnukleinsäuredonors mit einer thermostabilen RNA-Ligase, wobei zwischen den Nukleinsäuren eine Phosphodiesterbindung gebildet wird.

10. Verfahren nach Anspruch 9, wobei die Ligation bei einer Temperatur im Bereich von 50°C bis 75°C durchgeführt wird.

11. Verfahren nach Anspruch 1 zum Synthetisieren eines Oligonukleotidpolymers durch sich wiederholende Zyklen des Kombinierens eines Oligonukleotidprimers und eines blockierten Oligonukleotids, welches umfasst:
a) Kombinieren des Oligonukleotidprimers und eines am 3'- oder 5'-Ende blockierten Oligonukleotids in Gegenwart einer thermostabilen RNA-Ligase, wodurch ein verlängerter Primer mit einem blockierten 3'- oder 5'-Ende gebildet wird;
b) enzymatisches Entfernen der blockierten Phosphatgruppe am 3'- oder 5'-Ende oder enzymatisches Hinzufügen einer Phosphatgruppe an das 5'-Ende des verlängerten Primers; und
c) Wiederholen von a) und b), wobei der verlängerte Primer von b) als Primer für a) verwendet wird, wobei ein Oligonukleotidpolymer gebildet wird.

12. Verfahren nach Anspruch 11, wobei das gebildete Oligonukleotidpolymer ein Gen oder einen Teil eines Gens umfasst, welches ein Polypeptid kodiert.

13. Verfahren nach Anspruch 12, wobei die Ligation bei einer Temperatur im Bereich von 50°C bis 75°C durchgeführt wird.

14. Verfahren nach Anspruch 1 zum Synthetisieren eines rekombinanten Genproduktes, umfassend:
a) Bereitstellen einer Anordnung immobilisierter Oligonukleotide, umfassend vorbestimmte Bereiche auf einer Fläche eines festen Trägers, wobei auf jedem Bereich Kopien eines Oligonukleotids immobilisiert sind;
b) Hybridisieren erster einzelsträngiger terminaler Regionen von ersten zu ligierenden Nukleinsäuresträngen an die immobilisierten Oligonukleotide; und
c) Ligieren des hybridisierten ersten Endes einer ersten Nukleinsäure und einer zweiten Nukleinsäure mit einer thermostabilen RNA-Ligase;

15. Verfahren nach Anspruch 14, wobei die Ligation bei einer Temperatur im Bereich von 50°C bis 75°C durchgeführt wird.

16. Verfahren nach Anspruch 1 zum Feststellen von Nukleinsäuren, umfassend:
a) Kontaktieren einer ersten Sonde, einer zweiten Sonde, einer Nukleinsäurezielprobe und einer thermostabilen RNA, wobei die erste Sonde und die zweite Sonde derart an die Nukleinsäurezielprobe hybridisieren, dass das 5'-Ende der ersten Sonde und das 3'-Ende der zweiten Sonde benachbart sind und ligiert werden können; und
b) Inkubieren der ersten Sonde, der zweiten Sonde, der Zielprobe und der RNA-Ligase unter Bedingungen, welche die Hybridisierung der Sonden an die Zielsequenz fördern und die Ligation der Sonden fördern, wenn die Zielsequenz in der Zielprobe vorhanden ist.

17. Verfahren nach Anspruch 16, wobei die Ligation bei einer Temperatur im Bereich von 50°C bis 75°C durchgeführt wird.

18. Verfahren nach Anspruch 1 zum Amplifizieren von Nukleinsäuren, umfassend:
a) Kontaktieren einer eine Nukleinsäure enthaltenden Probe, wobei die Probe einen Pool von mRNA mit einem Poly-A-Schwanz umfasst, mit i) einem Oligonukleotid mit einem 5'-Ende und einem 3'-Ende, das eine Oligo-dT-Sequenz am 3'-Ende aufweist, eine von einer RNA-Polymerase erkannte Promotorsequenz am 5'-Ende aufweist und bei dem sich zwischen der Oligo-dT-Sequenz und der Promotorsequenz eine Transkriptionsinitiatoinsregion befindet, wobei das Oligonukleotid am 3'-Ende blockiert ist, um eine Verlängerung zu verhindern, ii) einem Enzym mit reverser Transkriptionsaktivität, das eine doppelsträngige Promotor-Primer-Sequenz ausbildet, iii) mindestens einem Enzym mit RNase-H-Aktivität, iv) einem Enzym mit RNA-Polymeraseaktivität und v) ausreichenden Mengen von dNTPs und rNTPs;
b) Halten des resultierenden Reaktionsgemisches für eine ausreichende Zeitdauer unter geeigneten Bedingungen für enzymatische Aktivität, so dass in Abwesenheit von cDNA-Zwischenstufen eine Antisense-RNA gebildet wird;
c) Kontaktieren der vielen Kopien von RNA mit i) einer thermostabilen RNA-Ligase, ii) einem doppelsträngigen DNA-Komplex, der eine doppelsträngige DNA-Promotorsequenz aufweist, wobei jeder Strang ein 5'-Ende und ein 3'-Ende aufweist, die Promotorsequenz von einer RNA-Polymerase erkannt werden kann, wobei ein Strang des Komplexes eine RNA-Strecke aufweist, die an dessen 5'-Ende angebracht ist, iii) einem Enzym mit RNA-Polymeraseaktivität und iv) ausreichenden Mengen von dNTPs und rNTPs; und
d) Halten des resultierenden Reaktionsgemisches für eine ausreichende Zeitdauer unter geeigneten Bedingungen, damit die enzymatischen Prozesse stattfinden können.

19. Verfahren nach Anspruch 18, wobei die Ligation bei einer Temperatur im Bereich von 50°C bis 75°C durchgeführt wird.

20. Verfahren nach Anspruch 1 zum Synthetisieren einer Wiederholungsregion eines Oligonukleotids mit einer definierten Sequenz, wobei die Wiederholungsregion ein wiederholtes Nukleotid aufweist, das mehr als einmal in Aufeinanderfolge auftritt, umfassend:
a) Ligieren eines Oligonukleotidprimers an ein 3'-phosphatblockiertes wiederholtes Nukleotid, um einen 3'-phosphatblockierten Primer zu bilden;
b) Entfernen der 3'-Phosphatblockierungsgruppe von dem 3'-phosphatblockierten Primer unter Verwendung eines 3'-Phosphataseenzyms, wodurch ein entblockter Primer hergestellt wird, ohne dass die 3'-Phosphatblockierungsgruppe von nicht umgesetztem 3'-phosphatblockiertem wiederholtem Nukleotid entfernt wird; und
c) Wiederholen von Schritt (a) und (b) unter Verwendung von nicht umgesetztem 3'-phosphatblockiertem wiederholtem Nukleotid von Schritt (b) als 3'-phosphatblockiertes wiederholtes Nukleotid von Schritt (a) und dem entblockten Primerprodukt von Schritt (b) als dem Oligonukleotidprimer von Schritt (a) ohne vorherige Trennung des nicht umgesetzten 3'-phosphatblockierten wiederholten Nukleotids von dem entblockten Primerprodukt, wobei die Zyklen wiederholt werden, um ein Oligonukleotid mit einer definierten Sequenz zu bilden.

21. Verfahren nach Anspruch 20, wobei die Ligation bei einer Temperatur im Bereich von 50°C bis 75°C durchgeführt wird.

22. Verfahren nach Anspruch 1 zum Einsetzen einer einzelsträngigen RNA-Sequenz in einen Klonierungsvektor, umfassend: Ligieren beider Termini der einzelsträngigen RNA-Sequenz in Gegenwart einer thermostabilen RNA-Ligase mit einem linearen doppelsträngigen Klonierungsvektor, der einzelsträngige Termini aufweist, welche zu beiden Termini der einzelsträngigen RNA-Sequenz komplementär sind, um ein Anheftungsprodukt (Annealing-Produkt) auszubilden, bei dem die komplementären Enden der einzelsträngigen RNA-Sequenz gebildet werden, indem mit einer thermostabilen RNA-Ligase Oligonukleotidlinker an die RNA-Sequenz angebracht werden.

23. Verfahren nach Anspruch 22, wobei die Ligation bei einer Temperatur im Bereich von 50°C bis 75°C durchgeführt wird.

24. Verfahren nach Anspruch 1 zum Ausbilden einer Bibliothek von DNA-Sequenzen, umfassend:
a) Ausbilden einer Bibliothek von RNA-Zielfragmenten durch Kontaktieren vieler Kopien nicht denaturierter RNA-Zielsequenzen mit einer Bibliothek zufälliger Oligonukleotide in Gegenwart eines hydrolytischen Agens unter Bedingungen, bei denen eine Teilgruppe der Bibliothek zufälliger Oligonukleotide an die Ziel-RNA hybridisiert, wobei das hydrolytische Agens anschließend die Ziel-RNA an einer Stelle in der Nähe des 5'-Endes eines jeden hybridisierten zufälligen Oligonukleotids hydrolysiert und wobei die 3'-Enden jedes Fragments die gesamte Sequenz enthalten, an welche ein zufälliges Oligonukleotid in der Teilgruppe hybridisierte; und
b) Ausbilden einer Bibliothek von Vorlagen (Templates) zur Primerverlängerung aus der Bibliothek von RNA-Zielfragmenten, und Ausbilden einer Bibliothek von DNA-Sequenzen, die zu den RNA-Zielfragmenten aus der Bibliothek von Vorlagen (Templates) zur Primerverlängerung komplementär sind, indem eine Nukleinsäureprimerkomplementsequenz mit einer thermostabilen RNA-Ligase an das 3'-Ende jedes RNA-Zielfragmentes angebracht wird.

25. Verfahren nach Anspruch 24, wobei die Ligation bei einer Temperatur im Bereich von 50°C bis 75°C durchgeführt wird.

26. Verfahren nach Anspruch 1 zum Amplifizieren einer 5'-Endregion von Ziel-mRNA, umfassend:
a) Entphosphorylieren von mRNA-Molekülen mit einer freien Phosphatgruppe am 5'-Ende;
b) Entfernen der 5'-Cap an Volllängen-mRNAs;
c) Ligieren von Linkern an das 5'-Ende von entcappten mRNA-Molekülen unter Verwendung einer thermostabilen RNA-Ligase;
d) Synthetisieren von cDNA unter Verwendung von reverser Transkriptase; und
e) Amplifizieren der cDNA durch PCR.

27. Verfahren nach Anspruch 26, wobei die Ligation bei einer Temperatur im Bereich von 50°C bis 75°C durchgeführt wird.

28. Verfahren nach Anspruch 1 zum Amplifizieren von mRNA, umfassend:
a) Synthetisieren von cDNA, wobei der erste Strang der cDNA durch reverse Transkription von Ziel-mRNA unter Verwendung eines 5'-endphosphorylierten Primers für die reverse Transkription synthetisiert wird, der für die Ziel-RNA spezifisch ist, wodurch ein DNA-RNA-Hybrid erzeugt wird;
b) Abbau des DNA-RNA-Hybrids durch Behandlung mit mindestens einem Enzym mit RNase-H-Aktivität zur Entfernung von RNA;
c) Zirkularisieren der einzelsträngigen cDNA zur Ausbildung von Konkatemeren mit einer thermostabilen RNA-Ligase; und
d) Amplifizieren von DNA durch PCR unter Verwendung spezifischer Primer, die zu bekannten Sequenzen komplementär sind.

29. Verfahren nach Anspruch 28, wobei die Ligation bei einer Temperatur im Bereich von 50°C bis 75°C durchgeführt wird.

30. Verfahren nach Anspruch 1 zum Amplifizieren von Nukleinsäuren mit einem einzelnen Primer, umfassend:
a) Hybridisieren eines Gemisches von Nukleinsäuren mit einem degenerierten oder nicht degenerierten Primer, der auf eine einzelne Region in einer Zielsequenz abzielt;
b) Synthetisieren einzelsträngiger DNA, die zu einer Region der Zielsequenz komplementär ist, wobei die Synthese von dem degenerierten oder nicht degenerierten Primer geprimt und von einer DNA-Polymerase oder einer reversen Transkriptase katalysiert wird, wodurch eine lineare Amplifizierung der Zielsequenz durch wiederholtes Thermozyklieren (Thermal Cycling) durchgeführt wird;
c) Ausstatten der einzelsträngigen DNA mit einer zweiten Primerstelle, indem an ihr 3'-Ende ein Oligonukleotid ligiert wird, wobei die Ligation von einer thermostabilen RNA-Ligase katalysiert wird;
d) Amplifizieren der einzelsträngigen DNA durch Verwendung eines Primer-Paars, wobei ein erster Primer mindestens einen Teil der degenerierten oder nicht degenerierten Primersequenz aufweist oder wobei einer erster Primer auf eine Region abzielt, die sich stromabwärts (downstream) von der degenerierten oder nicht degenerierten Primersequenz befindet, und der zweite Primer zu der 3'-Primerstelle von Schritt (c) komplementär ist.

31. Verfahren nach Anspruch 30, wobei die in Schritt (b) synthetisierte einzelsträngige DNA gereinigt wird.

32. Verfahren nach Anspruch 31, wobei die Ligation bei einer Temperatur im Bereich von 50°C bis 75°C durchgeführt wird.

33. Verfahren nach Anspruch 1 zum Sequenzieren von Oligonukleotiden, umfassend:
a) Kontaktieren eines Zieloligonukleotids mit einer thermostabilen RNA-Ligase unter Bedingungen, bei denen die Ligase dem 3'-Ende des Zieloligonukleotids ein Hilfsoligonukleotid hinzufügt; und
b) Sequenzieren des Oligonukleotids.

34. Verfahren nach Anspruch 33, wobei die Ligation bei einer Temperatur im Bereich von 50°C bis 75°C durchgeführt wird.

## Revendications

1. Procédé de ligature d'un nucléotide ou d'un analogue de nucléotide ou d'un acide nucléique contenant des nucléotides ou des analogues de nucléotides avec un autre nucléotide ou analogue de nucléotide ou un acide nucléique contenant des nucléotides ou des analogues de nucléotides, le procédé comprenant la mise en contact desdits nucléotides, desdits analogues de nucléotides ou desdits acides nucléiques avec une ARN ligase thermostable isolée, dans lequel la ligase catalyse une réaction de ligature des nucléotides, des analogues de nucléotides ou des acides nucléiques, dans lequel la ligature est réalisée à une température d'au moins 50°C.

2. Procédé selon la revendication 1, dans lequel la ligature est réalisée à une température dans la plage allant de 50°C à 75°C.

3. Procédé selon l'une quelconque des revendications 1 et 2, dans lequel l'ARN ligase est dérivée d'un microorganisme thermophile.

4. Procédé selon l'une quelconque des revendications 1 à 3, dans lequel les nucléotides sont l'ARN ou l'ADN.

5. Procédé selon l'une quelconque des revendications 1 à 4, dans lequel les analogues de nucléotides contiennent des bases modifiées, des sucres modifiés et/ou des groupes phosphate modifiés.

6. Procédé selon l'une quelconque des revendications 1 à 5, dans lequel les acides nucléiques sont l'ARN ou ADN monocaténaire.

7. Procédé selon l'une quelconque des revendications 1 à 6, dans lequel l'ARN ligase thermostable est une ARN ligase isolée choisie dans le groupe constitué par :
a) une ARN ligase obtenue d'un microorganisme thermophile ;
b) un polypeptide comprenant la séquence d'acides aminés de SEQ ID NO : 2 ou SEQ ID NO : 4 ;
c) un polypeptide codé par un acide nucléique comprenant la séquence de SEQ ID NO: 1 ou SEQ ID NO: 3 ;
d) un polypeptide ayant une activité ARN ligase et ayant une identité de séquence d'au moins 70 % avec la séquence d'acides aminés de SEQ ID NO: 2 ou SEQ ID NO: 4; et
e) un fragment ou un dérivé de a), b), c) ou d) ayant une activité ARN ligase.

8. Procédé selon l'une quelconque des revendications 1 à 7, dans lequel le microorganisme est choisi dans le groupe constitué par les bactéries, un bactériophage et les Archaea.

9. Procédé selon la revendication 1, pour la formation d'une liaison phosphodiester entre un accepteur d'acide nucléique ayant un groupe hydroxyle en 3' et un donneur d'acide nucléique ayant un groupe phosphate en 5', qui comprend :
a) la mise en contact d'un accepteur d'acide nucléique ayant un groupe hydroxyle en 3' ; et
b) d'un donneur d'acide nucléique ayant un groupe phosphate en 5' avec une ARN ligase thermostable, dans lequel une liaison phosphodiester est formée entre les acides nucléiques.

10. Procédé selon la revendication 9, dans lequel la ligature est réalisée à une température dans la plage allant de 50°C à 75°C.

11. Procédé selon la revendication 1, pour la synthèse d'un polymère d'oligonucléotides en répétant des cycles de combinaison d'un oligonucléotide amorce et d'un oligonucléotide bloqué, qui comprend :
a) la combinaison de l'oligonucléotide amorce et d'un oligonucléotide bloqué à l'extrémité 3' ou 5' en présence d'une ARN ligase thermostable, formant ainsi une amorce allongée avec une extrémité 3' ou 5' bloquée ;
b) l'élimination par voie enzymatique du groupe phosphate bloqué à l'extrémité 3' ou 5' ou l'ajout par voie enzymatique d'un groupe phosphate à l'extrémité 5' de l'amorce allongée; et
c) la répétition de a) et b) en utilisant l'amorce allongée de b) en tant que l'amorce pour a) où un polymère d'oligonucléotides est formé.

12. Procédé selon la revendication 11, dans lequel le polymère d'oligonucléotides formé comprend un gène ou une partie d'un gène codant pour un polypeptide.

13. Procédé selon la revendication 12, dans lequel la ligature est réalisée à une température dans la plage allant de 50°C à 75°C.

14. Procédé selon la revendication 1, pour la synthèse d'un produit génique recombinant, qui comprend :
a) la fourniture d'un réseau d'oligonucléotides immobilisés comprenant des zones prédéterminées sur une surface d'un support solide, chaque zone ayant immobilisées sur elle des copies d'un oligonucléotide ;
b) l'hybridation auxdits oligonucléotides immobilisés de premières régions terminales monocaténaires de premiers brins d'acide nucléique à ligaturer ; et
c) la ligature avec une ARN ligase thermostable, de la première extrémité hybridée d'un premier acide nucléique et d'un second acide nucléique.

15. Procédé selon la revendication 14, dans lequel la ligature est réalisée à une température dans la plage allant de 50°C à 75°C.

16. Procédé selon la revendication 1, pour la détection d'acides nucléiques, qui comprend :
a) la mise en contact d'une première sonde, d'une seconde sonde, d'un échantillon d'acide nucléique cible et d'une ARN ligase thermostable, dans lequel la première sonde et la seconde sonde s'hybrident à l'échantillon d'acide nucléique cible de telle manière que l'extrémité 5' de la première sonde et l'extrémité 3' de la seconde sonde sont adjacentes et peuvent être ligaturées ; et
b) l'incubation de la première sonde, de la seconde sonde, de l'échantillon cible et de l'ARN ligase dans des conditions qui promeuvent l'hybridation des sondes à la séquence cible et qui promeuvent la ligature des sondes qui sont ligaturées si la séquence cible est présente dans l'échantillon cible.

17. Procédé selon la revendication 16, dans lequel la ligature est réalisée à une température dans la plage allant de 50°C à 75°C.

18. Procédé selon la revendication 1, pour l'amplification d'acides nucléiques, qui comprend :
a) la mise en contact d'un échantillon contenant un acide nucléique, dans lequel l'échantillon comprend un pool d'ARNm ayant une queue poly-A, avec i) un oligonucléotide avec une extrémité 5' et une extrémité 3' comprenant une séquence oligo-dT à l'extrémité 3', une séquence promotrice reconnue par une ARN polymérase à l'extrémité 5' et une région d'initiation de la transcription située entre la séquence oligo-dT et la séquence promotrice dans lequel l'oligonucléotide est bloqué au niveau de l'extrémité 3' pour empêcher l'extension, ii) une enzyme ayant une activité de transcription inverse qui forme une séquence amorce promotrice bicaténaire, iii) au moins une enzyme ayant une activité RNase H, iv) une enzyme ayant une activité ARN polymérase, et v) des quantités suffisantes de dNTP et de rNTP ;
b) la conservation du mélange réactionnel résultant dans des conditions adaptées pendant une durée suffisante pour l'activité enzymatique, de telle manière qu'un ARN antisens est formé en l'absence d'intermédiaires d'ADNc ;
c) la mise en contact des copies multiples d'ARN avec i) une ARN ligase thermostable, ii) un complexe d'ADN bicaténaire comprenant une séquence promotrice d'ADN bicaténaire, où chaque brin contient une extrémité 5' et une extrémité 3', la séquence promotrice pouvant être reconnue par une ARN polymérase dans laquelle un brin dudit complexe a une extension d'ARN fixée à son extrémité 5', iii) une enzyme ayant une activité ARN polymérase, et iv) des quantités suffisantes de dNTP et de rNTP ; et
d) la conservation du mélange réactionnel résultant dans des conditions adaptées pendant une durée suffisante pour que les processus enzymatiques se produisent.

19. Procédé selon la revendication 18, dans lequel la ligature est réalisée à une température dans la plage allant de 50°C à 75°C.

20. Procédé selon la revendication 1, pour la synthèse d'une région de répétition d'un oligonucléotide ayant une séquence définie, la région de répétition comprenant un nucléotide répété qui apparaît plus d'une fois de suite, qui comprend :
a) la ligature d'une amorce oligonucléotidique à un nucléotide répété bloqué par un groupe phosphate en 3' pour former une amorce bloquée par un groupe phosphate en 3' ;
b) l'élimination du groupe de blocage phosphate en 3' de l'amorce bloquée par un groupe phosphate en 3' en utilisant une enzyme 3'-phosphatase, faisant ainsi une amorce débloquée sans éliminer le groupe de blocage phosphate en 3' du nucléotide répété bloqué par un groupe phosphate en 3' n'ayant pas réagi ; et
c) la répétition des étapes (a) et (b) en utilisant le nucléotide répété bloqué par un groupe phosphate en 3' n'ayant pas réagi de l'étape (b) en tant que le nucléotide répété bloqué par un groupe phosphate en 3' de l'étape (a) et le produit d'amorce débloquée de l'étape (b) en tant que l'amorce oligonucléotidique de l'étape (a) sans séparation préalable du nucléotide répété bloqué par un groupe phosphate en 3' n'ayant pas réagi du produit d'amorce débloquée, moyennant quoi les cycles sont répétés pour former un oligonucléotide ayant une séquence définie.

21. Procédé selon la revendication 20, dans lequel la ligature est réalisée à une température dans la plage allant de 50°C à 75°C.

22. Procédé selon la revendication 1, pour l'insertion d'une séquence d'ARN monocaténaire dans un vecteur de clonage, qui comprend : la ligature en présence d'une ARN ligase thermostable, des deux extrémités de la séquence d'ARN monocaténaire avec un vecteur de clonage bicaténaire linéaire ayant des extrémités monocaténaires complémentaires des deux extrémités de la séquence d'ARN monocaténaire pour former un produit annelé, dans lequel les extrémités complémentaires de la séquence d'ARN monocaténaire sont formées par fixation de lieurs oligonucléotidiques à la séquence d'ARN avec une ARN ligase thermostable.

23. Procédé selon la revendication 22, dans lequel la ligature est réalisée à une température dans la plage allant de 50°C à 75°C.

24. Procédé selon la revendication 1, pour la formation d'une banque de séquences d'ADN, qui comprend :
a) la formation d'une banque de fragments d'ARN cible par mise en contact de multiples copies de séquences d'ARN cible non dénaturé avec une banque d'oligonucléotides aléatoires en présence d'un agent hydrolytique dans des conditions dans lesquelles un sous-groupe de la banque d'oligonucléotides aléatoires s'hybride à l'ARN cible, après quoi l'agent hydrolytique hydrolyse l'ARN cible à un site proche de l'extrémité 5' de chaque oligonucléotide aléatoire hybridé, et dans lequel l'extrémité 3' de chaque fragment contient la totalité de la séquence à laquelle un oligonucléotide aléatoire dans le sous-groupe s'est hybridé ; et
b) la formation d'une banque de matrices pour l'extension d'amorces à partir de la banque de fragments d'ARN cible ; et la formation d'une banque de séquences d'ADN qui sont complémentaires des fragments d'ARN cible de la banque de matrices pour l'extension d'amorces par fixation d'une séquence complémentaire d'amorce d'acide nucléique à l'extrémité 3' de chaque fragment d'ARN cible avec une ARN ligase thermostable.

25. Procédé selon la revendication 24, dans lequel la ligature est réalisée à une température dans la plage allant de 50°C à 75°C.

26. Procédé selon la revendication 1, pour l'amplification d'une région de l'extrémité 5' d'ARNm cible, qui comprend :
a) la déphosphorylation de molécules d'ARNm avec un groupe phosphate libre à l'extrémité 5' ;
b) l'élimination de la coiffe en 5' sur les ARNm de longueur totale ;
c) la ligature de lieurs à l'extrémité 5' de molécules d'ARNm décoiffé en utilisant une ARN ligase thermostable ;
d) la synthèse de l'ADNc en utilisant une transcriptase inverse ; et
e) l'amplification de l'ADNc par PCR.

27. Procédé selon la revendication 26, dans lequel la ligature est réalisée à une température dans la plage allant de 50°C à 75°C.

28. Procédé selon la revendication 1, pour l'amplification d'ARNm, qui comprend :
a) la synthèse d'ADNc, dans laquelle le premier brin d'ADNc est synthétisé par transcription inverse d'ARNm cible en utilisant une amorce de transcription inverse phosphorylée à l'extrémité 5' qui est spécifique de l'ARN cible dans lequel un hybride ADN-ARN est généré ;
b) la dégradation de l'hybride ADN-ARN par traitement avec au moins une enzyme ayant une activité RNase H, pour éliminer l'ARN ;
c) la circularisation de l'ADNc monocaténaire pour former des concatémères avec une ARN ligase thermostable ; et
d) l'amplification d'ADN par PCR en utilisant des amorces spécifiques qui sont complémentaires de séquences connues.

29. Procédé selon la revendication 28, dans lequel la ligature est réalisée à une température dans la plage allant de 50°C à 75°C.

30. Procédé selon la revendication 1, pour l'amplification d'acides nucléiques avec une amorce unique, qui comprend :
a) l'hybridation d'un mélange d'acides nucléiques avec une amorce dégénérée ou non dégénérée ciblant une région unique dans une séquence cible ;
b) la synthèse d'ADN monocaténaire complémentaire d'une région de ladite séquence cible, ladite synthèse étant amorcée par ladite amorce dégénérée ou non dégénérée et catalysée par une ADN polymérase ou une transcriptase inverse, réalisant ainsi une amplification linéaire de ladite séquence cible par cyclage thermique répété ;
c) la fourniture d'un second site d'amorce au dit ADN monocaténaire par ligature d'un oligonucléotide à son extrémité 3', où la ligature est catalysée par une ARN ligase thermostable ;
d) l'amplification de l'ADN monocaténaire en utilisant une paire d'amorce dans laquelle une première amorce comprend au moins une partie d'une séquence d'amorce dégénérée ou non dégénérée, ou dans laquelle une première amorce cible une région en aval de la séquence d'amorce dégénérée ou non dégénérée, et la seconde amorce est complémentaire du site d'amorce en 3' de l'étape (c).

31. Procédé selon la revendication 30, dans lequel l'ADN monocaténaire synthétisé dans l'étape b) est purifié.

32. Procédé selon la revendication 31, dans lequel la ligature est réalisée à une température dans la plage allant de 50°C à 75°C.

33. Procédé selon la revendication 1, pour le séquençage d'oligonucléotides, qui comprend :
a) la mise en contact d'un oligonucléotide cible avec une ARN ligase thermostable dans des conditions dans lesquelles la ligase ajoute un oligonucléotide auxiliaire à l'extrémité 3' de l'oligonucléotide cible ; et
b) le séquençage de l'oligonucléotide.

34. Procédé selon la revendication 33, dans lequel la ligature est réalisée à une température dans la plage allant de 50°C à 75°C.
